# EUROPEAN PATENT APPLICATION

(11) **EP 4 752 132 A1**
(43) Date of publication of application: **03.06.2026**
(21) Application number: 24216361.6
(22) Date of filing: 29.11.2024
(51) Int. Cl.: C07C 255/51, C07D 401/10, H10K 85/60

(54) **COMPOUND OF FORMULA (I) AND THEIR USE IN AN ORGANIC ELECTRONIC DEVICE**

(71) Applicant: Novaled GmbH, 01099 Dresden (DE)
(72) Inventor: Luschtinetz, Regina, 01099 Dresden (DE); Nüllen, Max Peter, 01099 Dresden (DE); Schulze, Benjamin, 01099 Dresden (DE); Wudarczyk, Jakob Jacek, 01099 Dresden (DE)
(74) Representative: Michalski Hüttermann & Partner Patentanwälte mbB

(57) **Abstract**

The present invention relates to a compound of formula (I) and said use in organic electronic devices, an organic semiconductor layer comprising the compound of formula (I), an organic electronic device comprising the organic semiconductor layer, and a display device comprising the organic electronic device.

## Description

### Technical Field

The present invention relates to a compound of formula (I) and said use in organic electronic devices, an organic semiconductor layer comprising the compound of formula (I), an organic electronic device comprising the organic semiconductor layer, and a display device comprising the organic electronic device.

### Background Art

Organic electronic devices, such as organic light-emitting diodes OLEDs, which are self-emitting devices, have a wide viewing angle, excellent contrast, quick response, high brightness, excellent operating voltage characteristics, and color reproduction. A typical OLED comprises an anode, a hole transport layer HTL, an emission layer EML, an electron transport layer ETL, and a cathode, which are sequentially stacked on a substrate. In this regard, the HTL, the EML, and the ETL are thin films formed from organic compounds.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency and/or a long lifetime.

Performance of an organic light emitting diode may be affected by characteristics of the semiconductor layer, and among them, may be affected by characteristics of quinoid which are also contained in the semiconductor layer.

There remains a need to improve performance of organic semiconductor materials, semiconductor layers, as well as organic electronic devices thereof, in particular to achieve improved operating voltage, improved efficiency, improved lifetime and/or improved voltage stability over time through improving the characteristics of the compounds comprised therein. In addition, there remains a need to improve LUMO energy, improve the dipole moment and/or improve the thermal properties compared to comparative examples, in particular to improve thermal stability and/or processing properties at elevated temperatures.

### DISCLOSURE

An aspect of the present invention provides a compound of formula (I): 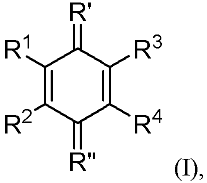 wherein
R¹, R², R³ and R⁴ are independently selected from the group comprising H, D, F or CN; wherein
   two R¹, R², R³ and R⁴ are selected from CN and two R¹, R², R³ and R⁴ are selected from H, D or F;
R' is selected from formula (II): 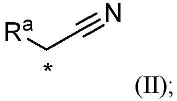
R^{a} is selected from formula (III): 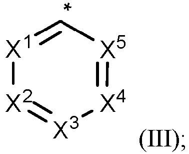
wherein the asterisks "*" denotes the binding position;
wherein
X¹ is selected from CR⁶ or N;
X² is selected from CR⁷ or N;
X³ is selected from CR⁸ or N;
X⁴ is selected from CR⁹ or N;
X⁵ is selected from CR¹⁰ or N;
wherein
   R⁶, R⁷, R⁸, R⁹ and R¹⁰ are independently selected from CN, unsubstituted C₁ to C₈ alkyl, substituted or unsubstituted C₆ to C₁₈ aryl, substituted or unsubstituted C₃ to C₁₈ heteroaryl F, SF₅, D or H,
   wherein the one or more substituent on R⁶, R⁷, R⁸, R⁹ and R¹⁰ are independently selected from CN, F, SF₅, D;
wherein
   at least one X¹ to X⁵ are independently selected from CH or CD and at least one R⁶ to R¹⁰ is CN or F;
R" is selected from formula (IV): 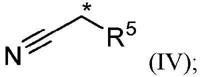
wherein the asterisks "*" denotes the binding position;
wherein
   R⁵ is selected from substituted or unsubstituted C₆ to C₁₉ aryl, substituted or unsubstituted C₆ to C₁₉ aryl with one, two or three aromatic six-member rings, substituted or unsubstituted C₉ to C₁₉ aryl with two or three fused aromatic six-member rings, substituted or unsubstituted C₆ to C₁₂ aryl with one or two aromatic six-member rings, substituted or unsubstituted phenyl, substituted or unsubstituted biphenyl, substituted or unsubstituted C₃ to C₂₀ heteroaryl, substituted or unsubstituted C₃ to C₂₀ heteroaryl with one, two or three aromatic six-member rings, substituted or unsubstituted C₇ to C₂₀ heteroaryl with two or three fused aromatic six-member rings or CN,
   wherein the one or more substituents are selected from electron-withdrawing group, CN, F, SF₅, unsubstituted C₁ to C₈ alkyl or D;
wherein the compounds E1 to E7 are excluded:

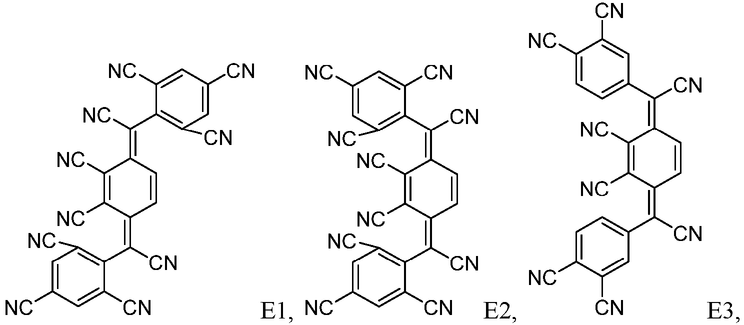

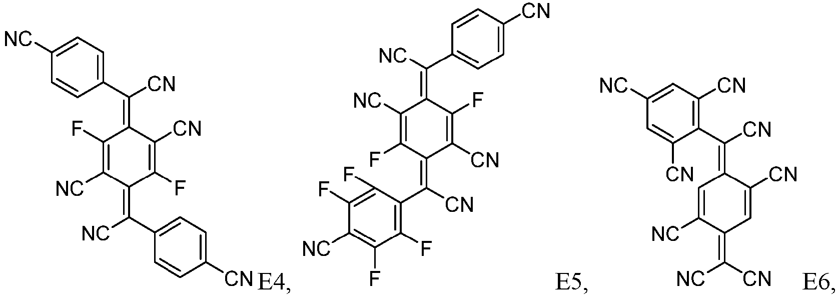

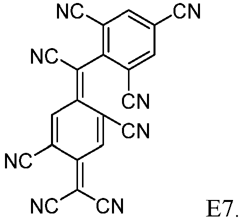

It should be noted that throughout the application and the claims any R¹, R², R³, R⁴, R⁵, R⁶, R⁷, R⁸, R⁹, R¹⁰, R', R", etc. always refer to the same moieties, unless otherwise noted.

In the present specification, when a definition is not otherwise provided, "substituted" refers to one substituted with a deuterium, C₁ to C₈ alkyl.

However, in the present specification "aryl substituted" refers to a substitution with one or more aryl groups, which themselves may be substituted with one or more aryl and/or heteroaryl groups.

Correspondingly, in the present specification "heteroaryl substituted" refers to a substitution with one or more heteroaryl groups, which themselves may be substituted with one or more aryl and/or heteroaryl groups.

In the present specification, when a definition is not otherwise provided, an "alkyl group" refers to a saturated aliphatic hydrocarbyl group. The alkyl group may be a C₁ to C₈ alkyl group. More specifically, the alkyl group may be a C₁ to C₆ alkyl group or a C₁ to C₄ alkyl group. For example, a C₁ to C₄ alkyl group includes 1 to 4 carbons in alkyl chain, and may be selected from methyl, ethyl, propyl, iso-propyl, n-butyl, iso-butyl, sec-butyl, and tert-butyl.

Specific examples of the alkyl group may be a methyl group, an ethyl group, a propyl group, an isopropyl group, a butyl group, an iso-butyl group, a sec-butyl group, a tert-butyl group, a pentyl group, a hexyl group.

The term "hetero" is understood the way that at least one carbon atom, in a structure which may be formed by covalently bound carbon atoms, is replaced by another polyvalent atom. Preferably, the heteroatoms are selected from B, Si, N, P, O, S; more preferably from N, P, O, S.

In the present specification, "aryl group" refers to a hydrocarbyl group which can be created by formal abstraction of one hydrogen atom from an aromatic ring in the corresponding aromatic hydrocarbon. Aromatic hydrocarbon refers to a hydrocarbon which contains at least one aromatic ring or aromatic ring system. Aromatic ring or aromatic ring system refers to a planar ring or ring system of covalently bound carbon atoms, wherein the planar ring or ring system comprises a conjugated system of delocalized electrons fulfilling Hückel's rule. Examples of aryl groups include monocyclic groups like phenyl or tolyl, polycyclic groups which comprise more aromatic rings linked by single bonds, like biphenyl, and polycyclic groups comprising fused rings, like naphthyl or fluoren-2-yl.

Analogously, under heteroaryl, it is especially where suitable understood a group derived by formal abstraction of one ring hydrogen from a heterocyclic aromatic ring in a compound comprising at least one such ring.

The term "six-member ring" is understood to mean a ring formed by 6 atoms. The ring-forming atoms of the "six-member ring" may be bonded to further atoms outside the ring, for example hydrogen atoms.

The term "five-member ring" is understood to mean a ring formed by 5 atoms. The ring-forming atoms of the "five-member ring "may be bonded to further atoms outside the ring, for example hydrogen atoms.

In the present specification, the single bond refers to a direct bond.

In the present specification, an electron-withdrawing group (EWG) is a group that reduces electron density in a molecule through the carbon atom it is bonded to. Typical examples of electron withdrawing groups are halogen, in particular F and Cl, -COR, -COH, -COOR, -COOH, partially fluorinated or perfluorinated aryl, partially fluorinated or perfluorinated heteroaryl, partially fluorinated or perfluorinated carbocyclyl, partially fluorinated or perfluorinated C₂ to C₂₀ heterocyclyl, -NO₂, and -CN.

The term "free of', "does not contain", "does not comprise" does not exclude impurities which may be present in the compounds prior to deposition. Impurities have no technical effect with respect to the object achieved by the present invention.

The term "contacting sandwiched" refers to an arrangement of three layers whereby the layer in the middle is in direct contact with the two adjacent layers.

The terms "light-absorbing layer" and "light absorption layer" are used synonymously.

The terms "light-emitting layer", "light emission layer" and "emission layer" are used synonymously.

The terms "p-type charge generation layer", "p-CGL" and "hole generation layer" are used synonymously.

The terms "n-type charge generation layer", "n-CGL" and "electron generation layer" are used synonymously.

The terms "OLED", "organic light-emitting diode" and "organic light-emitting device" are used synonymously.

The terms "anode", "anode layer" and "anode electrode" are used synonymously.

The terms "cathode", "cathode layer" and "cathode electrode" are used synonymously.

In the specification, hole characteristics refer to an ability to donate an electron to form a hole when an electric field is applied and that a hole formed in the anode may be easily injected into the emission layer and transported in the emission layer due to conductive characteristics according to a highest occupied molecular orbital (HOMO) level.

In addition, electron characteristics refer to an ability to accept an electron when an electric field is applied and that electrons formed in the cathode may be easily injected into the emission layer and transported in the emission layer due to conductive characteristics according to a lowest unoccupied molecular orbital (LUMO) level.

### Disclaimer

According to one embodiment, wherein compounds are excluded having less than three 6 - member aromatic rings.

According to one embodiment, wherein compounds are excluded having more than three 6 - member aromatics rings.

According to one embodiment, wherein compounds are excluded having less than three 6 - member aromatics rings and more than three 6 - member aromatics rings.

According to one embodiment, wherein compounds are excluded having at least one unsubstituted aromatic 6-member ring.

According to one embodiment, wherein compounds are excluded having at least three hetero aromatic 6-member rings.

According to one embodiment, wherein compounds are excluded having at least two hetero aromatic 6-member rings that are connected by a single bond to each other.

According to one embodiment, wherein compounds are excluded that do not consist of three aromatic 6-member rings comprising at least four to twelve F substituents and four to eight CN substituents.

According to one embodiment, wherein compounds are excluded having less than two F substituents or more than twelve F substituents.

According to one embodiment, wherein compounds are excluded having less than four F substituents or more than twelve F substituents.

According to one embodiment, wherein compounds are excluded having less than sixth F substituents or more than twelve F substituents.

According to one embodiment, wherein compounds are excluded having less than sixth CN substituents or more than eight CN substituents.

According to one embodiment, wherein compounds are excluded having at least one Cl substituents.

According to one embodiment, wherein compounds are excluded having no F substituent.

According to one embodiment, wherein compounds are excluded having no CN substituent.

According to one embodiment, wherein compounds are excluded having no F substituent directly bonded to an aromatic 6-member ring.

According to one embodiment, wherein compounds are excluded having no CN substituent directly bonded to an aromatic 6-member ring.

According to one embodiment, wherein compounds are excluded having no F substituent directly bonded to at least two aromatic 6-member ring.

According to one embodiment, wherein compounds are excluded having no CN substituent directly bonded to at least two aromatic 6-member ring.

According to one embodiment, wherein compounds are excluded having no F substituent directly bonded to at least three aromatic 6-member ring.

According to one embodiment, wherein compounds are excluded having no CN substituent directly bonded to at least three aromatic 6-member ring.

According to one embodiment, wherein compounds are excluded having at least one partially or fully halogen substituted alkyl group.

According to one embodiment, wherein compounds are excluded having at least one partially or fully F substituted alkyl group.

According to one embodiment, wherein compounds are excluded having at least one partially fluorinated alkyl group or perfluorinated alkyl group.

According to one embodiment, wherein the compounds E1 to E7 are excluded: 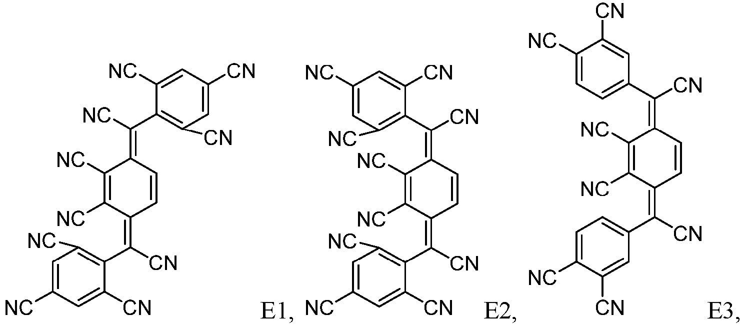 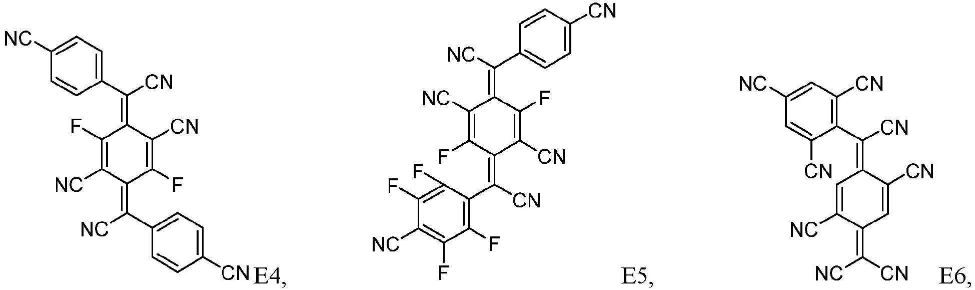 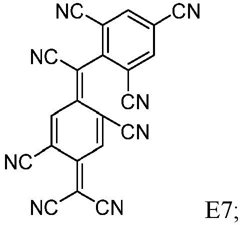 and
wherein the compounds of formulae (I) are excluded comprising the groups Ex-1 to Ex-4, wherein R¹, R², R³, R⁴, R' and R" are selected from Table 1 as follows:

**Table 1**

| group | R¹ | R² | R³ | R⁴ | R' | R" |
|---|---|---|---|---|---|---|
| Ex-1 | CN | CN | H | H | 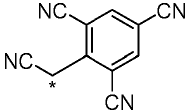 | 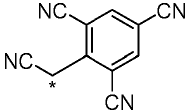 |
| Ex-2 | CN | CN | H | H | 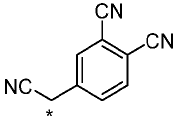 | 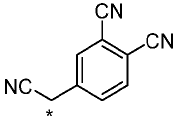 |
| Ex-3 | F | CN | CN | F | 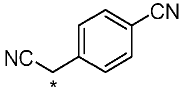 | 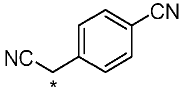 |
| Ex-4 | CN | F | F | CN | 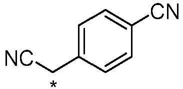 | 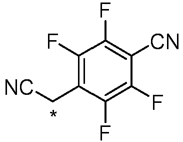 |

According to one embodiment, wherein the compounds E1 to E7 are excluded: 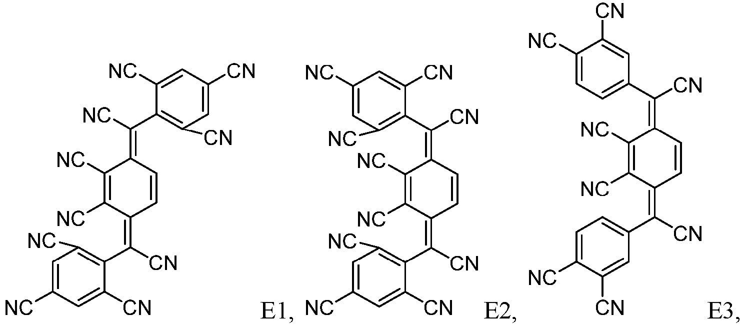 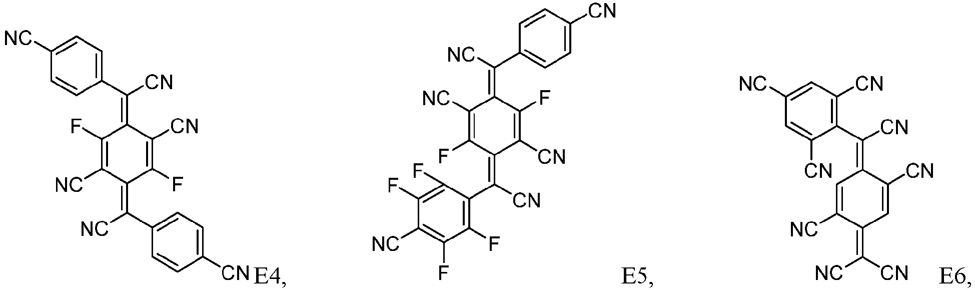 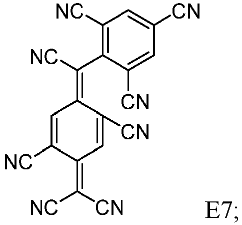 and
wherein the compounds of formulae (I) are excluded comprising the groups Ex-1 to Ex-4, wherein R¹, R², R³, R⁴, R' and R" are selected from Table 1a as follows:

**Table 1a**

| group | R¹ | R² | R³ | R⁴ | R' | R" |
|---|---|---|---|---|---|---|
| Ex-1 | CN | CN | H | H | 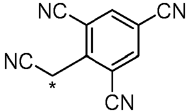 | 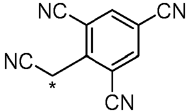 |
| Ex-2 | CN | CN | H | H | 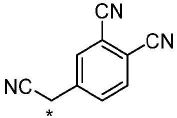 | 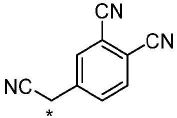 |
| Ex-3 | F | CN | CN | F | 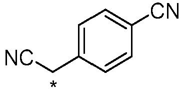 | 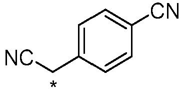 |
| Ex-4 | CN | F | F | CN | 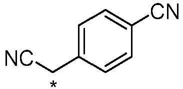 | 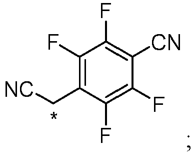 |

wherein compounds are excluded having at least one partially fluorinated alkyl group or perfluorinated alkyl group.

According to one embodiment, wherein the compounds E1 to E7 are excluded: 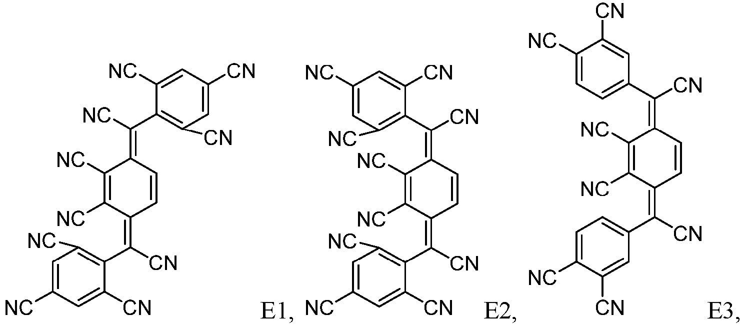 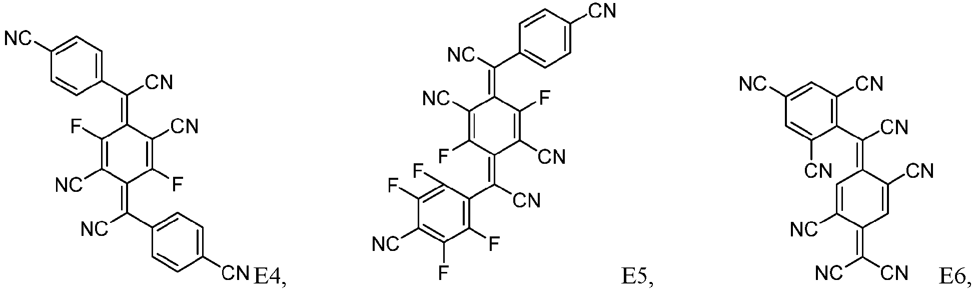 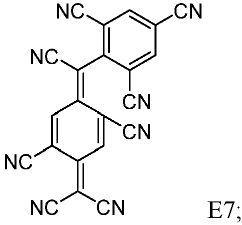 and wherein the compounds of formulae (I) are excluded comprising the groups Ex-1 to Ex-8, wherein R¹, R², R³, R⁴, R' and R" are selected from Table 2 as follows:

| group | R¹ | R² | R³ | R⁴ | R' | R" |
|---|---|---|---|---|---|---|
| Ex-1 | CN | CN | H | H | 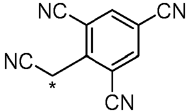 | 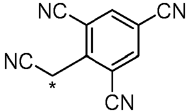 |
| Ex-2 | CN | CN | H | H | 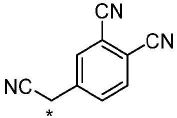 | 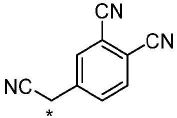 |
| Ex-3 | F | CN | CN | F | 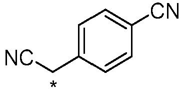 | 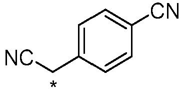 |
| Ex-4 | CN | F | F | CN | 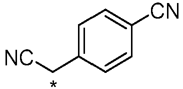 | 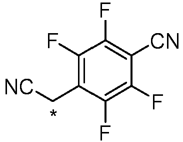 |
| Ex-5 | CN | H | H | CN | 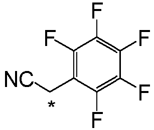 | 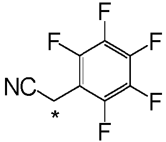 |
| Ex-6 | CN | F | F | CN | 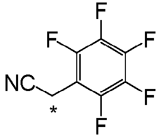 | 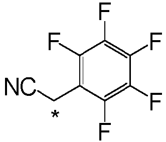 |
| Ex-7 | CN | H | H | CN | 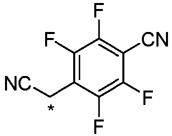 | 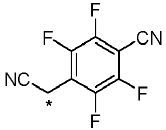 |
| Ex-8 | CN | F | F | CN | 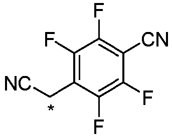 | 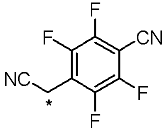 |

According to one embodiment, wherein the compounds E1 to E7 are excluded: 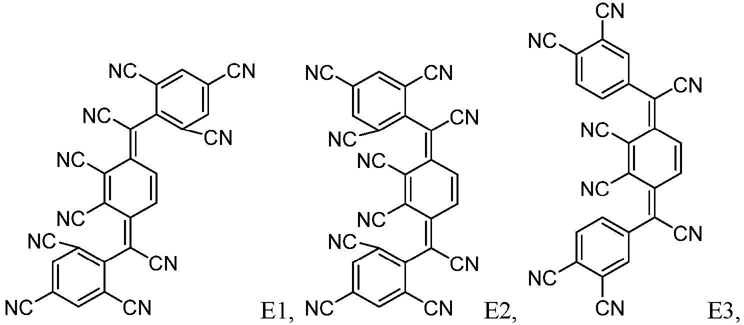 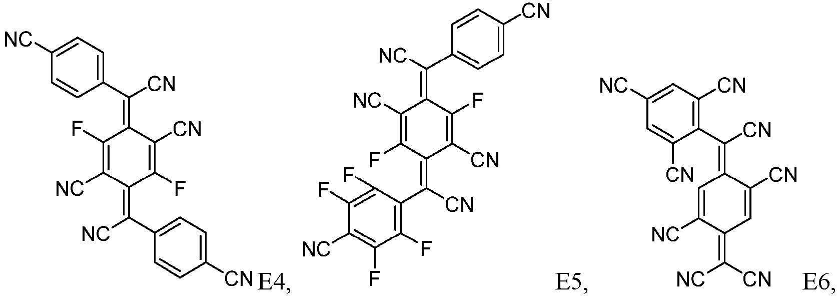 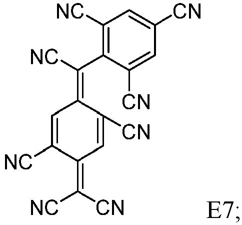 and wherein the compounds of formulae (I) are excluded comprising the groups Ex-1 to Ex-8, wherein R¹, R², R³, R⁴, R' and R" are selected from Table 3 as follows:

**Table 3**

| group | R¹ | R² | R³ | R⁴ | R' | R" |
|---|---|---|---|---|---|---|
| Ex-1 | CN | CN | H | H | 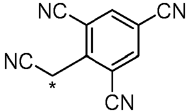 | 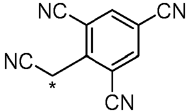 |
| Ex-2 | CN | CN | H | H | 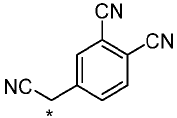 | 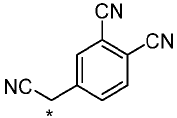 |
| Ex-3 | F | CN | CN | F | 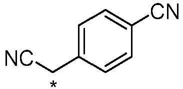 | 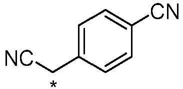 |
| Ex-4 | CN | F | F | CN | 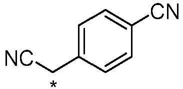 | 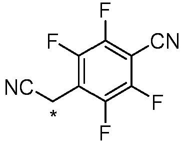 |
| Ex-5 | CN | H | H | CN | 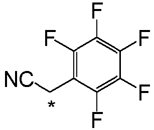 | 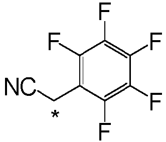 |
| Ex-6 | CN | F | F | CN | 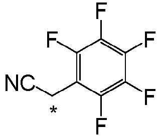 | 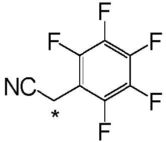 |
| Ex-7 | CN | H | H | CN | 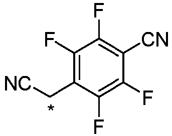 | 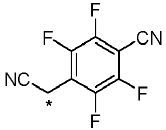 |
| Ex-8 | CN | F | F | CN | 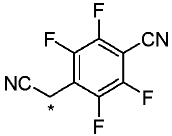 | 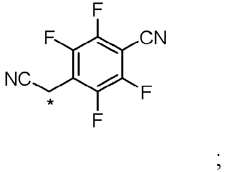 and |

wherein compounds are excluded having at least one partially fluorinated alkyl group or perfluorinated alkyl group.

### Technical problem to be solved

The technical objective of the present invention is the provision of a compound, a semiconductor layers, an organic light-emitting device, and a display device with improved properties.

The provided compound, semiconductor layer, organic light-emitting device, and display device have or lead to a good performance of an organic light-emitting device such as good current efficiency, external quantum efficiency good operational voltage, and good stability such as lifetime and good rise of the operational voltage over time.

In particular, the provided compound, semiconductor layer, organic light-emitting device and display device have or lead to a high sheet resistance; and thereby, the display device may exhibit a good color purity and/or good resolution.

Moreover, the provided compounds exhibit improved thermal properties.

### Advantageous Effects

Surprisingly, it was found that the organic compound of the present invention according to formula (I) solves the problem underlying the present invention by enabling devices in various aspects superior over the organic electroluminescent devices known in the art, with respect to operating voltage, efficiency and/or lifetime, and in particular with respect to operating voltage, efficiency and voltage stability over time. It has be further surprisingly found that compounds of formula (I) may have improved LUMO energy, improved dipole moment and/or improved thermal properties compared to comparative examples, in particular improved thermal stability and/or processing properties at elevated temperatures.

Without being bound by theory, the thermal properties may be improved when R' in formula (I) is selected in the claimed range. Thereby, the thermal stability, as determined by TGA5%, and volatility, as determined by the rate onset temperature, may be in the range required for mass production.

Surprisingly, it was found that the LUMO energy may be in the range required for efficient hole injection and/or hole generation, when the R' is selected in the claimed range.

### Compound of formula (I)

According to one embodiment, the compound is represented by formula (I): 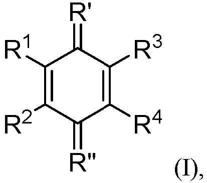 wherein
R¹, R², R³ and R⁴ are independently selected from the group comprising H, D, F or CN;
wherein
two R¹, R², R³ and R⁴ are selected from CN and two R¹, R², R³ and R⁴ are selected from H, D or F;
R' is selected from formula (II): 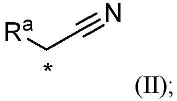 R^{a} is selected from formula (III): 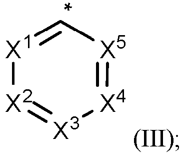 wherein the asterisks "*" denotes the binding position;
wherein
X¹ is selected from CR⁶ or N;
X² is selected from CR⁷ or N;
X³ is selected from CR⁸ or N;
X⁴ is selected from CR⁹ or N;
X⁵ is selected from CR¹⁰ or N;
wherein
R⁶, R⁷, R⁸, R⁹ and R¹⁰ are independently selected from CN, unsubstituted C₁ to C₈ alkyl, substituted or unsubstituted C₆ to C₁₈ aryl, substituted or unsubstituted C₃ to C₁₈ heteroaryl F, SF₅, D or H,
wherein the one or more substituent on R⁶, R⁷, R⁸, R⁹ and R¹⁰ are independently selected from CN, F, SF₅, D;
wherein
at least one X¹ to X⁵ are independently selected from CH or CD and at least one R⁶ to R¹⁰ is CN or F;
R" is selected from formula (IV): 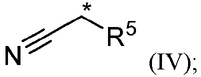 wherein the asterisks "*" denotes the binding position;
wherein
R⁵ is selected from substituted or unsubstituted C₆ to C₁₉ aryl, substituted or unsubstituted C₆ to C₁₉ aryl with one, two or three aromatic six-member rings, substituted or unsubstituted C₉ to C₁₉ aryl with two or three fused aromatic six-member rings, substituted or unsubstituted C₆ to C₁₂ aryl with one or two aromatic six-member rings, substituted or unsubstituted phenyl, substituted or unsubstituted biphenyl, substituted or unsubstituted C₃ to C₂₀ heteroaryl, substituted or unsubstituted C₃ to C₂₀ heteroaryl with one, two or three aromatic six-member rings, substituted or unsubstituted C₇ to C₂₀ heteroaryl with two or three fused aromatic six-member rings or CN,
wherein the one or more substituents are selected from electron-withdrawing group, CN, F, SF₅, unsubstituted C₁ to C₈ alkyl or D;
wherein the compounds E1 to E7 are excluded: 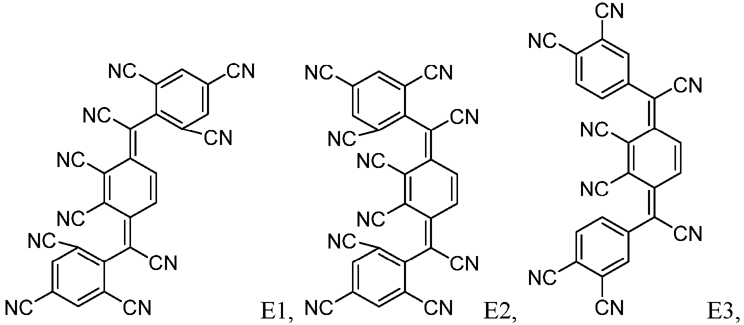 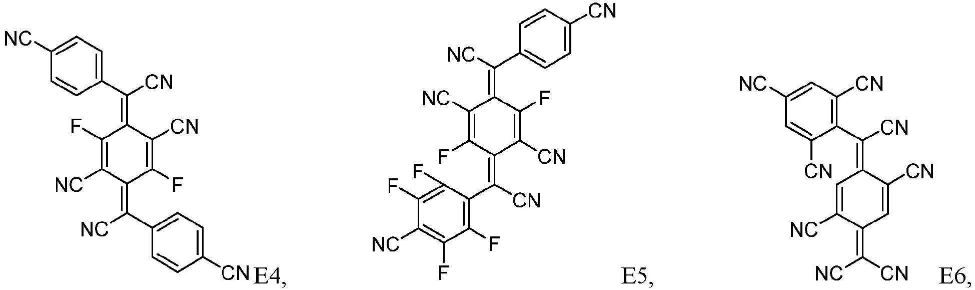 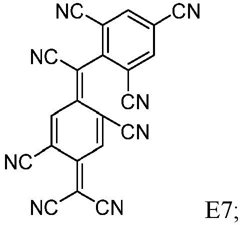 and
wherein the compounds of formulae (I) are excluded comprising the groups Ex-1 to Ex-8, wherein R¹, R², R³, R⁴, R' and R" are selected from Table 3 as follows:

**Table 3**

| group | R¹ | R² | R³ | R⁴ | R' | R" |
|---|---|---|---|---|---|---|
| Ex-1 | CN | CN | H | H | 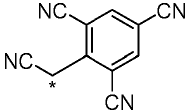 | 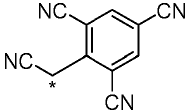 |
| Ex-2 | CN | CN | H | H | 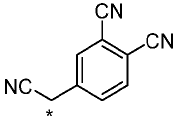 | 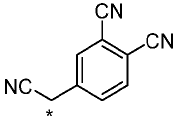 |
| Ex-3 | F | CN | CN | F | 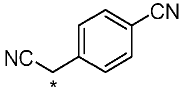 | 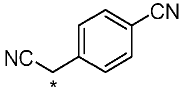 |
| Ex-4 | CN | F | F | CN | 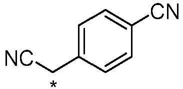 | 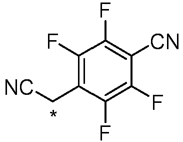 |
| Ex-5 | CN | H | H | CN | 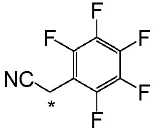 | 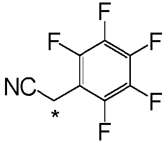 |
| Ex-6 | CN | F | F | CN | 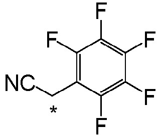 | 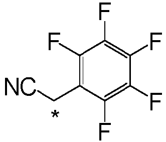 |
| Ex-7 | CN | H | H | CN | 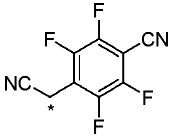 | 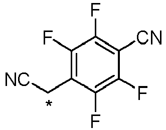 |
| Ex-8 | CN | F | F | CN | 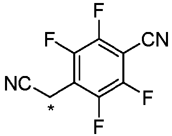 | 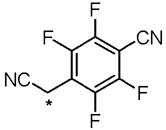 |

According to one embodiment, the compound is represented by formula (I): 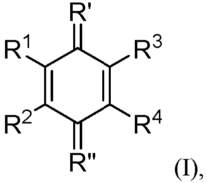 wherein
R¹, R², R³ and R⁴ are independently selected from the group comprising H, D, F or CN;
wherein
   two R¹, R², R³ and R⁴ are selected from CN and two R¹, R², R³ and R⁴ are selected from H, D or F;
R' is selected from formula (II): 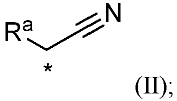
R^{a} is selected from formula (III): 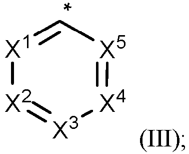
wherein the asterisks "*" denotes the binding position;
wherein
X¹ is selected from CR⁶ or N;
X² is selected from CR⁷ or N;
X³ is selected from CR⁸ or N;
X⁴ is selected from CR⁹ or N;
X⁵ is selected from CR¹⁰ or N;
wherein
   R⁶, R⁷, R⁸, R⁹ and R¹⁰ are independently selected from CN, unsubstituted C₁ to C₈ alkyl, substituted or unsubstituted C₆ to C₁₈ aryl, substituted or unsubstituted C₃ to C₁₈ heteroaryl F, SF₅, D or H,
   wherein the one or more substituent on R⁶, R⁷, R⁸, R⁹ and R¹⁰ are independently selected from CN, F, SF₅, D;
wherein
   at least one X¹ to X⁵ are independently selected from CH or CD and at least one R⁶ to R¹⁰ is CN or F;
R" is selected from formula (IV): 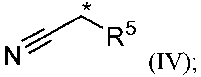
wherein the asterisks "*" denotes the binding position;
wherein
   R⁵ is selected from substituted or unsubstituted C₆ to C₁₉ aryl, substituted or unsubstituted C₆ to C₁₉ aryl with one, two or three aromatic six-member rings, substituted or unsubstituted C₉ to C₁₉ aryl with two or three fused aromatic six-member rings, substituted or unsubstituted C₆ to C₁₂ aryl with one or two aromatic six-member rings, substituted or unsubstituted phenyl, substituted or unsubstituted biphenyl, substituted or unsubstituted C₃ to C₂₀ heteroaryl, substituted or unsubstituted C₃ to C₂₀ heteroaryl with one, two or three aromatic six-member rings, substituted or unsubstituted C₇ to C₂₀ heteroaryl with two or three fused aromatic six-member rings or CN,
   wherein the one or more substituents are selected from electron-withdrawing group, CN, F, SF₅, unsubstituted C₁ to C₈ alkyl or D;
wherein the compounds E1 to E7 are excluded: 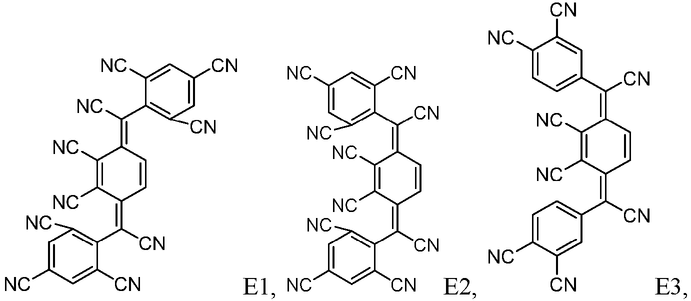 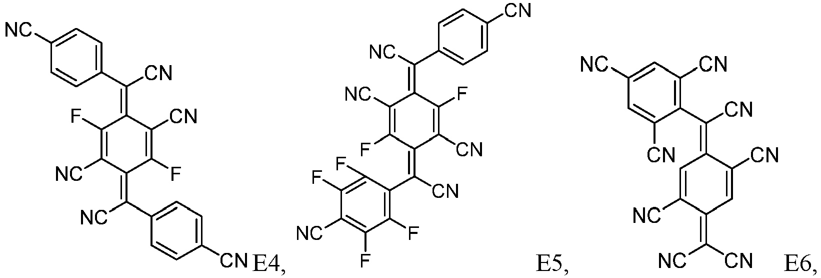 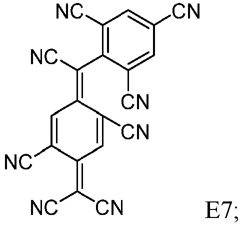 and wherein the compounds of formulae (I) are excluded comprising the groups Ex-1 to Ex-8, wherein R¹, R², R³, R⁴, R' and R" are selected from Table 3 as follows:

**Table 3**

| group | R¹ | R² | R³ | R⁴ | R' | R" |
|---|---|---|---|---|---|---|
| Ex-1 | CN | CN | H | H | 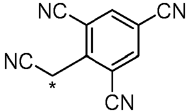 | 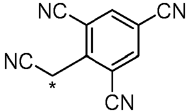 |
| Ex-2 | CN | CN | H | H | 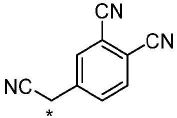 | 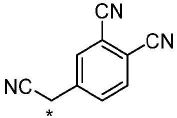 |
| Ex-3 | F | CN | CN | F | 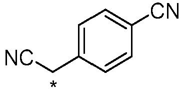 | 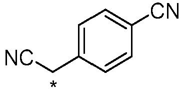 |
| Ex-4 | CN | F | F | CN | 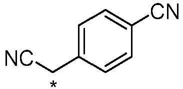 | 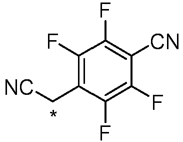 |
| Ex-5 | CN | H | H | CN | 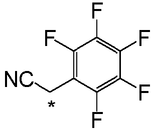 | 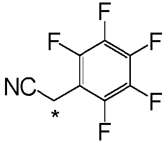 |
| Ex-6 | CN | F | F | CN | 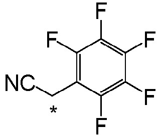 | 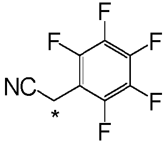 |
| Ex-7 | CN | H | H | CN | 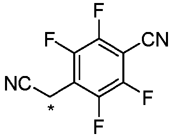 | 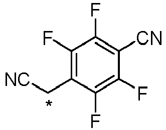 |
| Ex-8 | CN | F | F | CN | 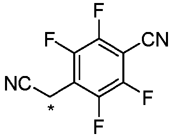 | 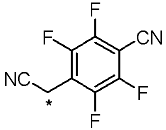 |

According to one embodiment, the compound is represented by formula (I): 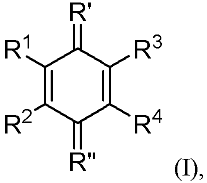 wherein
R¹, R², R³ and R⁴ are independently selected from the group comprising H, D, F or CN; wherein
two R¹, R², R³ and R⁴ are selected from CN and two R¹, R², R³ and R⁴ are selected from H, D or F;
R' is selected from formula (II): 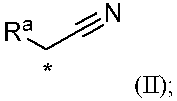 R^{a} is selected from formula (III): 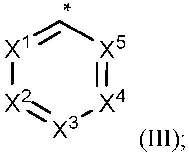 wherein the asterisks "*" denotes the binding position;
wherein
X¹ is selected from CR⁶ or N;
X² is selected from CR⁷ or N;
X³ is selected from CR⁸ or N;
X⁴ is selected from CR⁹ or N;
X⁵ is selected from CR¹⁰ or N;
wherein
R⁶, R⁷, R⁸, R⁹ and R¹⁰ are independently selected from CN, unsubstituted C₁ to C₈ alkyl, substituted or unsubstituted C₆ to C₁₈ aryl, substituted or unsubstituted C₃ to C₁₈ heteroaryl F, SF₅, D or H,
wherein the one or more substituent on R⁶, R⁷, R⁸, R⁹ and R¹⁰ are independently selected from CN, F, SF₅, D;
wherein
at least one X¹ to X⁵ are independently selected from CH or CD and at least one R⁶ to R¹⁰ is CN or F;
R" is selected from formula (IV): 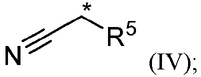 wherein the asterisks "*" denotes the binding position;
wherein
R⁵ is selected from substituted or unsubstituted C₆ to C₁₉ aryl, substituted or unsubstituted C₆ to C₁₉ aryl with one, two or three aromatic six-member rings, substituted or unsubstituted C₉ to C₁₉ aryl with two or three fused aromatic six-member rings, substituted or unsubstituted C₆ to C₁₂ aryl with one or two aromatic six-member rings, substituted or unsubstituted phenyl, substituted or unsubstituted biphenyl, substituted or unsubstituted C₃ to C₂₀ heteroaryl, substituted or unsubstituted C₃ to C₂₀ heteroaryl with one, two or three aromatic six-member rings, substituted or unsubstituted C₇ to C₂₀ heteroaryl with two or three fused aromatic six-member rings or CN, wherein the one or more substituents are selected from electron-withdrawing group,
CN, F, SF₅, unsubstituted C₁ to C₈ alkyl or D;
wherein the compounds E1 to E7 are excluded: 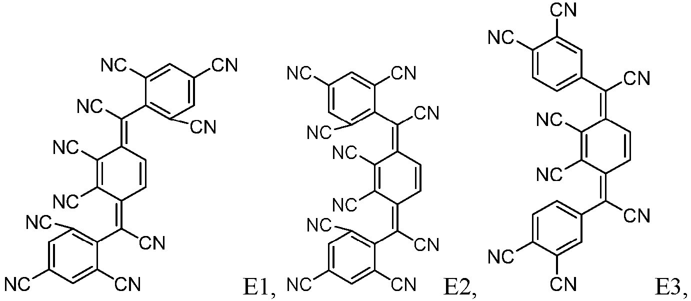 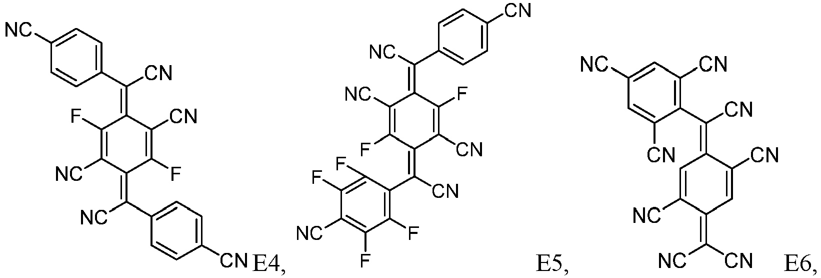 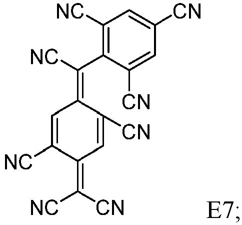 and wherein the compounds of formulae (I) are excluded comprising the groups Ex-1 to Ex-8, wherein R¹, R², R³, R⁴, R' and R" are selected from Table 3 as follows:

**Table 3**

| group | R¹ | R² | R³ | R⁴ | R' | R" |
|---|---|---|---|---|---|---|
| Ex-1 | CN | CN | H | H | 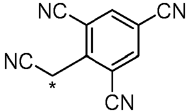 | 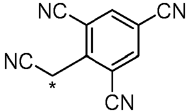 |
| Ex-2 | CN | CN | H | H | 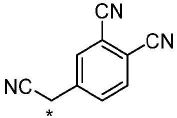 | 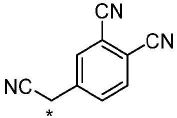 |
| Ex-3 | F | CN | CN | F | 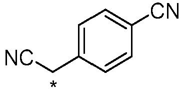 | 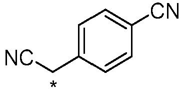 |
| Ex-4 | CN | F | F | CN | 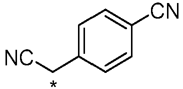 | 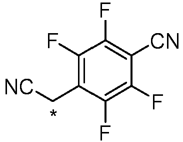 |
| Ex-5 | CN | H | H | CN | 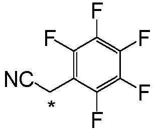 | 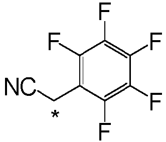 |
| Ex-6 | CN | F | F | CN | 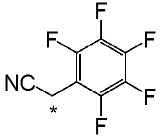 | 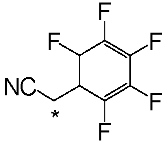 |
| Ex-7 | CN | H | H | CN | 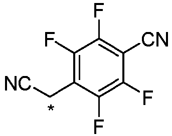 | 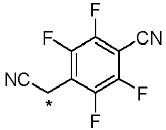 |
| Ex-8 | CN | F | F | CN | 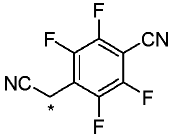 | 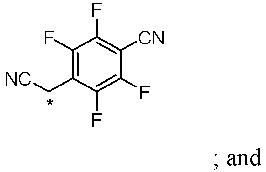 |

wherein compounds are excluded having at least one partially fluorinated alkyl group or perfluorinated alkyl group.

### R¹, R², R³ and R⁴

According to one embodiment, wherein R¹, R², R³ and R⁴ are independently selected from the group comprising F or CN; wherein two R¹, R², R³ and R⁴ are selected from CN and two R¹, R², R³ and R⁴ are selected from F.

### Formulae (V) to (XVIII)

According to one embodiment, wherein the compound of formula (I) is selected from formulae (V) to (XVIII):

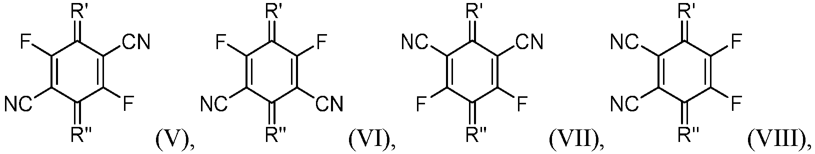

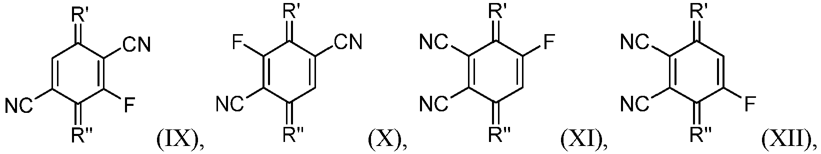

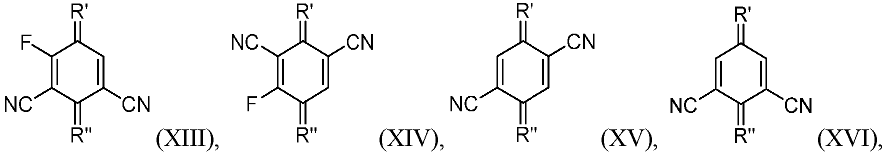

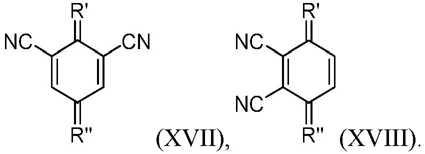

According to a preferred embodiment, wherein the compound of formula (I) is selected from formulae (IV) and (XIV):

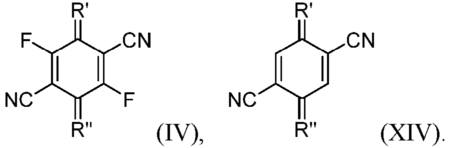

According to one embodiment, the compound of formula (I) is selected from formulae (IV) and (XIV): 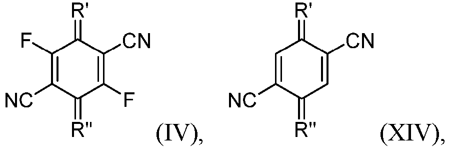 wherein
R' is selected from formula (II) 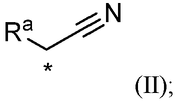
R^{a} is selected from formula (III) 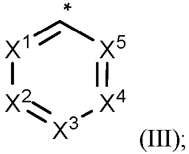
wherein the asterisks "*" denotes the binding position;
wherein
X¹ is selected from CR⁶ or N;
X² is selected from CR⁷ or N;
X³ is selected from CR⁸ or N;
X⁴ is selected from CR⁹ or N;
X⁵ is selected from CR¹⁰ or N;
wherein
   R⁶, R⁷, R⁸, R⁹ and R¹⁰ are independently selected from CN, unsubstituted C₁ to C₈ alkyl, substituted or unsubstituted C₆ to C₁₈ aryl, substituted or unsubstituted C₃ to C₁₈ heteroaryl F, SF₅, D or H,
   wherein the one or more substituent on R⁶, R⁷, R⁸, R⁹ and R¹⁰ are independently selected from CN, F, SF₅, D;
wherein
   at least one X¹ to X⁵ are independently selected from CH or CD and at least one R⁶ to R¹⁰ is CN or F;
R" is selected from formula (IV): 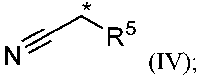
wherein the asterisks "*" denotes the binding position;
wherein
   R⁵ is selected from substituted or unsubstituted C₆ to C₁₉ aryl, substituted or unsubstituted C₆ to C₁₉ aryl with one, two or three aromatic six-member rings, substituted or unsubstituted C₉ to C₁₉ aryl with two or three fused aromatic six-member rings, substituted or unsubstituted C₆ to C₁₂ aryl with one or two aromatic six-member rings, substituted or unsubstituted phenyl, substituted or unsubstituted biphenyl, substituted or unsubstituted C₃ to C₂₀ heteroaryl, substituted or unsubstituted C₃ to C₂₀ heteroaryl with one, two or three aromatic six-member rings, substituted or unsubstituted C₇ to C₂₀ heteroaryl with two or three fused aromatic six-member rings or CN,
   wherein the one or more substituents are selected from electron-withdrawing group, CN, F, SF₅, unsubstituted C₁ to C₈ alkyl or D;
wherein the compounds E4 to E7 are excluded:

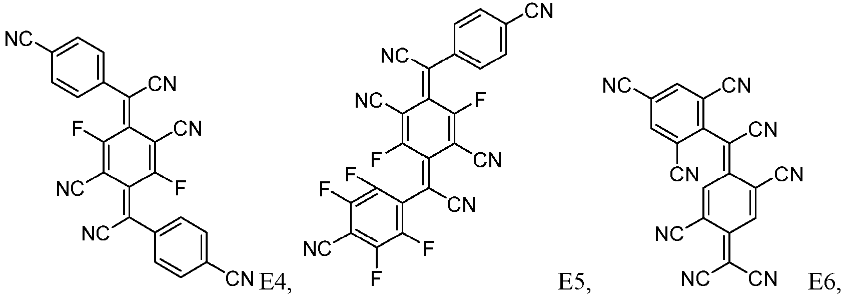

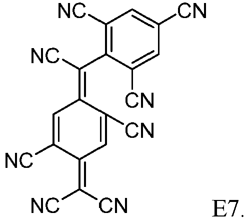

According to a more preferred embodiment, wherein the compound of formula (I) is selected from formula (IV):

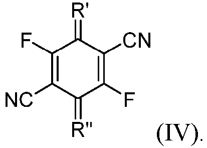

According to one embodiment, the compound of formula (I) is selected from formula (IV): 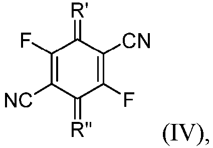 wherein
R' is selected from formula (II): 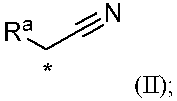
R^{a} is selected from formula (III): 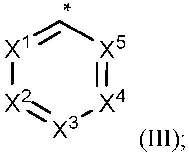
wherein the asterisks "*" denotes the binding position;
wherein
X¹ is selected from CR⁶ or N;
X² is selected from CR⁷ or N;
X³ is selected from CR⁸ or N;
X⁴ is selected from CR⁹ or N;
X⁵ is selected from CR¹⁰ or N;
wherein
   R⁶, R⁷, R⁸, R⁹ and R¹⁰ are independently selected from CN, unsubstituted C₁ to C₈ alkyl, substituted or unsubstituted C₆ to C₁₈ aryl, substituted or unsubstituted C₃ to C₁₈ heteroaryl F, SF₅, D or H,
   wherein the one or more substituent on R⁶, R⁷, R⁸, R⁹ and R¹⁰ are independently selected from CN, F, SF₅, D;
wherein
   at least one X¹ to X⁵ are independently selected from CH or CD and at least one R⁶ to R¹⁰ is CN or F;
R" is selected from formula (IV): 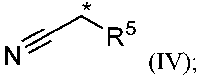
wherein the asterisks "*" denotes the binding position;
wherein
   R⁵ is selected from substituted or unsubstituted C₆ to C₁₉ aryl, substituted or unsubstituted C₆ to C₁₉ aryl with one, two or three aromatic six-member rings, substituted or unsubstituted C₉ to C₁₉ aryl with two or three fused aromatic six-member rings, substituted or unsubstituted C₆ to C₁₂ aryl with one or two aromatic six-member rings, substituted or unsubstituted phenyl, substituted or unsubstituted biphenyl, substituted or unsubstituted C₃ to C₂₀ heteroaryl, substituted or unsubstituted C₃ to C₂₀ heteroaryl with one, two or three aromatic six-member rings, substituted or unsubstituted C₇ to C₂₀ heteroaryl with two or three fused aromatic six-member rings or CN,
   wherein the one or more substituents are selected from electron-withdrawing group, CN, F, SF₅, unsubstituted C₁ to C₈ alkyl or D;
wherein the compounds E4 an E5 are excluded:

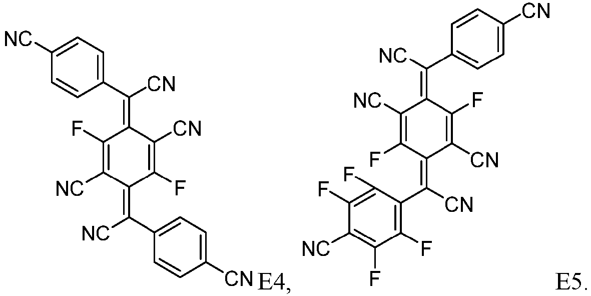

### Isomers

According to an embodiment, wherein the compound of formula (I) is selected from formulae (XIX) to (XXX):

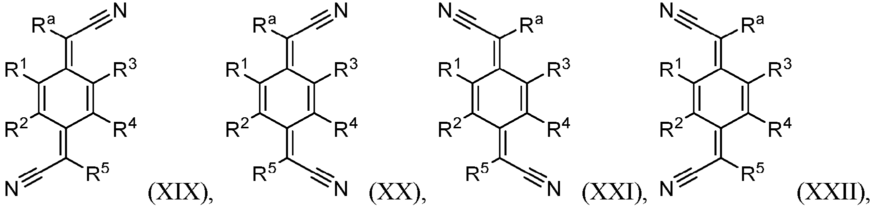

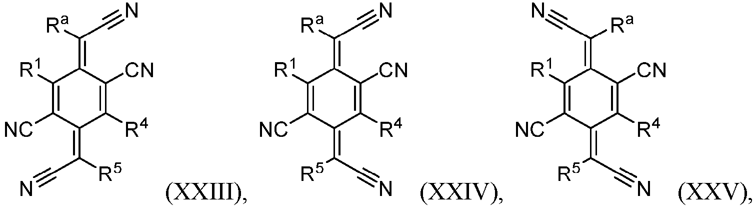

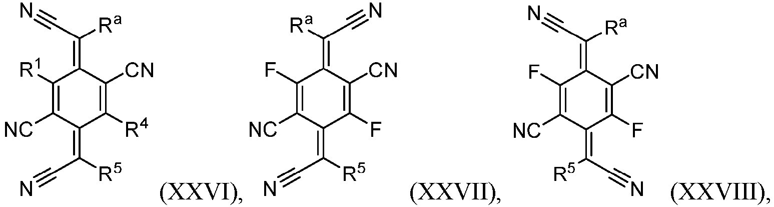

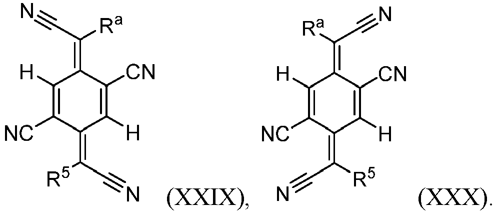

According to an embodiment, wherein the compound of formula (I) is preferably selected from formulae (XIX) to (XXII):

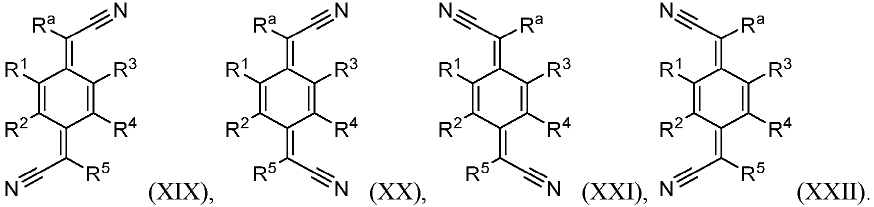

According to an embodiment, wherein the compound of formula (I) is further preferred selected from formulae (XXIII) to (XXVI):

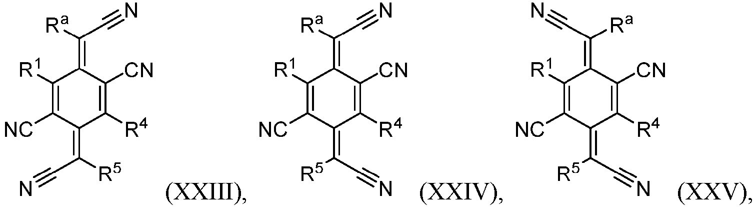

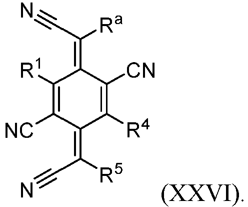

According to an embodiment, wherein the compound of formula (I) is selected from formulae (XXVII) to (XXX):

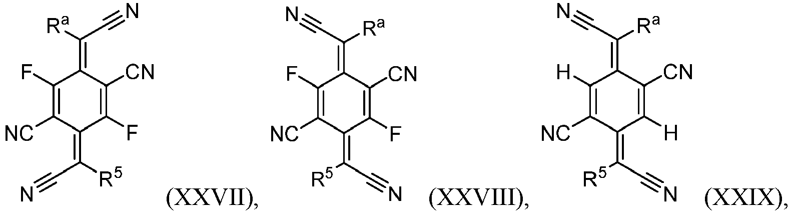

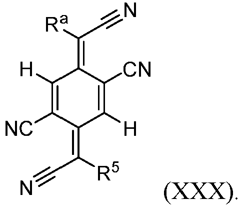

According to an embodiment, wherein the compound of formula (I) is selected from formulae (XXVII) to (XXVIII)

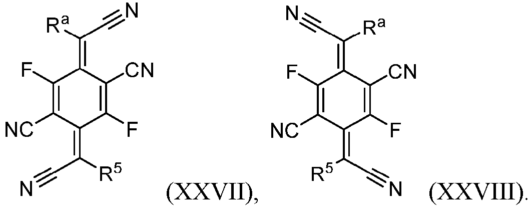

### X¹ to X⁵

According to an embodiment, wherein at least one X¹ to X⁵ is selected from the group comprising CH or CD.

According to an embodiment, wherein at least one to at most three X¹ to X⁵ are independently selected from CH or CD.

According to an embodiment, wherein at least one to at most two X¹ to X⁵ are independently selected from CH or CD.

According to an embodiment, wherein one to three X¹ to X⁵ are selected from CH or CD. According to an embodiment, wherein ≥ 1 and ≤ 3 of X¹ to X⁵ are selected from CH or CD. According to an embodiment, wherein one to two X¹ to X⁵ are selected from CH or CD. According to an embodiment, wherein ≥ 1 and ≤ 2 of X¹ to X⁵ are selected from CH or CD.

According to an embodiment, wherein additionally at least one X¹ to X⁵ is CCN or N.

According to an embodiment, wherein at least one X¹ to X⁵ is CCN or N.

Additionally ≥ 0 and ≤ 3 of X¹, X², X³, X⁴ and X⁵ are selected from N.

According to an embodiment, wherein ≥ 0 and ≤ 3 of X¹, X², X³, X⁴ and X⁵ are selected from N.

### R⁶ to R¹⁰

According to an embodiment, wherein at least one of R⁶ to R¹⁰ is CN or F

According to an embodiment, wherein at least two R⁶ to R¹⁰ is CN or F. The volatility of the compound of formula (I) may be further adjusted.

According to an embodiment, wherein at least three of R⁶ to R¹⁰ is CN or F. The volatility of the compound of formula (I) may be further adjusted.

According to an embodiment, wherein at least one of R⁶ to R¹⁰ is CN. The volatility of the compound of formula (I) may be further adjusted.

According to an embodiment, wherein at least one of R⁶ to R¹⁰ is F. The volatility of the compound of formula (I) may be further adjusted in particular the volatility may be reduced.

According to an embodiment, wherein at least two of R⁶ to R¹⁰ is F. The volatility of the compound of formula (I) may be further adjusted in particular the volatility may be reduced.

According to an embodiment, wherein at least three of R⁶ to R¹⁰ is F. The volatility of the compound of formula (I) can be further adjusted in particular the volatility can be reduced.

According to an embodiment, wherein R⁶, R⁷, R⁸, R⁹ and R¹⁰ are independently selected from CN, F, SF₅, D or H.

### X¹ to X⁵ and R⁶ to R¹⁰

According to an embodiment, wherein at least one X¹ to X⁵ is CH or CD, and at least one of R⁶ to R¹⁰ is CN or F.

According to an embodiment, wherein at least one X¹ to X⁵ are independently selected from CH, or CD; and at least two R⁶ to R¹⁰ is CN or F. The volatility of the compound of formula (I) may be further adjusted.

According to an embodiment, wherein at least one X¹ to X⁵ are independently selected from CH or CD, and at least two of R⁶ to R¹⁰ are independently selected from CN or F; preferably wherein at least one X¹ to X⁵ are independently selected from CH or CD, and at least three of R⁶ to R¹⁰ are independently selected from CN or F; also preferred at least two to three X¹ to X⁵ are independently selected from CH or CD, and at least one R⁶ to R¹⁰ are independently selected from CN or F; furthermore preferred wherein at least one to at most three X¹ to X⁵ are independently selected from CH or CD, and at least two R⁶ to R¹⁰ are independently selected from CN or F; additionally preferred wherein at least one to two X¹ to X⁵ are independently selected from CH or CD, and at least one R⁶ to R¹⁰are independently selected from CN or F; and moreover preferred at least one to at most two X¹ to X⁵ are independently selected from CH or CD, and at least two R⁶ to R¹⁰ are independently selected from CN or F.

According to an embodiment, wherein at least one X¹ to X⁵ are independently selected from CH, or CD; and at least three of R⁶ to R¹⁰ is CN or F. The volatility of the compound of formula (I) may be further adjusted.

According to an embodiment, wherein at least one to at most three X¹ to X⁵ are independently selected from CH, or CD; and at least one R⁶ to R¹⁰ is CN or F.

According to an embodiment, wherein at least one to at most three X¹ to X⁵ are independently selected from CH, or CD; and at least two R⁶ to R¹⁰ is CN or F.

According to an embodiment, wherein at least one to at most two X¹ to X⁵ are independently selected from CH, or CD; and at least one R⁶ to R¹⁰ is CN or F.

According to an embodiment, wherein at least one to at most two X¹ to X⁵ are independently selected from CH, or CD; and at least two R⁶ to R¹⁰ is CN or F.

According to an embodiment, wherein at least one X¹ to X⁵ are independently selected from CH, or CD; and at least one of R⁶ to R¹⁰ is CN.

According to an embodiment, wherein at least one to at most three X¹ to X⁵ are independently selected from CH, or CD; and at least one of R⁶ to R¹⁰ is CN.

According to an embodiment, wherein at least one to at most two X¹ to X⁵ are independently selected from CH, or CD; and at least one of R⁶ to R¹⁰ is CN.

According to an embodiment, wherein at least one X¹ to X⁵ are independently selected from CH, or CD; and at least one of R⁶ to R¹⁰ is F. The volatility of the compound of formula (I) may be further adjusted.

According to an embodiment, wherein at least one X¹ to X⁵ are independently selected from CH, or CD; and at least two of R⁶ to R¹⁰ is F. The volatility of the compound of formula (I) may be further adjusted.

According to an embodiment, wherein at least one X¹ to X⁵ are independently selected from CH, or CD; and at least three of R⁶ to R¹⁰ is F. The volatility of the compound of formula (I) can be further adjusted.

According to an embodiment, wherein at least one to at most three X¹ to X⁵ are independently selected from CH, or CD; and at least one of R⁶ to R¹⁰ is F. The volatility of the compound of formula (I) may be further adjusted.

According to an embodiment, wherein at least one to at most three X¹ to X⁵ are independently selected from CH, or CD; and at least two of R¹ to R⁵ is F. The volatility of the compound of formula (I) may be further adjusted.

According to an embodiment, wherein at least one to at most two X¹ to X⁵ are independently selected from CH, or CD; and at least one of R¹ to R⁵ is F. The volatility of the compound of formula (I) may be further adjusted.

According to an embodiment, wherein at least one to at most two X¹ to X⁵ are independently selected from CH, or CD; and at least two of R¹ to R⁵ is F. The volatility of the compound of formula (I) may be further adjusted.

### Combination of X¹ to X⁵

According to an embodiment, wherein at least one X¹ to X⁵ is CH or CD, and at least one of X¹ to X⁵ is CCN or N.

According to an embodiment, wherein at least one X¹ to X⁵ are independently selected from CH or CD, and at least one of X¹ to X⁵ are independently selected from CCN or N.

According to an embodiment, wherein at least one to at most three X¹ to X⁵ are independently selected from CH or CD, and at least one of X¹ to X⁵ are independently selected from CCN or N.

According to an embodiment, wherein one to three X¹ to X⁵ are selected from CH or CD, and at least one of X¹ to X⁵ are independently selected from CCN or N.

According to an embodiment, wherein ≥ 1 and ≤ 3 of X¹ to X⁵ are selected from CH or CD, and at least one of X¹ to X⁵ are independently selected from CCN or N.

According to an embodiment, wherein at least one to at most two X¹ to X⁵ are independently selected from CH or CD, and at least one of X¹ to X⁵ are independently selected from CCN or N.

According to an embodiment, wherein one to two X¹ to X⁵ are selected from CH or CD, and at least one of X¹ to X⁵ are independently selected from CCN or N.

According to an embodiment, wherein ≥ 1 and ≤ 2 of X¹ to X⁵ are selected from CH or CD, and at least one of X¹ to X⁵ are independently selected from CCN or N.

According to an embodiment, wherein at least one X¹ to X⁵ are independently selected from CH or CD, and at least one of X¹ to X⁵ are independently selected from CCN or N; further preferred wherein at least one to at most three X¹ to X⁵ are independently selected from CH or CD, and at least one of X¹ to X⁵ are independently selected from CCN or N; further more preferred wherein one to three X¹ to X⁵ are independently selected from CH or CD, and at least one of X¹ to X⁵ are independently selected from CCN or N; and additionally preferred wherein at least one to at most two X¹ to X⁵ are independently selected from CH or CD, and at least one of X¹ to X⁵ are independently selected from CCN or N.

### Combination of X¹ to X⁵ and R⁶ to R¹⁰

According to an embodiment, wherein at least one of X¹ to X⁵ are independently selected from CCN or N, and at least one R¹ to R⁵ is CN or F.

According to an embodiment, wherein at least one of X¹ to X⁵ are independently selected from CCN or N, and at least two R¹ to R⁵ is CN or F.

According to an embodiment, wherein at least one of X¹ to X⁵ are independently selected from CCN or N, and at least one of R¹ to R⁵ is F.

According to an embodiment, wherein at least one of X¹ to X⁵ are independently selected from CCN or N, and at least two of R¹ to R⁵ is F.

According to an embodiment, wherein at least one of X¹ to X⁵ are independently selected from CCN or N, and at least one R⁶ to R¹⁰ are independently selected from CN or F; preferably wherein at least one of X¹ to X⁵ are independently selected from CCN or N, and at least two R⁶ to R¹⁰ are independently selected from CN or F; further preferred wherein at least one of X¹ to X⁵ are independently selected from CCN or N, and at least one of R⁶ to R¹⁰ is F; and additionally preferred wherein at least one of X¹ to X⁵ are independently selected from CCN or N, and at least two of R⁶ to R¹⁰ are F.

According to an embodiment, wherein at least one X¹ to X⁵ are independently selected from CH or CD, and at least one of X¹ to X⁵ are independently selected from CCN or N, and at least one R⁶ to R¹⁰ is CN or F.

According to an embodiment, wherein at least one X¹ to X⁵ are independently selected from CH or CD, and at least one of X¹ to X⁵ are independently selected from CCN or N, and at least two R⁶ to R¹⁰ is CN or F.

According to an embodiment, wherein at least one X¹ to X⁵ are independently selected from CH or CD, and at least one of X¹ to X⁵ are independently selected from CCN or N, and at least one of R⁶ to R¹⁰ is F.

According to an embodiment, wherein at least one X¹ to X⁵ are independently selected from CH or CD, and at least one of X¹ to X⁵ are independently selected from CCN or N, and at least two of R⁶ to R¹⁰ is F.

According to an embodiment, wherein at least one to at most three X¹ to X⁵ are independently selected from CH or CD, and at least one of X¹ to X⁵ are independently selected from CCN or N, and at least one R⁶ to R¹⁰ is CN or F.

According to an embodiment, wherein at least one to at most three X¹ to X⁵ are independently selected from CH or CD, and at least one of X¹ to X⁵ are independently selected from CCN or N, and at least two R⁶ to R¹⁰ is CN or F.

According to an embodiment, wherein at least one to at most three X¹ to X⁵ are independently selected from CH or CD, and at least one of X¹ to X⁵ are independently selected from CCN or N, and at least one of R⁶ to R¹⁰ is F.

According to an embodiment, wherein at least one to at most three X¹ to X⁵ are independently selected from CH or CD, and at least one of X¹ to X⁵ are independently selected from CCN or N, and at least two of R⁶ to R¹⁰ is F.

According to an embodiment, wherein at least one to at most two X¹ to X⁵ are independently selected from CH or CD, and at least one of X¹ to X⁵ are independently selected from CCN or N, and at least one R⁶ to R¹⁰ is CN or F.

According to an embodiment, wherein at least one to at most two X¹ to X⁵ are independently selected from CH or CD, and at least one of X¹ to X⁵ are independently selected from CCN or N, and at least two R⁶ to R¹⁰ is CN or F.

According to an embodiment, wherein at least one to at most two X¹ to X⁵ are independently selected from CH or CD, and at least one of X¹ to X⁵ are independently selected from CCN or N, and at least one of R⁶ to R¹⁰ is F.

According to an embodiment, wherein at least one to at most two X¹ to X⁵ are independently selected from CH or CD, and at least one of X¹ to X⁵ are independently selected from CCN or N, and at least two of R⁶ to R¹⁰ is F.

According to an embodiment, wherein at least one X¹ to X⁵ are independently selected from CH or CD, and at least one of X¹ to X⁵ are independently selected from CCN or N, and at least one R⁶ to R¹⁰are independently selected from CN or F; preferably wherein at least one X¹ to X⁵ are independently selected from CH or CD, and at least one of X¹ to X⁵ are independently selected from CCN or N, and at least two R⁶ to R¹⁰ are independently selected from CN or F; further preferred wherein at least one to at most three X¹ to X⁵ are independently selected from CH or CD, and at least one of X¹ to X⁵ are independently selected from CCN or N, and at least one R⁶ to R¹⁰ are independently selected from CN or F; also preferred wherein at least one to at most three X¹ to X⁵ are independently selected from CH or CD, and at least one of X¹ to X⁵ are independently selected from CCN or N, and at least two R⁶ to R¹⁰ are independently selected from CN or F; furthermore preferred wherein at least one to at most two X¹ to X⁵ are independently selected from CH or CD, and at least one of X¹ to X⁵ are independently selected from CCN or N, and at least one R⁶ to R¹⁰ are independently selected from CN or F; and still preferred wherein at least one to at most two X¹ to X⁵ are independently selected from CH or CD, and at least one of X¹ to X⁵ are independently selected from CCN or N, and at least two R⁶ to R¹⁰ are independently selected from CN or F.

### R⁵

According to an embodiment, wherein R⁵ is selected from substituted or unsubstituted C₆ to C₁₉ aryl, substituted or unsubstituted C₆ to C₁₉ aryl with one, two or three aromatic six-member rings, substituted or unsubstituted C₉ to C₁₉ aryl with two or three fused aromatic six-member rings, substituted or unsubstituted C₆ to C₁₂ aryl with one or two aromatic six-member rings, substituted or unsubstituted phenyl, substituted or unsubstituted biphenyl, substituted or unsubstituted C₃ to C₂₀ heteroaryl, substituted or unsubstituted C₃ to C₂₀ heteroaryl with one, two or three aromatic six-member rings, substituted or unsubstituted C₇ to C₂₀ heteroaryl with two or three fused aromatic six-member rings or CN,
wherein the one or more substituents are selected from electron-withdrawing group, CN, F, SF₅, unsubstituted C₁ to C₈ alkyl or D.

According to an embodiment, wherein R⁵ is selected from substituted or unsubstituted C₆ to C₁₉ aryl, substituted or unsubstituted C₆ to C₁₉ aryl with one, two or three aromatic six-member rings, substituted or unsubstituted C₉ to C₁₉ aryl with two or three fused aromatic six-member rings, substituted or unsubstituted C₆ to C₁₂ aryl with one or two aromatic six-member rings, substituted or unsubstituted phenyl, substituted or unsubstituted biphenyl, substituted or unsubstituted C₃ to C₂₀ heteroaryl, substituted or unsubstituted C₃ to C₂₀ heteroaryl with one, two or three aromatic six-member rings, or substituted or unsubstituted C₇ to C₂₀ heteroaryl with two or three fused aromatic six-member rings,
wherein the one or more substituents are selected from electron-withdrawing group, CN, F, SF₅, unsubstituted C₁ to C₈ alkyl or D.

According to an embodiment, wherein R⁵ is selected from substituted or unsubstituted C₆ to C₁₉ aryl, substituted or unsubstituted C₆ to C₁₉ aryl with one, two or three aromatic six-member rings, substituted or unsubstituted C₉ to C₁₉ aryl with two or three fused aromatic six-member rings, substituted or unsubstituted C₆ to C₁₂ aryl with one or two aromatic six-member rings, substituted or unsubstituted phenyl, substituted or unsubstituted biphenyl, substituted or unsubstituted C₃ to C₂₀ heteroaryl, substituted or unsubstituted C₃ to C₂₀ heteroaryl with one, two or three aromatic six-member rings, substituted or unsubstituted C₇ to C₂₀ heteroaryl with two or three fused aromatic six-member rings or CN,
wherein the one or more substituents are selected from CN, F, SF₅, unsubstituted C₁ to C₈ alkyl or D.

According to an embodiment, wherein R⁵ is selected from substituted or unsubstituted C₆ to C₁₉ aryl, substituted or unsubstituted C₆ to C₁₉ aryl with one, two or three aromatic six-member rings, substituted or unsubstituted C₉ to C₁₉ aryl with two or three fused aromatic six-member rings, substituted or unsubstituted C₆ to C₁₂ aryl with one or two aromatic six-member rings, substituted or unsubstituted phenyl, substituted or unsubstituted biphenyl, substituted or unsubstituted C₃ to C₂₀ heteroaryl, substituted or unsubstituted C₃ to C₂₀ heteroaryl with one, two or three aromatic six-member rings, or substituted or unsubstituted C₇ to C₂₀ heteroaryl with two or three fused aromatic six-member rings,
wherein the one or more substituents are selected from CN, F, SF₅, unsubstituted C₁ to C₈ alkyl or D.

According to an embodiment, wherein R⁵ has the formulae (IVa) or (IVb);
wherein formula (IVa) is represented by: 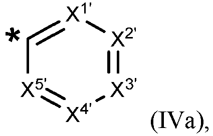
wherein
X^{1'} is selected from CR^{6'} or N;
X^{2'} is selected from CR^{7'} or N;
X^{3'} is selected from CR^{8'} or N;
X^{4'} is selected from CR^{9'} or N;
X^{5'} is selected from CR¹⁰ or N;
wherein
R^{6'}, R^{7'}, R^{8'}, R^{9'} and R^{10'} are independently selected from CN, F, SF₅, D, H, substituted or unsubstituted C₆ to C₁₈ aryl or substituted or unsubstituted C₃ to C₁₈ heteroaryl, preferably H, D, CN, F, substituted or unsubstituted C₆ aryl or substituted or unsubstituted C₃ to C₅ heteroaryl;
   wherein the one or more substituents on substituted C₆ to C₁₃ aryl, substituted C₃ to C₁₄ heteroaryl, substituted C₆ aryl, substituted C₃ to C₅ heteroaryl is selected from CN, F, unsubstituted C₁ to C₈ alkyl or D, preferably CN, F;
   or
formula (IVb) is represented by: 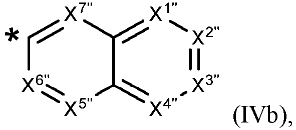
wherein
X^{1"} is selected from CR^{6"} or N;
X^{2"} is selected from CR^{7"} or N;
X^{3"} is selected from CR^{8"} or N;
X^{4"} is selected from CR^{9"} or N;
X^{5"} is selected from CR¹⁰ or N;
X^{6"} is selected from CR^{11"} or N;
X^{7"} is selected from CR^{12"} or N;
wherein R^{6"} , R^{7"}, R^{8"}, R^{9"}, R^{10"}, R^{11"} and R^{12"} are independently selected from CN, F, SF₅, D or H, preferably H, D, CN, F; and wherein the "*" denotes the binding position.

### R^{a} and/or R⁵

According to an embodiment, wherein R^{a} and R⁵ are selected the same.

According to an embodiment, wherein R^{a} and/or R⁵ is independently selected from the group of F1 to F5, G1 to G8, H1 to H9, and I1 to I46: 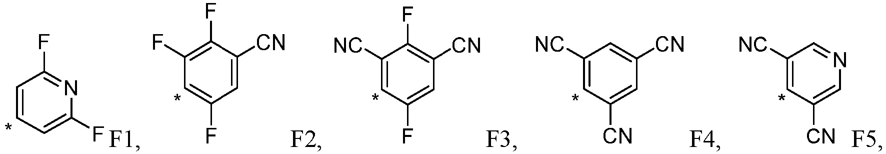 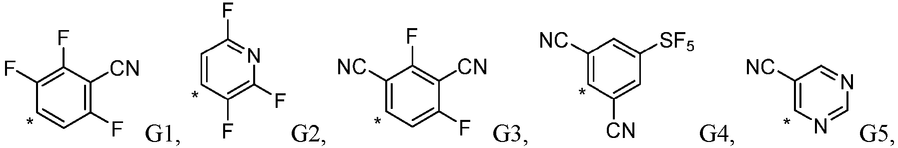 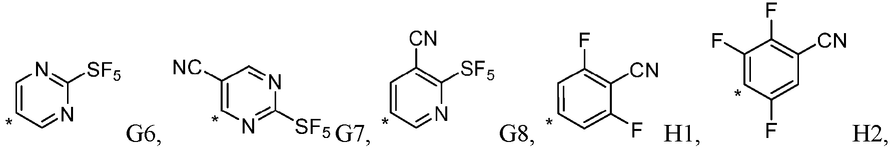 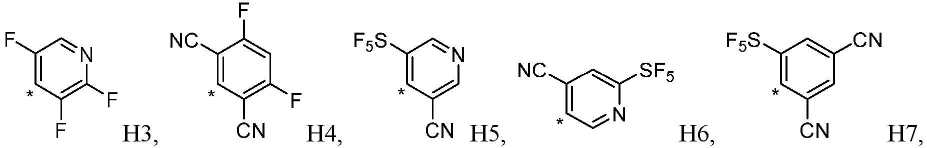 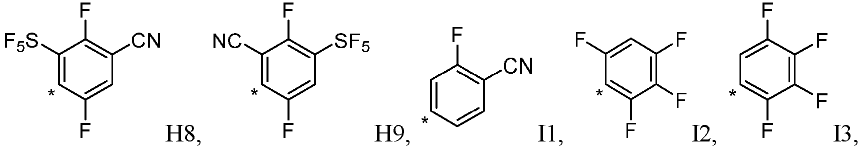 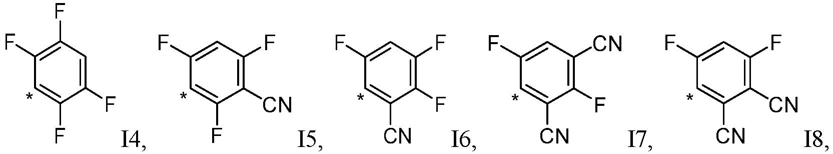 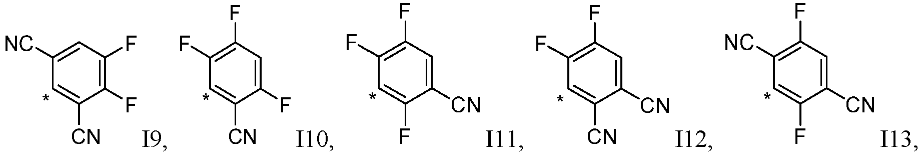 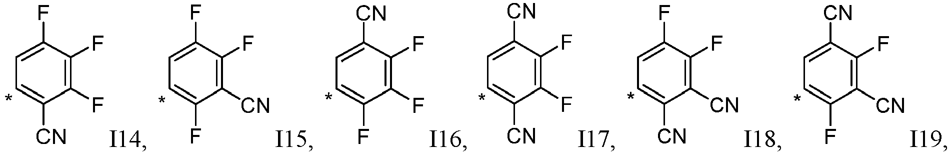 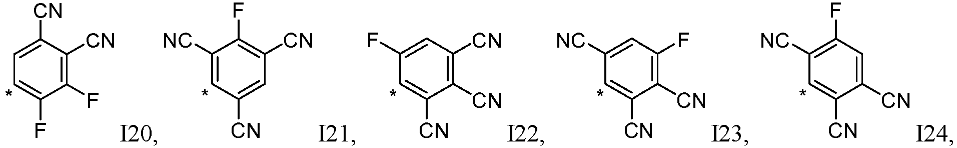 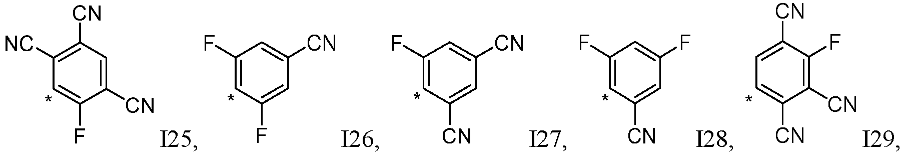 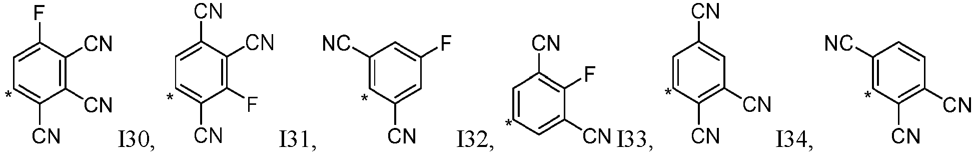 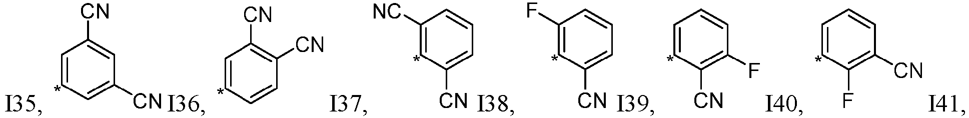 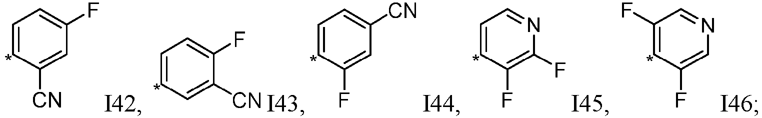 preferably R^{a} and/or R⁵ is independently selected from F1 to F5, G1 to G8, and H1 to H9; further preferred wherein R^{a} and/or R⁵ is independently selected from F1 to F5, and G1 to G8; and additionally preferred wherein R^{a} and/or R⁵ is independently selected from F1 to F5.

According to an embodiment R^{a} is selected from the group of F1 to F5, G1 to G8, H1 to H9, and I1 to I46. According to an embodiment R⁵ is selected from the group of F1 to F5, G1 to G8, H1 to H9, and I1 to I46.

According to an embodiment, R^{a} is preferably selected from F1 to F5, G1 to G8, and H1 to H9; further preferred wherein R^{a} is selected from F1 to F5, and G1 to G8; and additionally preferred wherein R^{a} is selected from F1 to F5.

According to an embodiment, R⁵ is preferably selected from F1 to F5, G1 to G8, and H1 to H9; further preferred wherein R⁵ is selected from F1 to F5, and G1 to G8; and additionally preferred wherein R⁵ is selected from F1 to F5.

According to an embodiment, wherein R^{a} and R⁵ are independently selected from formula (III). According to an embodiment, wherein R^{a} and R⁵ are selected from formula (III) and R^{a} and R⁵ are selected the same.

### R' and/or R"

According to an embodiment, wherein R' and/or R" is independently selected from the group comprising F-CN1 to F-CN5, G-CN1 to G-CN8, H-CN1 to H-CN9, and I-CN1 to I-CN46: 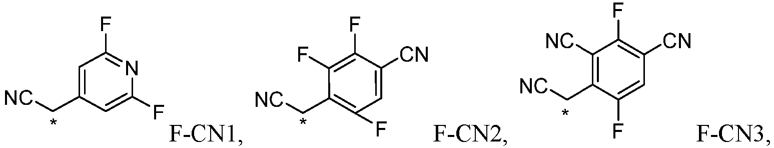 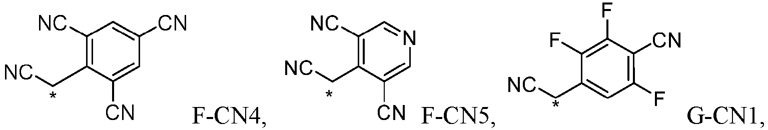 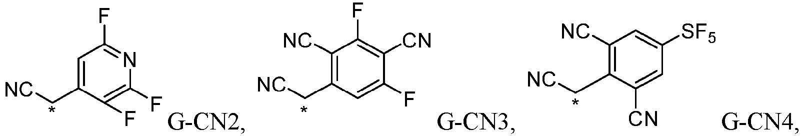 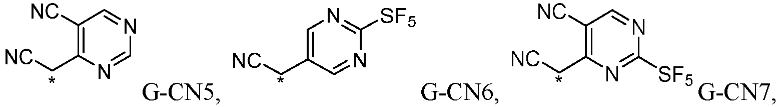 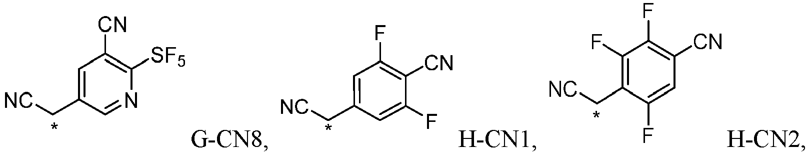 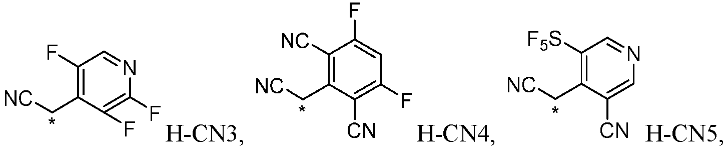 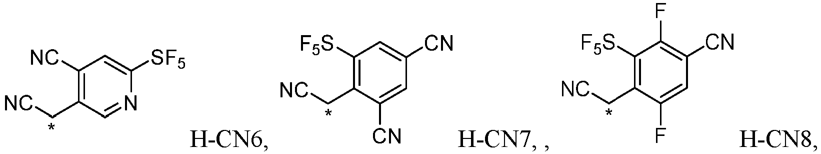 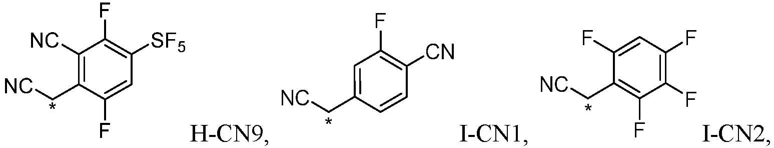 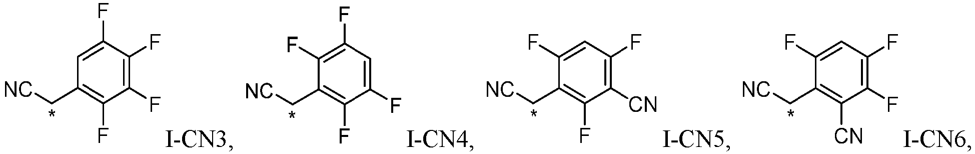 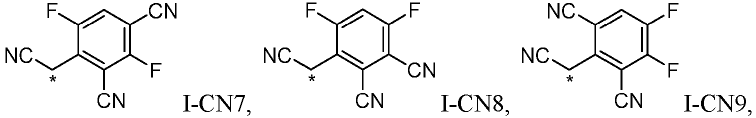 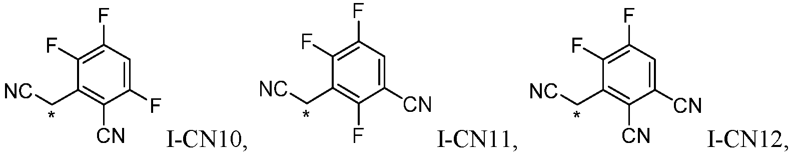 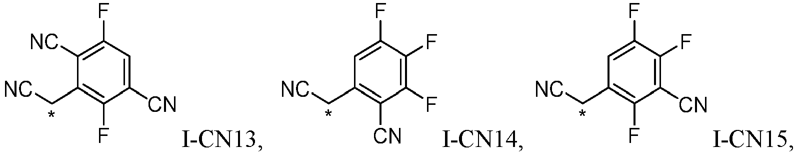 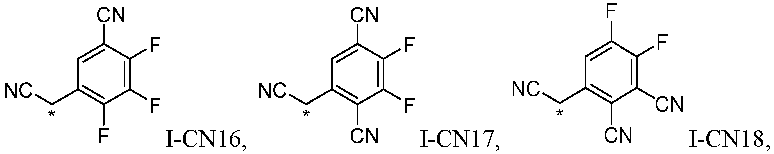 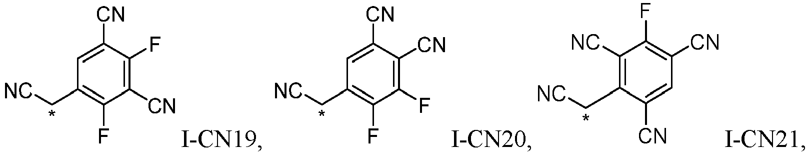 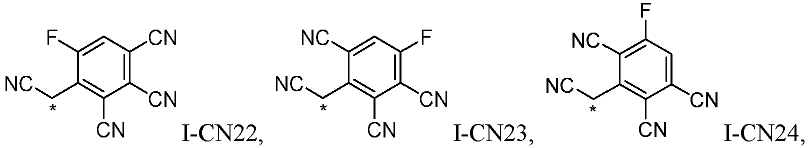 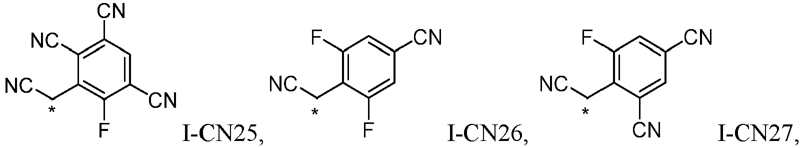 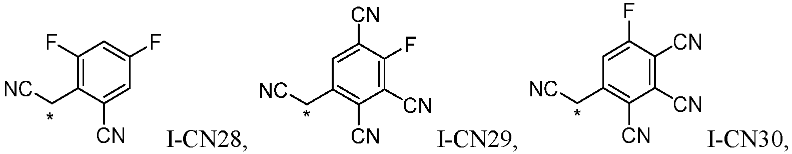 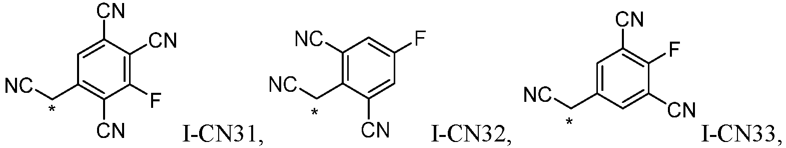 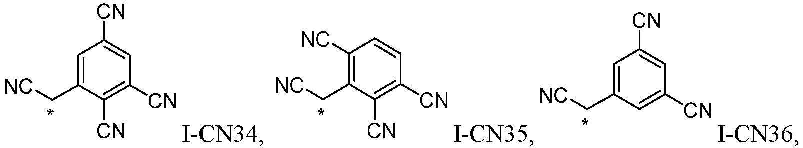 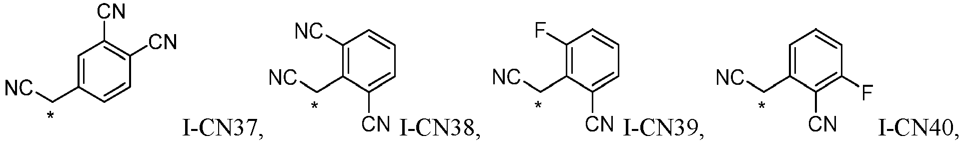 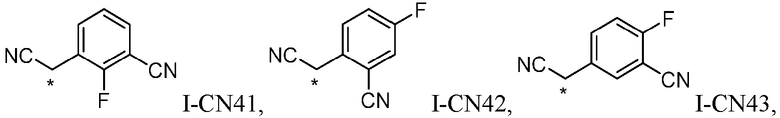 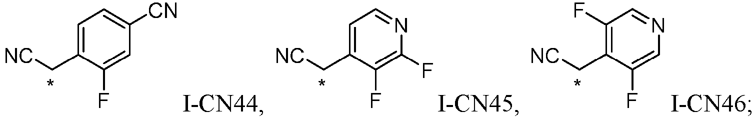 preferably wherein R' and/or R" is independently selected from F-CN1 to F-CN5, G-CN1 to G-CN8, and H-CN1 to H-CN9; further preferred wherein R' and/or R" is independently selected from F-CN1 to F-CN5, and G-CN1 to G-CN8; and additionally preferred wherein R' and/or R" is independently selected from F-CN1 to F-CN5.

According to an embodiment, wherein R' is preferably independently selected from F-CN1 to F-CN5, G-CN1 to G-CN8, and H-CN1 to H-CN9. According to an embodiment, wherein R' is more preferably independently selected from F-CN1 to F-CN5, and G-CN1 to G-CN8. According to an embodiment, wherein R' is most preferably independently selected from F-CN1 to F-CN5.

According to an embodiment, wherein R" is preferably independently selected from F-CN1 to F-CN5, G-CN1 to G-CN8, and H-CN1 to H-CN9. According to an embodiment, wherein R" is more preferably independently selected from F-CN1 to F-CN5, and G-CN1 to G-CN8. According to an embodiment, wherein R" is most preferably independently selected from F-CN1 to F-CN5.

According to an embodiment, wherein R' is selected from formula (II). According to an embodiment, wherein R" is selected from formula (II).

According to an embodiment, wherein R' and R" are independently selected from formula (II); and preferably wherein R' and R" are selected from formula (II) and R' and R" are selected the same.

### Compound of formula (I)

According to an embodiment, wherein the compound of formula (I) comprises 4 to 9 CN groups; preferably 4 to 8 groups. The volatility of the compound of formula (I) may be further adjusted in particular the volatility may be reduced.

According to an embodiment, wherein compound of formula (I) is free of a five member ring.

According to an embodiment, wherein the compound of formula (I) comprises 1 to 6, or 1 to 5, or 1 to 4, or 1 to 3, or 1 to 2 aromatic six member rings.

According to an embodiment, wherein the ratio between F and CN is in the range from ≥ 0.1 to ≤ 1.5, preferably ≥ 0.2 to ≤ 1.5; and more preferably ≥ 0.25 to ≤ 1.5. The volatility of the compound of formula (I) may be further adjusted in particular the volatility may be reduced.

According to an embodiment, wherein R¹, R², R³, R⁴, R' and R" of the compound of formula (I) are selected from the groups J1 to J26 of Table 4 as follows:

**Table 4**

| Group | R¹ | R² | R³ | R⁴ | R' | R" |
|---|---|---|---|---|---|---|
| J1 | F | CN | CN | F | 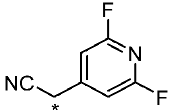 | 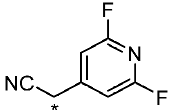 |
| J2 | F | CN | CN | F | 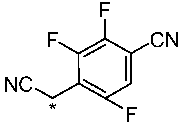 | 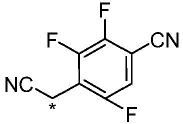 |
| J3 | F | CN | CN | F | 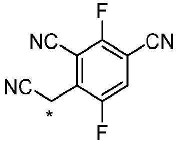 | 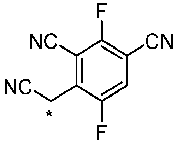 |
| J4 | F | CN | CN | F | 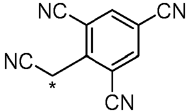 | 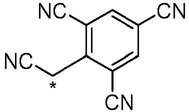 |
| J5 | F | CN | CN | F | 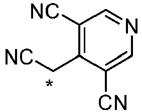 | 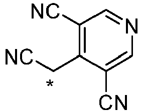 |
| J6 | F | CN | CN | F | 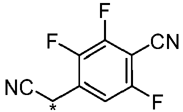 | 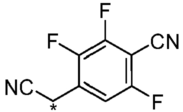 |
| J7 | F | CN | CN | F | 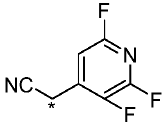 | 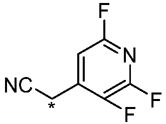 |
| J8 | F | CN | CN | F | 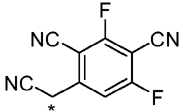 | 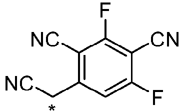 |
| J9 | F | CN | CN | F | 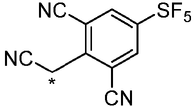 | 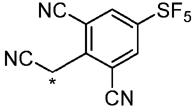 |
| J10 | F | CN | CN | F | 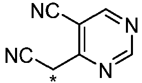 | 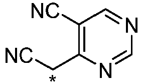 |
| J11 | F | CN | CN | F | 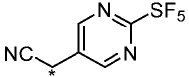 | 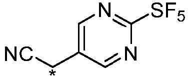 |
| J12 | F | CN | CN | F | 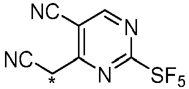 | 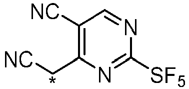 |
| J13 | F | CN | CN | F | 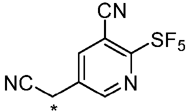 | 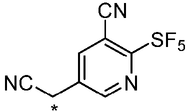 |
| J14 | F | CN | CN | F | 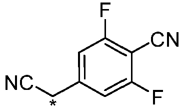 | 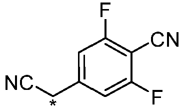 |
| J15 | CN | H | H | CN | 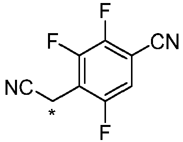 | 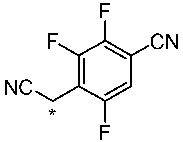 |
| J16 | CN | H | H | CN | 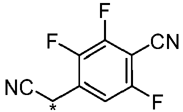 | 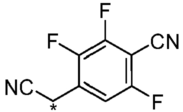 |
| J17 | F | CN | CN | F | 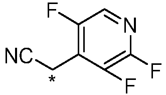 | 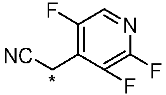 |
| J18 | F | CN | CN | F | 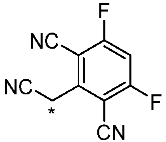 | 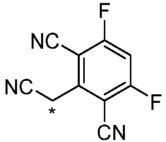 |
| J19 | F | CN | CN | F | 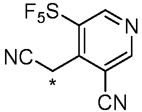 | 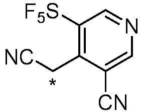 |
| J20 | F | CN | CN | F | 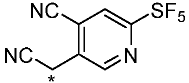 | 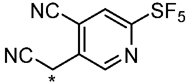 |
| J21 | F | CN | CN | F | 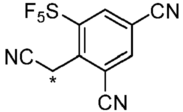 | 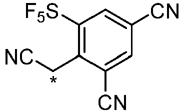 |
| J22 | H | CN | CN | H | 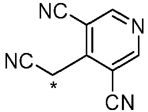 | 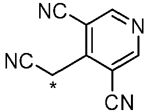 |
| J23 | F | CN | CN | F | 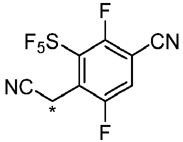 | 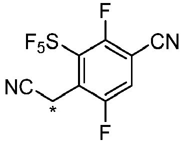 |
| J24 | F | CN | CN | F | 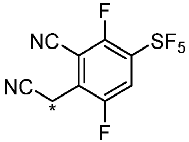 | 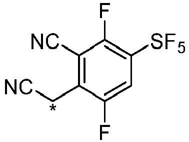 |
| J25 | F | CN | CN | F | 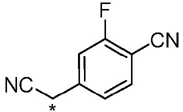 | 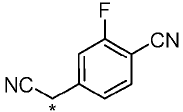 |
| J26 | H | CN | CN | H | 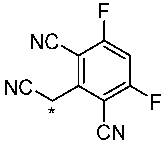 | 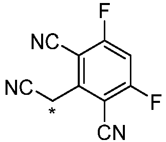 |

According to an embodiment, wherein the compound of formula (I) is preferably selected from J1 to J24. According to an embodiment, wherein the compound of formula (I) is more preferably selected from J1 to J13. According to an embodiment, wherein the compound of formula (I) is most preferably selected from J1 to J5.

According to an embodiment, wherein the compound of formula (I) is selected from the compounds A1 to A15, B1 to B22, C1 to C28, and D1 to D4: 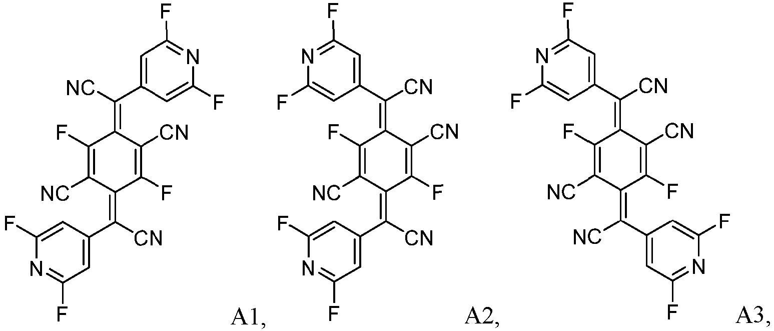 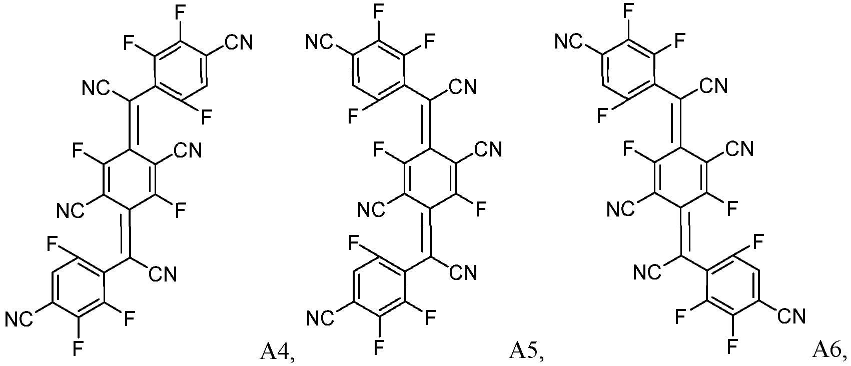 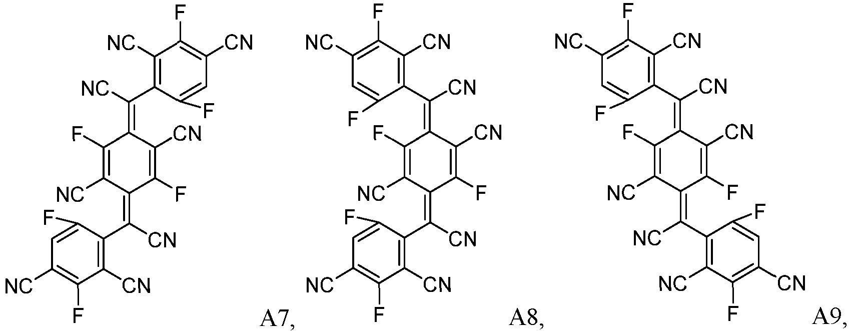 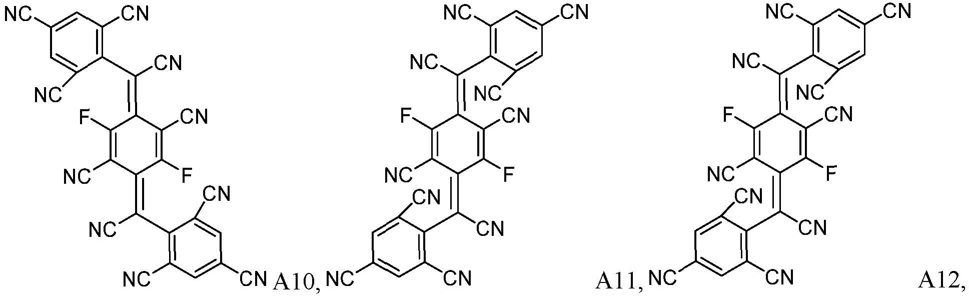 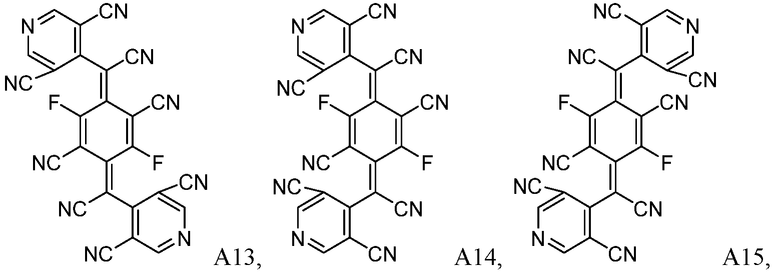 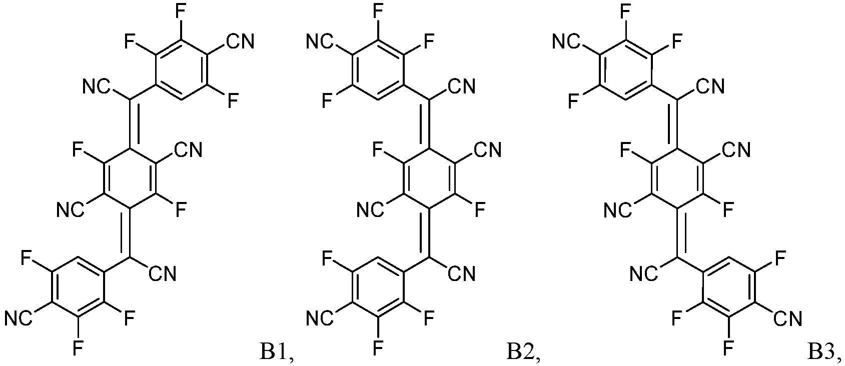 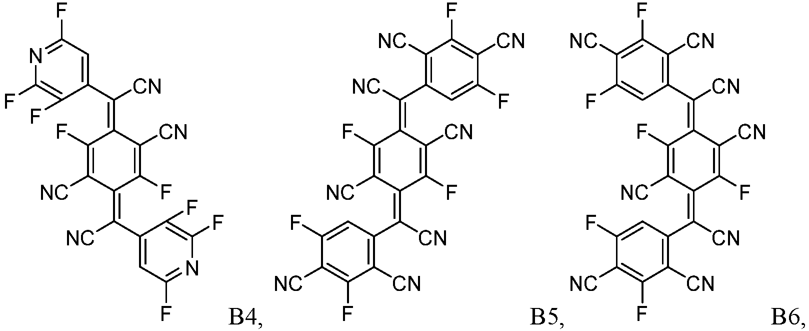 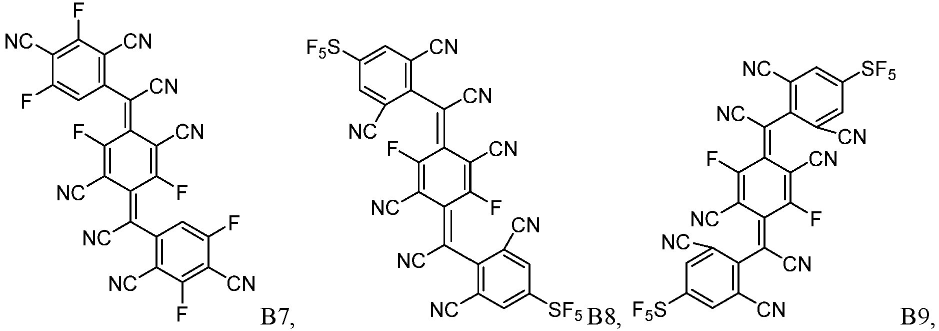 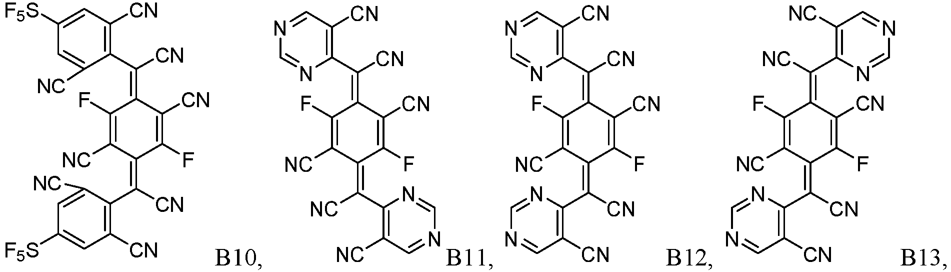 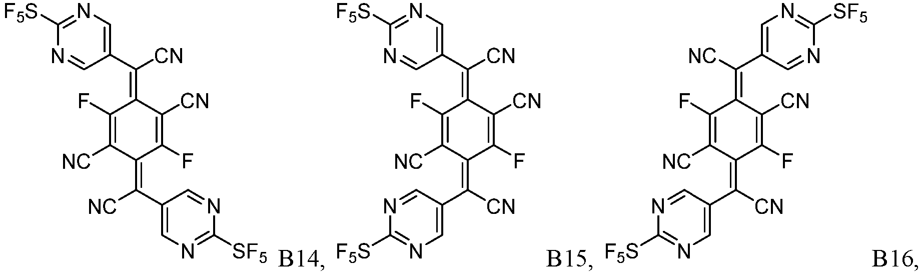 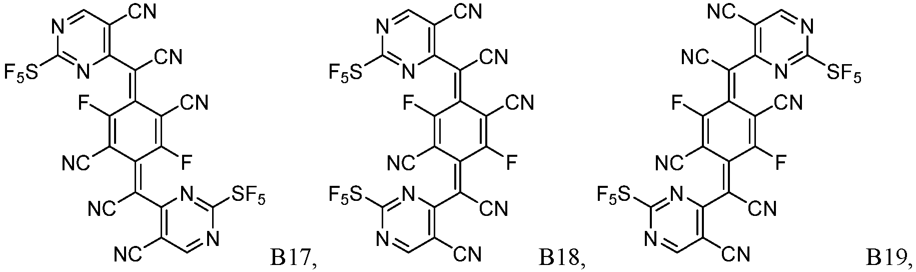 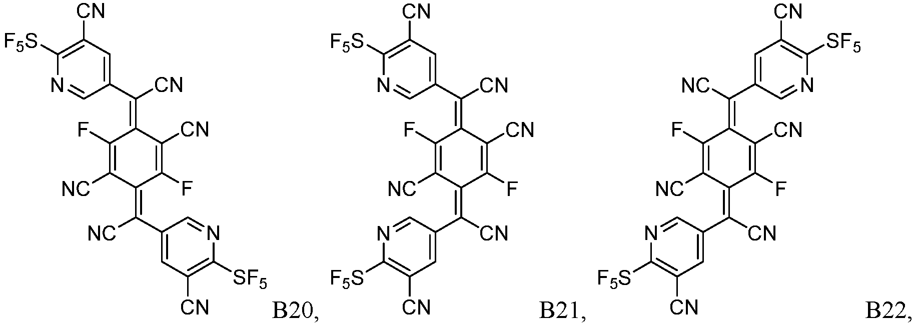 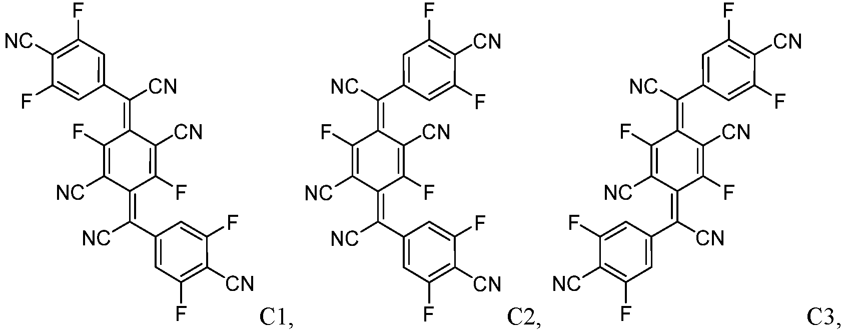 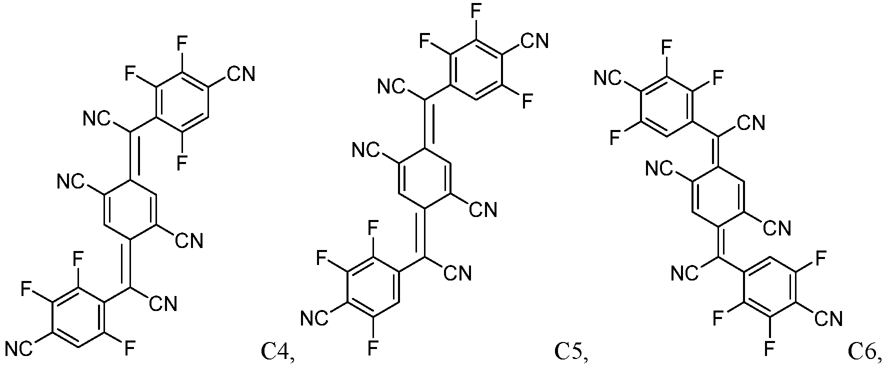 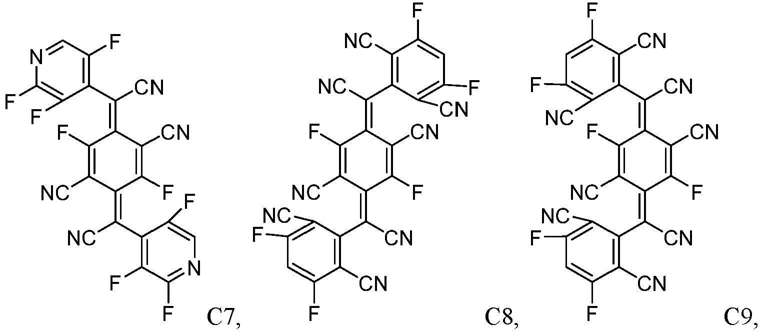 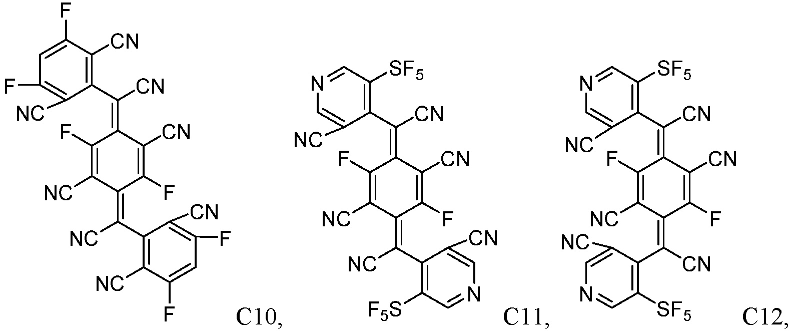 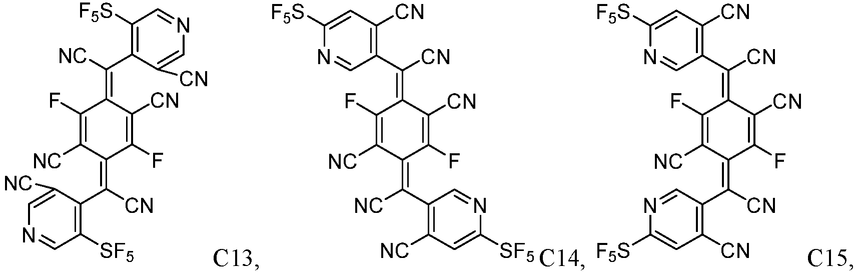 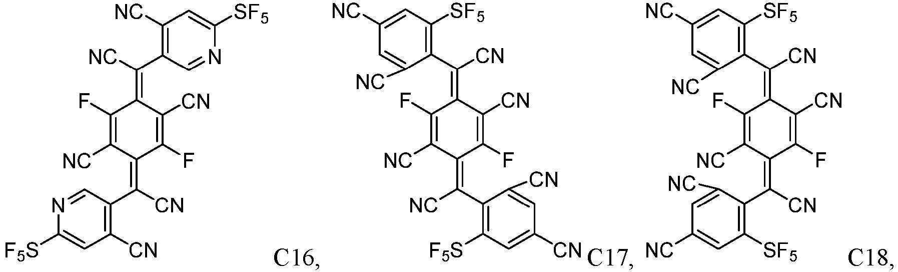 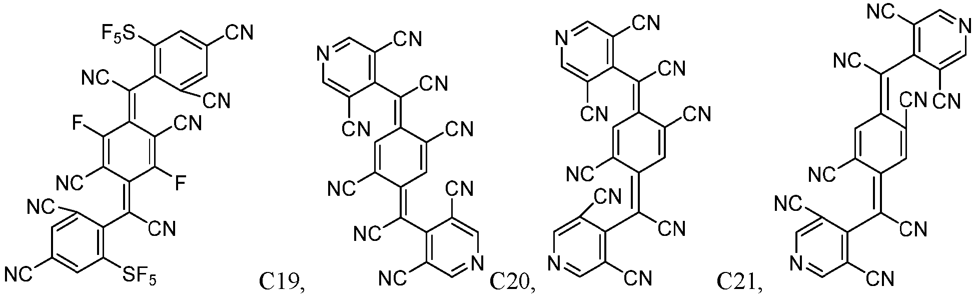 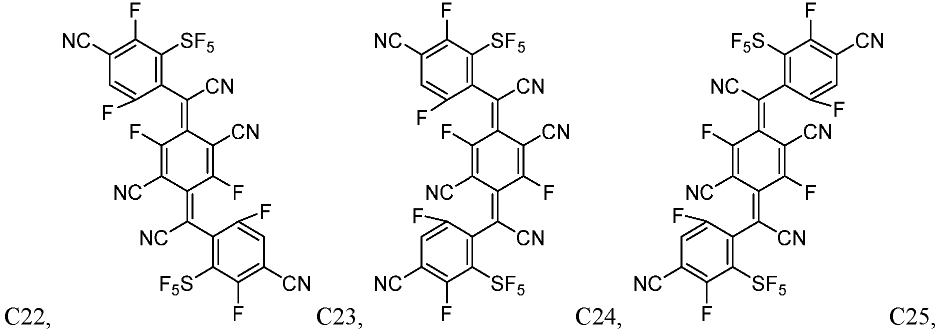 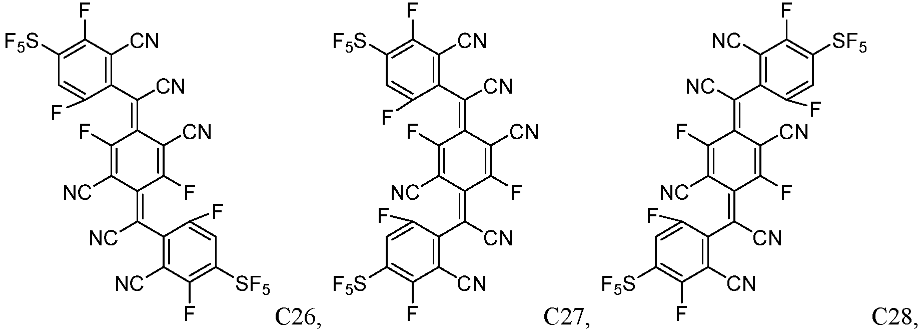 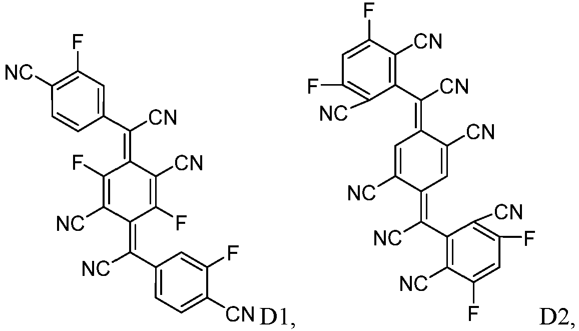 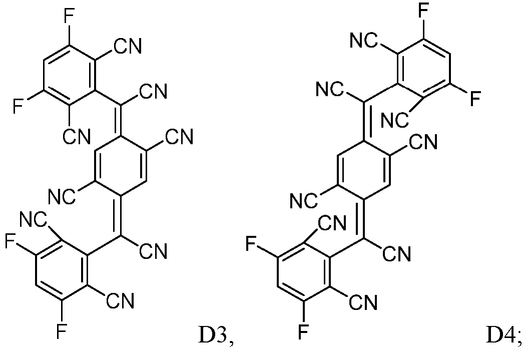 wherein the compound of formula (I) is preferably selected from the compounds A1 to A15, B1 to B22, and C1 to C28; further preferred wherein the compound of formula (I) is more preferably selected from the compounds A1 to A15, and B1 to B22; and additionally preferred wherein the compound of formula (I) is most preferably selected from the compounds A1 to A15.

According to an embodiment, wherein the compound of formula (I) is preferably selected from the compounds A1 to A15, B1 to B22, and C1 to C28. According to an embodiment, wherein the compound of formula (I) is more preferably selected from the compounds A1 to A15, and B1 to B22. According to an embodiment, wherein the compound of formula (I) is most preferably selected from the compounds A1 to A15.

### Organic semiconductor layer

The present invention furthermore relates to an organic semiconductor layer, whereby the organic semiconductor layer comprises a compound according to the present invention or a composition according to the present invention.

The present invention furthermore relates to an organic semiconductor layer, whereby the organic semiconductor layer comprises a compound according to formula (I).

In case the organic semiconductor layer comprises a compound according to the invention, throughout this application text the term "compound of formula (I)" shall also intend to include a composition, wherein the composition comprises at least one compound according to the invention as described above.

According to one embodiment of the present invention the organic semiconductor layer and/or the compound of formula (I) are non-emissive.

In the context of the present specification the term "essentially non-emissive" or "non-emissive" means that the contribution of the compound or layer to the visible emission spectrum from the device is less than 10 %, preferably less than 5 % relative to the visible emission spectrum. The visible emission spectrum is an emission spectrum with a wavelength of about ≥ 380 nm to about ≤ 780 nm.

According to one embodiment of the invention, the at least one organic semiconductor layer may further comprise at least one matrix compound, also named covalent matrix compound, or substantially covalent matrix compound.

### Substantially covalent matrix compound

The organic semiconductor layer may further comprise a covalent matrix compound also named substantially covalent matrix compound. According to one embodiment the substantially covalent matrix compound may be selected from at least one organic compound. The substantially covalent matrix may consist substantially of covalently bound C, H, O, N, S, which optionally comprise in addition covalently bound B, P, As and/or Se.

According to one embodiment of the organic electronic device, the organic semiconductor layer further comprises a substantially covalent matrix compound, wherein the substantially covalent matrix compound may be selected from organic compounds consisting substantially of covalently bound C, H, O, N, S, which optionally comprise in addition covalently bound B, P, As and/or Se.

Organometallic compounds comprising covalent bonds carbon-metal, metal complexes comprising organic ligands and metal salts of organic acids are further examples of organic compounds that may serve as substantially covalent matrix compounds of the hole injection layer.

In one embodiment, the substantially covalent matrix compound lacks metal atoms and majority of its skeletal atoms may be selected from C, O, S, N. Alternatively, the substantially covalent matrix compound lacks metal atoms and majority of its skeletal atoms may be selected from C and N.

According to one embodiment, the substantially covalent matrix compound may have a molecular weight Mw of ≥ 400 and ≤ 2000 g/mol, preferably a molecular weight Mw of ≥ 450 and ≤ 1500 g/mol, further preferred a molecular weight Mw of ≥ 500 and ≤ 1000 g/mol, in addition preferred a molecular weight Mw of ≥ 550 and ≤ 900 g/mol, also preferred a molecular weight Mw of ≥ 600 and ≤ 800 g/mol.

Preferably, the substantially covalent matrix compound comprises at least one arylamine moiety, alternatively a diarylamine moiety, alternatively a triarylamine moiety.

Preferably, the substantially covalent matrix compound is free of metals and/or ionic bonds.

### Compound of formula (XXXI) or a compound of formula (XXXII)

According to another aspect of the present invention, the at least one matrix compound, also referred to as "substantially covalent matrix compound", may comprises at least one arylamine compound, diarylamine compound, triarylamine compound, a compound of formula (XXXI) or a compound of formula (XXXII) 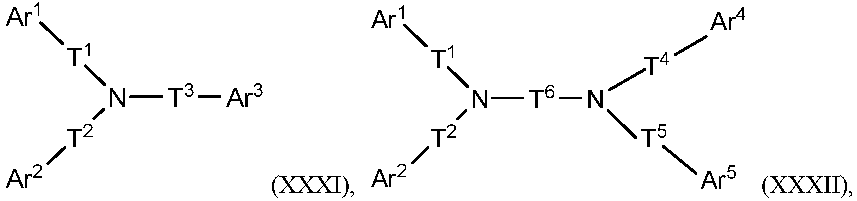 wherein:
T¹, T², T³, T⁴ and T⁵ are independently selected from a single bond, phenylene, biphenylene, terphenylene or naphthenylene, preferably a single bond or phenylene;
T⁶ is phenylene, biphenylene, terphenylene or naphthenylene;
Ar¹, Ar², Ar³, Ar⁴ and Ar⁵ are independently selected from substituted or unsubstituted C₆ to C₂₀ aryl, or substituted or unsubstituted C₃ to C₂₀ heteroarylene, substituted or unsubstituted biphenylene, substituted or unsubstituted fluorene, substituted 9-fluorene, substituted 9,9-fluorene, substituted or unsubstituted naphthalene, substituted or unsubstituted anthracene, substituted or unsubstituted phenanthrene, substituted or unsubstituted pyrene, substituted or unsubstituted perylene, substituted or unsubstituted triphenylene, substituted or unsubstituted tetracene, substituted or unsubstituted tetraphene, substituted or unsubstituted dibenzofurane, substituted or unsubstituted dibenzothiophene, substituted or unsubstituted xanthene, substituted or unsubstituted carbazole, substituted 9-phenylcarbazole, substituted or unsubstituted azepine, substituted or unsubstituted dibenzo[b,f]azepine, substituted or unsubstituted 9,9'-spirobi[fluorene], substituted or unsubstituted spiro[fluorene-9,9'-xanthene], or a substituted or unsubstituted aromatic fused ring system comprising at least three substituted or unsubstituted aromatic rings selected from the group comprising substituted or unsubstituted non-hetero, substituted or unsubstituted hetero 5-member rings, substituted or unsubstituted 6-member rings and/or substituted or unsubstituted 7-member rings, substituted or unsubstituted fluorene, or a fused ring system comprising 2 to 6 substituted or unsubstituted 5- to 7-member rings and the rings are selected from the group comprising (i) unsaturated 5- to 7-member ring of a heterocycle, (ii) 5- to 6-member of an aromatic heterocycle, (iii) unsaturated 5- to 7-member ring of a non-heterocycle, (iv) 6-member ring of an aromatic non-heterocycle;
wherein the substituents of Ar¹, Ar², Ar³, Ar⁴ and Ar⁵ are selected the same or different from the group comprising H, D, F, C(-O)R², CN, Si(R²)₃, P(-O)(R²)₂, OR², S(-O)R², S(-O)₂R², substituted or unsubstituted straight-chain alkyl having 1 to 20 carbon atoms, substituted or unsubstituted branched alkyl having 1 to 20 carbon atoms, substituted or unsubstituted cyclic alkyl having 3 to 20 carbon atoms, substituted or unsubstituted alkenyl or alkynyl groups having 2 to 20 carbon atoms, substituted or unsubstituted alkoxy groups having 1 to 20 carbon atoms, substituted or unsubstituted aromatic ring systems having 6 to 40 aromatic ring atoms, and substituted or unsubstituted heteroaromatic ring systems having 5 to 40 aromatic ring atoms, unsubstituted C₆ to C₁₈ aryl, unsubstituted C₃ to C₁₈ heteroaryl, a fused ring system comprising 2 to 6 unsubstituted 5-to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle, wherein R² may be selected from H, D, straight-chain alkyl having 1 to 6 carbon atoms, branched alkyl having 1 to 6 carbon atoms, cyclic alkyl having 3 to 6 carbon atoms, alkenyl or alkynyl groups having 2 to 6 carbon atoms, C₆ to C₁₈ aryl or C₃ to C₁₈ heteroaryl.

According to an embodiment wherein T¹, T², T³, T⁴ and T⁵ may be independently selected from a single bond, phenylene, biphenylene or terphenylene. According to an embodiment wherein T¹, T², T³, T⁴ and T⁵ may be independently selected from phenylene, biphenylene or terphenylene and one of T¹, T², T³, T⁴ and T⁵ are a single bond. According to an embodiment wherein T¹, T², T³, T⁴ and T⁵ may be independently selected from phenylene or biphenylene and one of T¹, T², T³, T⁴ and T⁵ are a single bond. According to an embodiment wherein T¹, T², T³, T⁴ and T⁵ may be independently selected from phenylene or biphenylene and two of T¹, T², T³, T⁴ and T⁵ are a single bond.

According to an embodiment wherein T¹, T² and T³ may be independently selected from phenylene and one of T¹, T² and T³ are a single bond. According to an embodiment wherein T¹, T² and T³ may be independently selected from phenylene and two of T¹, T² and T³ are a single bond.

According to an embodiment wherein T⁶ may be phenylene, biphenylene, terphenylene. According to an embodiment wherein T⁶ may be phenylene. According to an embodiment wherein T⁶ may be biphenylene. According to an embodiment wherein T⁶ may be terphenylene.

According to an embodiment wherein Ar¹, Ar², Ar³, Ar⁴ and Ar⁵ may be independently selected from K1 to K16: 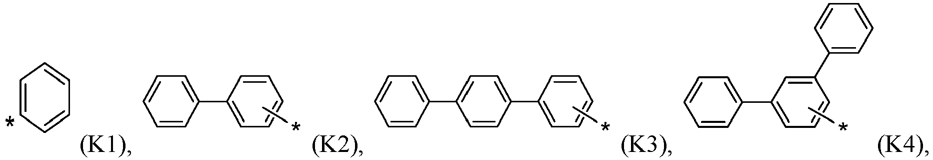 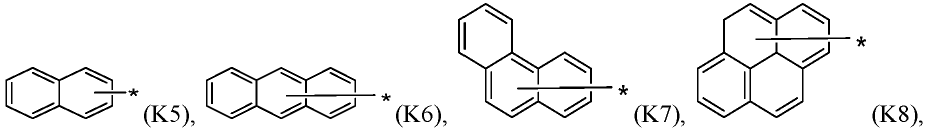 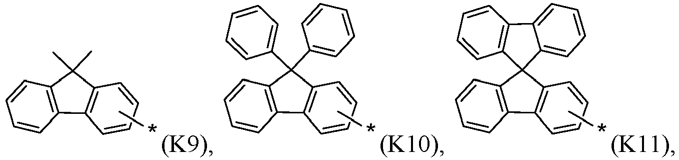 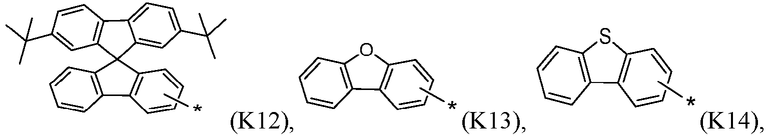 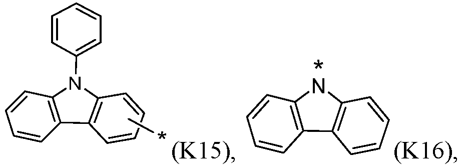 wherein
the asterix "*" denotes the binding position.

According to an embodiment, wherein Ar¹, Ar², Ar³, Ar⁴ and Ar⁵ may be independently selected from K1 to K15; alternatively selected from K1 to K10 and K13 to K15.

According to an embodiment, wherein Ar¹, Ar², Ar³, Ar⁴ and Ar⁵ may be independently selected from the group consisting of K1, K2, K5, K7, K9, K10, K13 to K16.

The rate onset temperature may be in a range particularly suited to mass production, when Ar¹, Ar², Ar³, Ar⁴ and Ar⁵ are selected in this range.

The "matrix compound of formula (XXXI) or formula (XXXII)" may be also referred to as "hole transport compound".

According to one embodiment, the substantially covalent matrix compound comprises at least one naphthyl group, carbazole group, dibenzofuran group, dibenzothiophene group and/or substituted fluorenyl group, wherein the substituents are independently selected from methyl, phenyl or fluorenyl.

According to an embodiment of the electronic device, wherein the matrix compound of formula (XXXI) or formula (XXXII) are selected from L1 to L18:

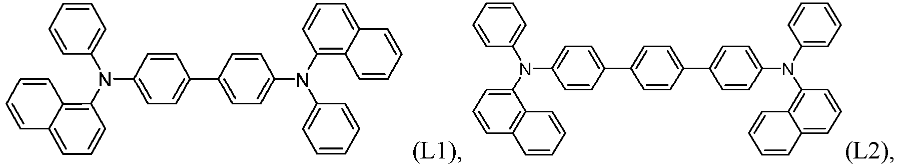

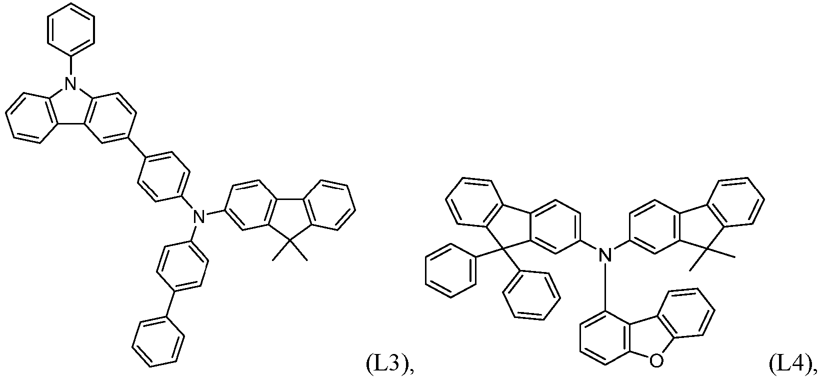

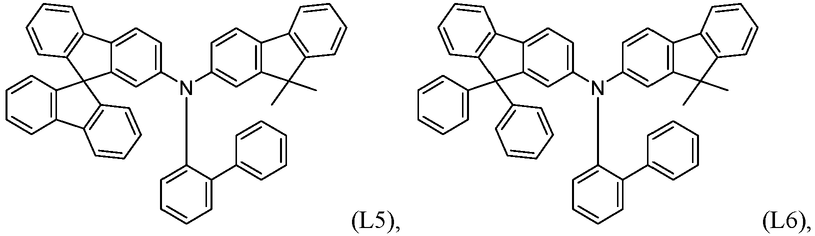

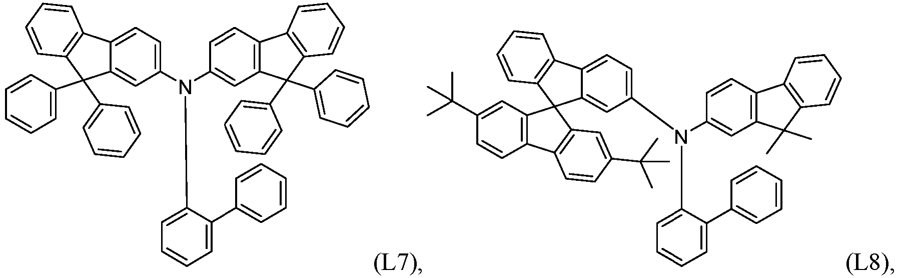

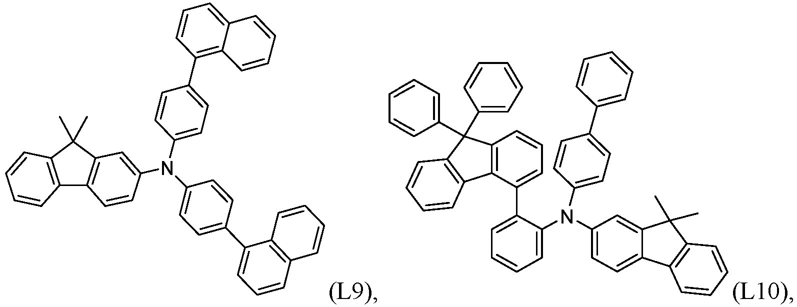

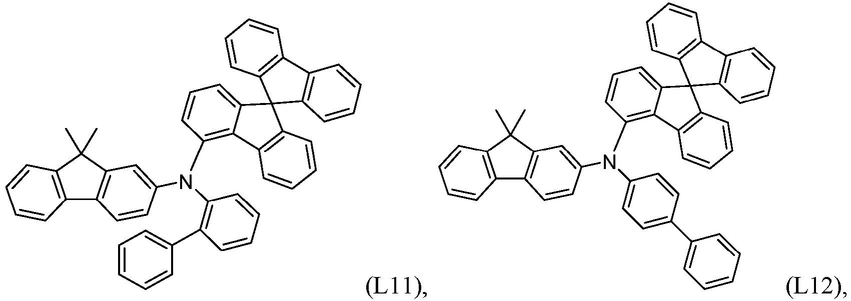

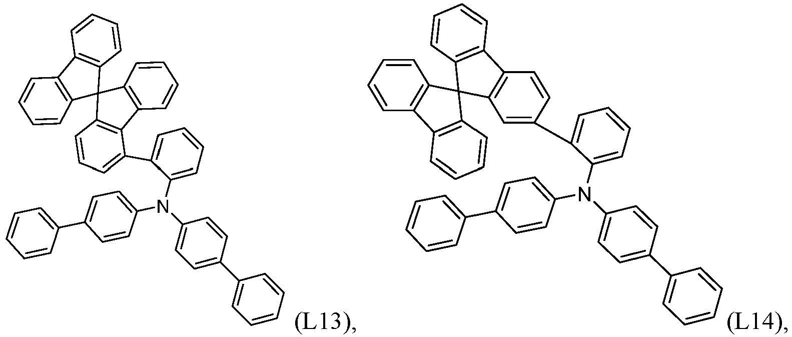

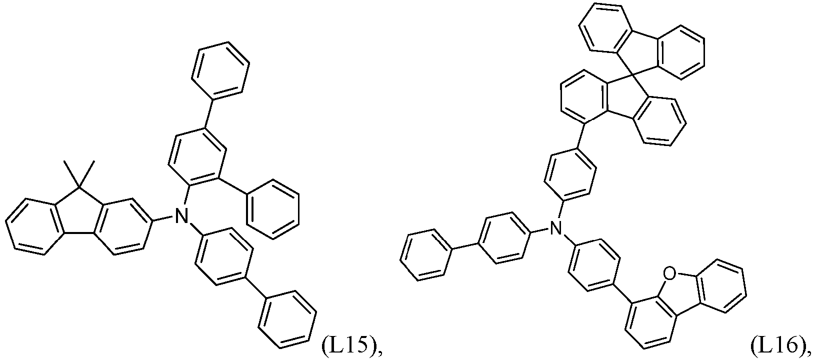

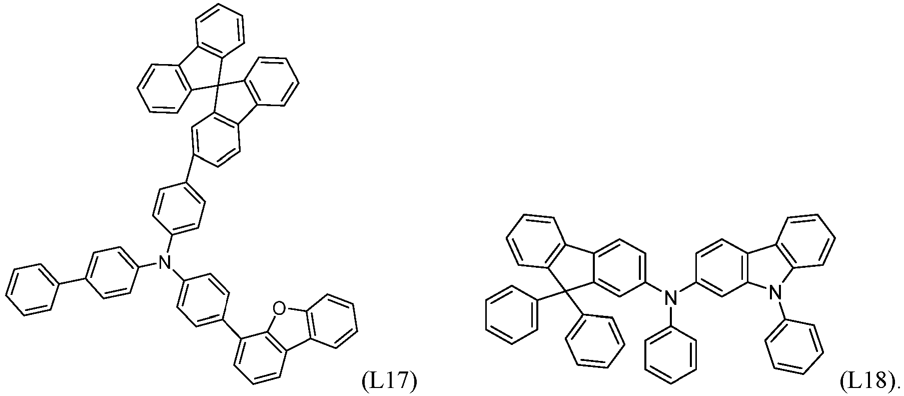

### Organic electronic device

The present invention furthermore relates to an organic electronic device comprising an anode layer, a cathode layer, and at least one organic semiconductor layer, wherein the at least one organic semiconductor layer is arranged between the anode layer and the cathode layer, and wherein the at least one organic semiconductor layer is the organic semiconductor layer according to the present invention.

According to one embodiment of the organic electronic device according to the invention whereby the anode layer comprises at least a first anode sub-layer and a second anode sub-layer.

According to one embodiment of the present invention, wherein the organic electronic device further comprises at least one photoactive layer, wherein the at least one photoactive layer is arranged between the anode layer and the cathode layer and at least one of the at least one organic semiconductor layers is arranged between the anode layer and the at least one photoactive layer.

According to one embodiment of the present invention, wherein the organic semiconductor layer is a hole injection layer.

According to one embodiment of the present invention, the hole injection layer is in direct contact with the anode layer.

According to one embodiment of the present invention, the hole injection layer is in direct contact with the anode layer and the anode layer is in direct contact with the substrate, wherein the substrate is selected from a glass substrate, a plastic substrate, a metal substrate or a backplane.

According to one embodiment of the present invention, wherein the photoactive layer is a light emitting layer.

According to one embodiment of the present invention, wherein the organic electronic device is an electroluminescent device, an organic light emitting diode (OLED), a light emitting device, thin film transistor, a battery, a display device or an organic photovoltaic cell (OPV).

According to one embodiment of the present invention, wherein the organic semiconductor layer is a hole injection layer and/or p-type charge generation layer.

According to one embodiment of the present invention, wherein the organic semiconductor layer is a p-type charge generation layer.

According to one embodiment of the present invention, the organic electronic device comprises at least two emission layers, wherein at least one of the at least one organic semiconductor layers is arranged between the first and the second emission layer.

According to one embodiment of the present invention, the organic electronic device is an electroluminescent device, preferably an organic light emitting diode.

According to one embodiment of the present invention, the organic electronic device further comprises a substrate.

According to one embodiment of the invention, the electronic organic device is an organic light emitting diode comprising a substrate, an anode layer, an organic semiconductor layer comprising a compound of formula (I), an emission layer, an optional electron transport layer, an optional electron injection and a cathode layer. The organic light emitting diode may further comprise a p-type charge generation layer, the p-type charge generation layer is arranged between the anode layer and the cathode layer; and the p-type layer comprises a compound of formula (I).

According to one embodiment of the invention, the electronic organic device is an organic light emitting diode comprising a substrate, an anode layer, a hole injection layer optionally comprising a compound of formula (I), a first emission layer, a charge generation layer comprising a p-type charge generation layer comprising a compound of formula (I), a second emission layer, an optional electron transport layer, an optional electron injection and a cathode layer.

The present invention furthermore relates to a display device comprising an organic electronic device according to the present invention.

### Further layers

In accordance with the invention, the organic electronic device may comprise, besides the layers already mentioned above, further layers. Exemplary embodiments of respective layers are described in the following:

### Substrate

The substrate may be any substrate that is commonly used in manufacturing of, electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate, a silicon substrate or a backplane.

### Anode layer

The anode layer may be formed by depositing or sputtering a material that is used to form the anode layer. The material used to form the anode layer may be a high work-function material, to facilitate hole injection. The anode material may also be selected from a low work function material (i.e. aluminum). The anode electrode may be a transparent or reflective electrode. Transparent conductive oxides, such as indium tin oxide (ITO), indium zinc oxide (IZO), tin-dioxide (SnO2), aluminum zinc oxide (AlZO) and zinc oxide (ZnO), may be used to form the anode electrode. The anode layer may also be formed using metals, typically silver (Ag), gold (Au), or metal alloys.

According to one embodiment of the present invention, the anode layer comprises a first anode sub-layer and a second anode sub-layer, wherein
- the first anode sub-layer comprises a first metal having a work function in the range of ≥ 4 and ≤ 6 eV, and
- the second anode sub-layer comprises a transparent conductive oxide; and
- the second anode sub-layer is arranged closer to the hole injection layer.

According to one embodiment of the present invention, the first metal of the first anode sub-layer may be selected from the group comprising Ag, Mg, Al, Cr, Pt, Au, Pd, Ni, Nd, Ir, preferably Ag, Au or Al, and more preferred Ag.

According to one embodiment of the present invention, the first anode sub-layer has have a thickness in the range of 5 to 200 nm, alternatively 8 to 180 nm, alternatively 8 to 150 nm, alternatively 100 to 150 nm.

According to one embodiment of the present invention, the first anode sub-layer is formed by depositing the first metal via vacuum thermal evaporation.

It is to be understood that the first anode layer is not part of the substrate.

According to one embodiment of the present invention, the transparent conductive oxide of the second anode sub layer is selected from the group selected from the group comprising indium tin oxide or indium zinc oxide, more preferred indium tin oxide.

According to one embodiment of the present invention, the second anode sub-layer may have a thickness in the range of 3 to 200 nm, alternatively 3 to 180 nm, alternatively 3 to 150 nm, alternatively 3 to 20 nm.

According to one embodiment of the present invention, the second anode sub-layer may be formed by sputtering of the transparent conductive oxide.

According to one embodiment of the present invention, anode layer of the organic electronic device comprises in addition a third anode sub-layer comprising a transparent conductive oxide, wherein the third anode sub-layer is arranged between the substrate and the first anode sub-layer.

According to one embodiment of the present invention, the third anode sub-layer comprises a transparent oxide, preferably from the group selected from the group comprising indium tin oxide or indium zinc oxide, more preferred indium tin oxide.

According to one embodiment of the present invention, the third anode sub-layer may has a thickness in the range of 3 to 200 nm, alternatively 3 to 180 nm, alternatively 3 to 150 nm, alternatively 3 to 20 nm.

According to one embodiment of the present invention, the third anode sub-layer may be formed by sputtering of the transparent conductive oxide.

It is to be understood that the third anode layer is not part of the substrate.

According to one embodiment of the present invention, the anode layer comprises a first anode sub-layer comprising of Ag, a second anode sub-layer comprising of transparent conductive oxide, preferably ITO, and a third anode sub-layer comprising of transparent conductive oxide, preferably ITO; wherein the first anode sub-layer is arranged between the second and the third anode sub-layer.

### Hole transport layer

According to one embodiment of the present invention, the organic electronic device comprises a hole transport layer, wherein the hole transport layer is arranged between the hole injection layer and the at least one first emission layer.

The hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the compound that is used to form the HTL.

The HTL may be formed of any compound that is commonly used to form a HTL. Compounds that can be suitably used are disclosed for example in Yasuhiko Shirota and Hiroshi Kageyama, Chem. Rev. 2007, 107, 953-1010 and incorporated by reference. Examples of the compound that may be used to form the HTL are: carbazole derivatives, such as N-phenyl-carbazole or polyvinylcarbazole; benzidine derivatives, such as N,N'-bis(3-methylphenyl)-N,N'-diphenyl-[1,1-biphenyl]-4,4'-diamine (TPD), or N,N'-di(naphthalen-1-yl)-N,N'-diphenyl benzidine (alpha-NPD); and triphenylamine-based compound, such as 4,4',4"-tris(N-carbazolyl)triphenylamine (TCTA). Among these compounds, TCTA can transport holes and inhibit excitons from being diffused into the EML.

According to one embodiment of the present invention, the hole transport layer may comprise a substantially covalent matrix compound as described above.

According to one embodiment of the present invention, the hole transport layer may comprise a compound of formula (XXXI) or (XXXII) as described above.

According to one embodiment of the present invention, the hole injection layer and the hole transport layer comprises the same substantially covalent matrix compound as described above.

According to one embodiment of the present invention, the hole injection layer and the hole transport layer comprises the same compound of formula (XXXI) or (XXXII) as described above.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 110 nm to about 140 nm.

When the thickness of the HTL is within this range, the HTL may have excellent hole transporting characteristics, without a substantial penalty in driving voltage.

### Electron blocking layer

The function of an electron blocking layer (EBL) is to prevent electrons from being transferred from an emission layer to the hole transport layer and thereby confine electrons to the emission layer. Thereby, efficiency, operating voltage and/or lifetime are improved. Typically, the electron blocking layer comprises a triarylamine compound. The triarylamine compound may have a LUMO level closer to vacuum level than the LUMO level of the hole transport layer. The electron blocking layer may have a HOMO level that is further away from vacuum level compared to the HOMO level of the hole transport layer. The thickness of the electron blocking layer may be selected between 2 and 20 nm.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer can be achieved. The triplet control layer is selected from triarylamine compounds with a triplet level above the triplet level of the phosphorescent emitter in the adjacent emission layer. Suitable compounds for the triplet control layer, in particular the triarylamine compounds, are described in EP 2 722 908 A1.

### Photoactive layer (PAL)

The photoactive layer converts an electrical current into photons or photons into an electrical current. The PAL may be formed on the HTL by vacuum deposition, spin coating, slot-die coating, printing, casting, LB deposition, or the like. When the PAL is formed using vacuum deposition or spin coating, the conditions for deposition and coating may be similar to those for the formation of the HIL. However, the conditions for deposition and coating may vary, according to the compound that is used to form the PAL. It may be provided that the photoactive layer does not comprise the a metal complex according to formula (I). The photoactive layer may be a light-emitting layer or a light-absorbing layer.

### Emission layer (EML)

The EML may be formed on the HTL by vacuum deposition, spin coating, slot-die coating, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML.

According to one embodiment of the present invention, the emission layer does not comprise the compound of formula (I).

The emission layer (EML) may be formed of a combination of a host and an emitter dopant. Example of the host are Alq3, 4,4'-N,N'-dicarbazole-biphenyl (CBP), poly(n-vinylcarbazole) (PVK), 9,10-di(naphthalene-2-yl)anthracene (ADN), 4,4',4"-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc (Zn(BTZ)2).

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, Ir(piq)3, and Btp2lr(acac), but are not limited thereto. These compounds are phosphorescent emitters, however, fluorescent red emitter dopants could also be used.

Examples of phosphorescent green emitter dopants are Ir(ppy)3 (ppy = phenylpyridine), Ir(ppy)2(acac), Ir(mpyp)3.

Examples of phosphorescent blue emitter dopants are F2Irpic, (F2ppy)2Ir(tmd) and Ir(dfppz)3 and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.

The amount of the emitter dopant may be in the range from about 0.01 to about 50 parts by weight, based on 100 parts by weight of the host. Alternatively, the emission layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in driving voltage.

### Hole blocking layer (HBL)

A hole blocking layer (HBL) may be formed on the EML, by using vacuum deposition, spin coating, slot-die coating, printing, casting, LB deposition, or the like, in order to prevent the diffusion of holes into the ETL. When the EML comprises a phosphorescent dopant, the HBL may have also a triplet exciton blocking function.

The HBL may also be named auxiliary ETL or a-ETL.

When the HBL is formed using vacuum deposition or spin coating, the conditions for deposition and coating may be similar to those for the formation of the HIL. However, the conditions for deposition and coating may vary, according to the compound that is used to form the HBL. Any compound that is commonly used to form a HBL may be used. Examples of compounds for forming the HBL include oxadiazole derivatives, triazole derivatives, phenanthroline derivatives and azine derivatives, preferably triazine or pyrimidine derivatives.

The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving voltage.

### Electron transport layer (ETL)

The organic electronic device according to the present invention may further comprise an electron transport layer (ETL).

According to another embodiment of the present invention, the electron transport layer may further comprise an azine compound, preferably a triazine compound.

In one embodiment, the electron transport layer may further comprise a dopant selected from an alkali organic complex, preferably LiQ.

The thickness of the ETL may be in the range from about 15 nm to about 50 nm, for example, in the range from about 20 nm to about 40 nm. When the thickness of the EII, is within this range, the ETL may have satisfactory electron-injecting properties, without a substantial penalty in driving voltage.

According to another embodiment of the present invention, the organic electronic device may further comprise a hole blocking layer and an electron transport layer, wherein the hole blocking layer and the electron transport layer comprise an azine compound. Preferably, the azine compound is a triazine compound.

### Electron injection layer (EIL)

An optional EIL, which may facilitates injection of electrons from the cathode, may be formed on the ETL, preferably directly on the electron transport layer. Examples of materials for forming the EII, include lithium 8-hydroxyquinolinolate (LiQ), LiF, NaCl, CsF, Li2O, BaO, Ca, Ba, Yb, Mg which are known in the art. Deposition and coating conditions for forming the EII, are similar to those for formation of the HIL, although the deposition and coating conditions may vary, according to the material that is used to form the EIL.

The thickness of the EII, may be in the range from about 0.1 nm to about 10 nm, for example, in the range from about 0.5 nm to about 9 nm. When the thickness of the EII, is within this range, the EII, may have satisfactory electron-injecting properties, without a substantial penalty in driving voltage.

### Charge generation layer

The organic electronic device according to the present invention may further comprise a charge generation layer, wherein the organic semiconductor layer of the present invention is a p-type charge generation layer (p-CGL), wherein the p-type charge generation layer is arranged closer to the cathode layer.

The charge generation layer may further comprise a n-type charge generation layer (n-CGL), wherein the n-type charge generation layer is arranged between the p-type charge generation layer and the anode layer.

Preferably, the n-type charge generation layer and the p-type charge generation layer are arranged in direct contact.

The thickness of the n-CGL may be in the range from about 0.5 nm to about 15 nm, for example, in the range from about 1 nm to about 10 nm. When the thickness of the n-CGL is within this range, the EII, may have satisfactory electron-injecting properties, without a substantial penalty in driving voltage.

The n-CGL may comprise a metal dopant, wherein the metal dopant is selected from an alkali metal, alkaline earth metal or rare earth metal.

The n-CGL may comprise an azine compound. According to a preferred embodiment, the nCGL may comprise an azine compound and a metal dopant, wherein the metal dopant is selected from an alkali metal, alkaline earth metal or rare earth metal.

### Cathode layer

The cathode layer is formed on the ETL or optional EIL. The cathode layer may be formed of a metal, an alloy, an electrically conductive compound, or a mixture thereof. The cathode electrode may have a low work function. For example, the cathode layer may be formed of lithium (Li), magnesium (Mg), aluminum (Al), aluminum (Al)-lithium (Li), calcium (Ca), barium (Ba), ytterbium (Yb), magnesium (Mg)-indium (In), magnesium (Mg)-silver (Ag), or the like. Alternatively, the cathode electrode may be formed of a transparent conductive oxide, such as ITO or IZO.

The thickness of the cathode layer may be in the range from about 5 nm to about 1000 nm, for example, in the range from about 10 nm to about 100 nm. When the thickness of the cathode layer is in the range from about 5 nm to about 50 nm, the cathode layer may be transparent or semitransparent even if formed from a metal or metal alloy.

It is to be understood that the cathode layer is not part of an electron injection layer or the electron transport layer.

### Organic light-emitting diode (OLED)

The organic electronic device according to the invention may be an organic light-emitting device.

According to one aspect of the present invention, there is provided an organic light-emitting diode (OLED) comprising: a substrate; an anode layer formed on the substrate; a hole injection layer comprising a compound of formula (I), a hole transport layer, an emission layer, an electron transport layer and a cathode layer.

According to another aspect of the present invention, there is provided an OLED comprising: a substrate; an anode layer formed on the substrate; a hole injection layer comprising a compound of formula (I), a hole transport layer, an electron blocking layer, an emission layer, a hole blocking layer, an electron transport layer and a cathode layer.

According to another aspect of the present invention, there is provided an OLED comprising: a substrate; an anode layer formed on the substrate; a hole injection layer comprising a compound of formula (I), a hole transport layer, an electron blocking layer, an emission layer, a hole blocking layer, an electron transport layer, an electron injection layer, and a cathode layer.

According to various embodiments of the present invention, there may be provided OLEDs layers arranged between the above mentioned layers, on the substrate or on the top layer.

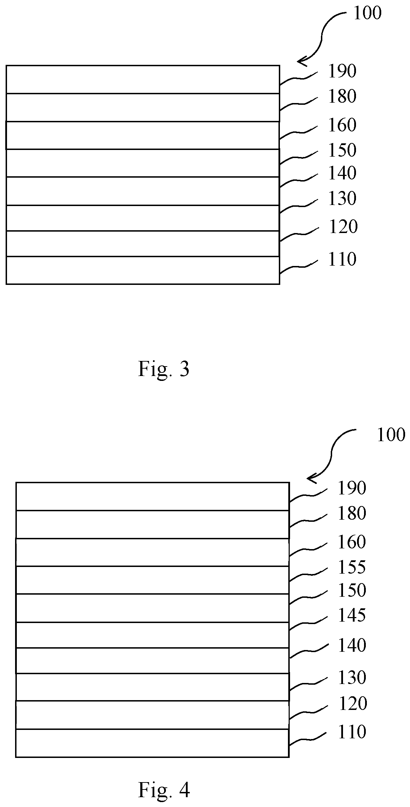

For example, the OLED according to Fig. 3 may be formed by a process, wherein on a substrate (110), an anode (120), a hole injection layer (130) which may comprise compound of formula (I), a hole transport layer (140), an electron blocking layer (145), an emission layer (150), a hole blocking layer (155), an electron transport layer (160), an electron injection layer (180) and the cathode layer (190) are subsequently formed in that order.

According to one aspect, the OLED may comprise a layer structure of a substrate that is adjacent arranged to an anode layer, the anode layer is adjacent arranged to a hole injection layer, the hole injection layer is adjacent arranged to a first hole transport layer, the first hole transport layer is adjacent arranged to a first electron blocking layer, the first electron blocking layer is adjacent arranged to a first emission layer, the first emission layer is adjacent arranged to a first electron transport layer, the first electron transport layer is adjacent arranged to an n-type charge generation layer, the n-type charge generation layer is adjacent arranged to a p-type charge generation layer, the p-type charge generation layer is adjacent arranged to a second hole transport layer, the second hole transport layer is adjacent arranged to a second electron blocking layer, the second electron blocking layer is adjacent arranged to a second emission layer, between the second emission layer and the cathode layer an optional electron transport layer and/or an optional injection layer are arranged.

The organic semiconductor layer according to the invention may be the first hole injection layer and/or the p-type charge generation layer.

### Organic electronic device

The organic electronic device according to the invention may be a light emitting device, or a photovoltaic cell, and preferably a light emitting device.

According to another aspect of the present invention, there is provided a method of manufacturing an organic electronic device, the method using:
- at least one deposition source, preferably two deposition sources and more preferred at least three deposition sources.

The methods for deposition that can be suitable comprise:
- deposition via vacuum thermal evaporation;
- deposition via solution processing, preferably the processing is selected from spin-coating, printing, casting; and/or
- slot-die coating.

According to various embodiments of the present invention, there is provided a method using:
- a first deposition source to release the compound of formula (I) according to the invention, and
- a second deposition source to release the substantially covalent matrix compound;
   the method comprising the steps of forming the hole injection layer; whereby for an organic light-emitting diode (OLED):
- the hole injection layer is formed by releasing the compound of formula (I) according to the invention from the first deposition source and the substantially covalent matrix compound from the second deposition source.

According to various embodiments of the present invention, the method may further include forming on the anode layer, at least one layer selected from the group consisting of forming a hole transport layer or forming a hole blocking layer, and an emission layer between the anode layer and the first electron transport layer.

According to various embodiments of the present invention, the method may further include the steps for forming an organic light-emitting diode (OLED), wherein
- on a substrate an anode layer is formed,
- on the anode layer a hole injection layer comprising a compound of formula (I) is formed,
- on the hole injection layer comprising a compound of formula (I) a hole transport layer is formed,
- on the hole transport layer an emission layer is formed,
- on the emission layer an electron transport layer is formed, optionally a hole blocking layer is formed on the emission layer,
- and finally a cathode layer is formed,
- optional a hole blocking layer is formed in that order between the first anode layer and the emission layer,
- optional an electron injection layer is formed between the electron transport layer and the cathode layer.

According to various embodiments, the OLED may have the following layer structure, wherein the layers having the following order:
anode layer, hole injection layer comprising a compound of formula (I) according to the invention, first hole transport layer, second hole transport layer, emission layer, optional hole blocking layer, electron transport layer, optional electron injection layer, and cathode layer.

According to another aspect of the invention, it is provided an electronic device comprising at least one organic light emitting device according to any embodiment described throughout this application, preferably, the electronic device comprises the organic light emitting diode in one of embodiments described throughout this application. More preferably, the electronic device is a display device.

Hereinafter, the embodiments are illustrated in more detail with reference to examples. However, the present disclosure is not limited to the following examples. Reference will now be made in detail to the exemplary aspects.

### Description of the Drawings

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object of the invention are disclosed in the dependent claims and the following description of the respective figures which in an exemplary fashion show preferred embodiments according to the invention. Any embodiment does not necessarily represent the full scope of the invention, however, and reference is made therefore to the claims and herein for interpreting the scope of the invention. It is to be understood that both the foregoing general description and the following detailed description are exemplary and explanatory only and are intended to provide further explanation of the present invention as claimed.
- FIG. 1: is a schematic sectional view of an organic electronic device, according to an exemplary embodiment of the present invention;
- FIG. 2: is a schematic sectional view of an organic electronic device, according to an exemplary embodiment of the present invention;
- FIG. 3: is a schematic sectional view of an organic electronic device , according to an exemplary embodiment of the present invention;
- FIG. 4: is a schematic sectional view of an OLED, according to an exemplary embodiment of the present invention.
- FIG. 5: is a schematic sectional view of an OLED, according to an exemplary embodiment of the present invention.
- FIG. 6: is a schematic sectional view of an OLED, according to an exemplary embodiment of the present invention.
- FIG. 7: is a schematic sectional view of an OLED, according to an exemplary embodiment of the present invention.

Hereinafter, the figures are illustrated in more detail with reference to examples. However, the present disclosure is not limited to the following figures.

Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.

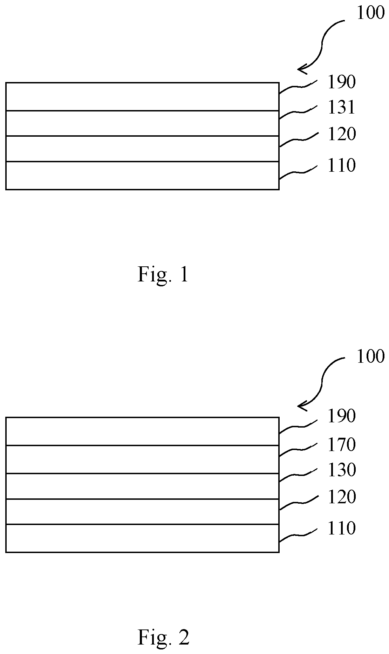

FIG. 1 is a schematic sectional view of an organic electronic device 100, according to an exemplary embodiment of the present invention. The organic electronic device 100 includes an anode layer 120 and an organic semiconductor layer 131 which may comprise a compound of formula (I). The organic semiconductor layer 131 is disposed on the anode layer 120. Onto the organic semiconductor layer 131 a cathode layer 190 are disposed.

FIG. 2 is a schematic sectional view of an organic electronic device 100, according to an exemplary embodiment of the present invention. The organic electronic device 100 includes a substrate 110, an anode layer 120 and a hole injection layer (HIL) 130 which may comprise a compound of formula (I). The HIL 130 is disposed on the anode layer 120. Onto the HIL 130, a photoactive layer (PAL) 170 and a cathode layer 190 are disposed.

FIG. 3 is a schematic sectional view of an organic light-emitting diode (OLED) 100, according to an exemplary embodiment of the present invention. The OLED 100 includes a substrate 110, an anode layer 120 and a hole injection layer (HIL) 130 which may comprise a compound of formula (I). The HIL 130 is disposed on the anode layer 120. Onto the HIL 130, a hole transport layer (HTL) 140, an emission layer (EML) 150, an electron transport layer (ETL) 160, an electron injection layer (EIL) 180 and a cathode layer 190 are disposed. Instead of a single electron transport layer 160, optionally an electron transport layer stack (ETL) can be used.

FIG. 4 is a schematic sectional view of an OLED 100, according to another exemplary embodiment of the present invention. Fig. 4 differs from Fig. 3 in that the OLED 100 of Fig. 4 comprises an electron blocking layer (EBL) 145 and a hole blocking layer (HBL) 155.

Referring to Fig. 4, the OLED 100 includes a substrate 110, an anode layer 120, a hole injection layer (HIL) 130 which may comprise a compound of formula (I), a hole transport layer (HTL) 140, an electron blocking layer (EBL) 145, an emission layer (EML) 150, a hole blocking layer (HBL) 155, an electron transport layer (ETL) 160, an electron injection layer (EIL) 180 and a cathode layer 190.

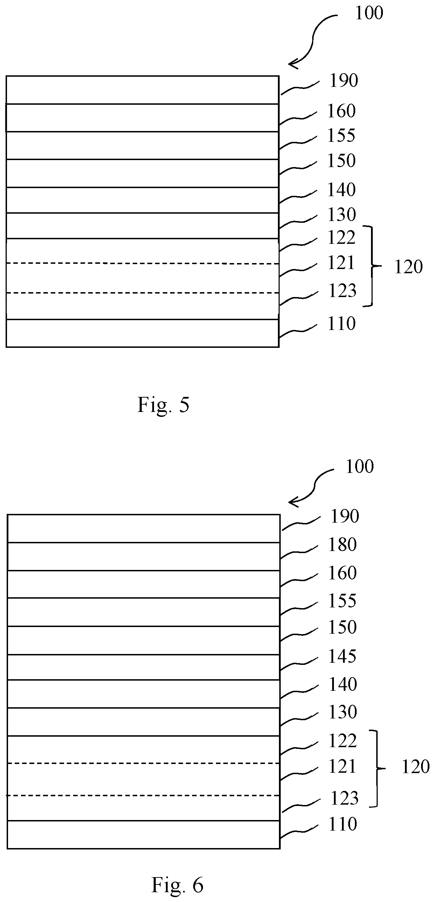

FIG. 5 is a schematic sectional view of an organic electronic device 100, according to an exemplary embodiment of the present invention. The organic electronic device 100 includes a substrate 110, an anode layer 120 that comprises a first anode sub-layer 121, a second anode sub-layer 122 and a third anode sub-layer 123, and a hole injection layer (HIL) 130. The HIL 130 is disposed on the anode layer 120. Onto the HIL 130, an hole transport layer (HTL) 140, a first emission layer (EML) 150, a hole blocking layer (HBL) 155, an electron transport layer (ETL) 160, and a cathode layer 190 are disposed. The hole injection layer 130 may comprise a compound of formula (I).

FIG. 6 is a schematic sectional view of an organic electronic device 100, according to an exemplary embodiment of the present invention. The organic electronic device 100 includes a substrate 110, an anode layer 120 that comprises a first anode sub-layer 121, a second anode sub-layer 122 and a third anode sub-layer 123, and a hole injection layer (HIL) 130. The HIL 130 is disposed on the anode layer 120. Onto the HIL 130, a hole transport layer (HTL) 140, an electron blocking layer (EBL) 145, a first emission layer (EML) 150, a hole blocking layer (HBL) 155, an electron transport layer (ETL) 160, an electron injection layer (EIL) 180 and a cathode layer 190 are disposed. The hole injection layer 130 may comprise a compound of formula (I).

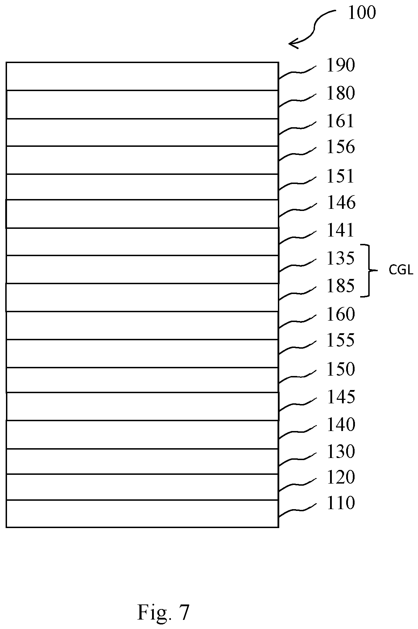

FIG. 7 is a schematic sectional view of an organic electronic device 100, according to an exemplary embodiment of the present invention. The organic electronic device 100 includes a substrate 110, an anode layer 120, a hole injection layer (HIL) 130, a first hole transport layer (HTL) 140, a first electron blocking layer (EBL1) 145, a first emission layer (EMI,1) 150, an optional first hole blocking layer (HBL) 155, a first electron transport layer (ETL1) 160, an n-type charge generation layer (n-CGL) 185, a p-type charge generation layer (p-GCL) 135 which may comprise compound of formula (I), a second hole transport layer (HTL2) 141, a second electron blocking layer (EBL2) 146, a second emission layer (EML2) 151, an optional second hole blocking layer (HBL2) 156, a second electron transport layer (ETL2) 161, an electron injection layer (EIL) 180 and a cathode layer 190. The HIL may also comprise a compound of formula (I). The hole injection layer may comprise compound of formula (I). The anode layer may comprise a first anode sub-layer, a second anode sub-layer, and an optional third anode sub-layer.

While not shown in Fig. 1 to Fig. 7 , a capping and/or sealing layer may further be formed on the cathode layer 190, in order to seal the organic electronic device 100. In addition, various other modifications may be applied thereto.

Hereinafter, one or more exemplary embodiments of the present invention will be described in detail with, reference to the following examples. However, these examples are not intended to limit the purpose and scope of the one or more exemplary embodiments of the present invention.

### Method of preparation

The invention is furthermore illustrated by the following examples which are illustrative only and non-binding.

Compounds of the invention may be prepared by methods known in the art, for example US2005121667A1, or as described below.

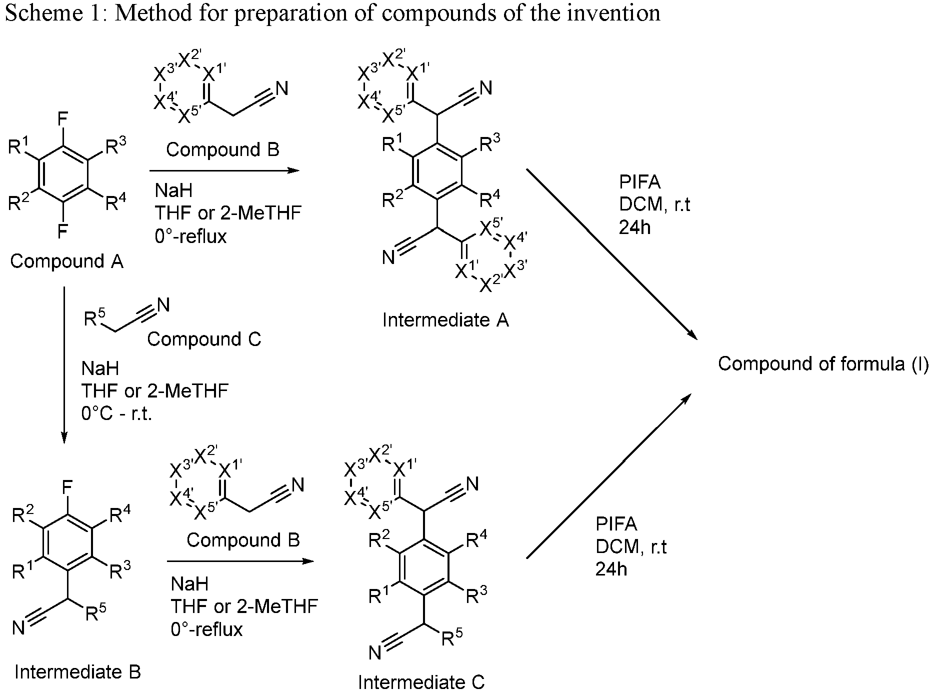

### Intermediate A

In a 3-neck round-bottle flask equipped with a reflux condenser dropping funnel and a septum, 2.4 equivalents of 100% sodium hydride was suspended in dry THF (tetrahydrofuran) or dry 2-MeTHF (2-methyl-tetrahydrofuran) and cooled below 10°C using an ice bath under inert gas atmosphere. After dropwise addition of a solution of 2.3 equivalents of Compound B in anhydrous THF or anhydrous 2-MeTHF, the mixture was stirred for 15 minutes at 0 to 10°C. Then 1 equivalent Compound A was added as a solid in one portion and the mixture was stirred for 15 minutes at 0°C, 15 minutes at room temperature and at 80°C overnight. If the reaction was incomplete on the next morning, the reaction mixture was added dropwise to a suspension of 0.5 to 2 equivalents of additional Compound B deprotonated with a small excess of 100% sodium hydride (0.6 to 2.1 equivalents respectively) and stirred at 80°C for further 24 hours. After completion of the reaction, the mixture was poured on an ice/water mixture and acidified with hydrochloric acid. The aqueous phase was extracted with ethyl acetate twice and the organic phase was then washed with half-concentrated brine three times and additionally with concentrated brine and dried using sodium sulfate. After removal of solvents, the product was stirred in DCM (dichloromethane) over night at room temperature, filtered and dissolved in a small amount of ethyl acetate again. The concentrated solution was then added dropwise into an 8-fold excess of dichloromethane. The precipitated product was filtered, washed with dichloromethane twice and had a purity of > 98% by HPLC.

### Intermediate B

In a 3-neck round-bottle flask equipped with a reflux condenser dropping funnel and a septum, 1.1 equivalents of 100% sodium hydride was suspended in dry THF or dry 2-MeTHF and cooled below 10°C using an ice bath under inert gas atmosphere. After dropwise addition of a solution of 1 equivalent of Compound C in dry THF or dry 2-MeTHF, the mixture was stirred for 15 minutes at 0 to 10°C. Then 1 equivalent Compound A was added as a solid in one portion and the mixture was stirred for 15 minutes at 0°C and at room temperature overnight. If the reaction was found to be incomplete on the next morning, the reaction mixture was added dropwise to a suspension of 0.5 to 1 equivalents of additional Compound C deprotonated with a small excess of 100% sodium hydride (0.6 to 1.1 equivalents respectively) and stirred for further 24 hours at room temperature. After completion of the reaction, the mixture was poured on an ice/water mixture and acidified with hydrochloric acid. The aqueous phase was extracted with ethyl acetate twice and the organic phase was then washed with half-concentrated brine three times and additionally with concentrated brine and dried using sodium sulfate. After removal of solvents, the product was stirred in toluene over night at room temperature, filtered off, washed with toluene several times to yield a purity of >95% by HPLC.

### Intermediate C

In a 3-neck round-bottle flask equipped with a reflux condenser dropping funnel and a septum, 1.5 equivalents of 100% sodium hydride was suspended in dry THF or dry 2-MeTHF and cooled down below 10°C using an ice bath under inert gas atmosphere. After dropwise addition of a solution of 1.4 equivalents of Compound B in anhydrous THF or anhydrous 2-MeTHF, the mixture was stirred for 15 minutes at 0° to 10°C. Then 1 equivalent Intermediate B was added as a solid in one portion and the mixture was stirred for 15 minutes at 0°C, 15 minutes at room temperature and at 80°C overnight. If the reactions was incomplete on the next morning, the reaction mixture was added dropwise to a suspension of 0.5 to 1.5 equivalents of additional Compound B deprotonated with a small excess of 100% sodium hydride (0.6 to 1.6 equivalents respectively) and stirred at 80°C for additional 24 hours. After completion of the reaction, the mixture was poured on an ice/water mixture and acidified with hydrochloric acid. The aqueous phase was extracted with ethyl acetate twice and the organic phase was then washed with half-concentrated brine three times and additionally with concentrated brine and dried using sodium sulfate. After removal of solvents, the product was stirred in DCM over night at room temperature, filtered and dissolved in a small amount of ethyl acetate again. The concentrated solution was then added dropwise into an 8-fold excess of dichloromethane. The precipitated product was filtered, washed with dichloromethane twice and had a purity of >98% by HPLC.

### Experimental data

### Calculated HOMO and LUMO

The HOMO and LUMO are calculated with the program package ORCA V5.0.4 (Max Planck Institute für Kohlenforschung, Kaiser Wilhelm Platz 1, 45470, Muelheim/Ruhr, Germany) and WEASEL 1.11.0 (FAccTs GmbH, Rolandstrasse 67, 50677 Köln, Germany). The dipole moment, the HOMO and LUMO energy levels of the molecular structures are determined by applying the hybrid functional B3LYP with a def2-tzvp basis set from the optimized geometries obtained by applying the composite method HF-3c in the gas phase. All the calculations were performed in the gas phase. If more than one conformation is viable, the conformation with the lowest total energy is selected.

### Dipole moment

The dipole moment of the molecular structures are determined by applying the hybrid functional B3LYP with a def2-tzvp basis set from the optimized geometries obtained by applying the composite method HF-3c in the gas phase. All the calculations were performed in the gas phase. If more than one conformation is viable, the conformation with the lowest total energy is selected.

### General method for determining the sheet resistance of the OLED

The sheet resistance of the OLED, also named "OLED-Rs", is determined in giga Ohm per square (GQ/sq).

The sheet resistance Rs may be determined via the transfer length method. The measurement may be conducted by methods known in the art or as described in WO2022263524A1.

For the examples according to the invention and comparative examples in Table 7, the OLED-Rs is determined as follows:

In a first step, the complete OLED as described in the examples and comparative examples is deposited.

In a second step, the sheet resistance Rs is determined for each device by using the transfer length method, while the cathode is not connected.

The higher the sheet resistance, the lower is the current flow between adj acent pixels. Thereby, individual pixels in an electronic device can be addressed without adjacent pixels lighting up. Thereby, the so-called pixel crosstalk may be reduced.

### Glass transition temperature

The glass transition temperature (Tg) is measured under nitrogen and using a heating rate of 10 K per min in a Mettler Toledo DSC 822e differential scanning calorimeter as described in DIN EN ISO 11357, published in March 2010.

### Melting point (mp)

Melting point (mp) temperatures were measured by DSC at heating rate 10 K/min, the reported values correspond the peak temperature for the observed melting endotherm on the DSC curve.

### Thermogravimetric analysis

The term "TGA5%" denotes the temperature at which 5 % weight loss occurs during thermogravimetric analysis and is measured in °C.

The TGA5% value may be determined by heating a 9-11 mg sample in a thermogravimetric analyzer at a heating rate of 10 K/min in an open 100 µL aluminum pan, under a stream of nitrogen at a flow rate of 20 mL/min in the balance area and of 30 mL/min in the oven area.

The TGA5% value may provide an indirect measure of the volatility and/or decomposition temperature of a compound. In first approximation, the higher the TGA5% value the lower is the volatility of a compound and/or the higher the decomposition temperature.

### Rate onset temperature

The rate onset temperature (T_{RO}) is determined by loading 100 mg compound into a VTE source. As VTE source a point source for organic materials may be used as supplied by Kurt J. Lesker Company (www.lesker.com) or CreaPhys GmbH (http://www.creaphys.com). The VTE source is heated at a constant rate of 15 K/min at a pressure of less than 10⁻⁵ mbar and the temperature inside the source measured with a thermocouple. Evaporation of the compound is detected with a QCM detector which detects deposition of the compound on the quartz crystal of the detector. The deposition rate on the quartz crystal is measured in Ǻngstrom per second. To determine the rate onset temperature, the deposition rate is plotted against the VTE source temperature. The rate onset is the temperature at which noticeable deposition on the QCM detector occurs. For accurate results, the VTE source is heated and cooled three time and only results from the second and third run are used to determine the rate onset temperature.

To achieve good control over the evaporation rate of an organic compound, the rate onset temperature may be in the range of 175 to 280 °C. If the rate onset temperature is below 175 °C the evaporation may be too rapid and therefore difficult to control. If the rate onset temperature is above 280 °C the evaporation rate may be too low which may result in low tact time and decomposition of the organic compound in VTE source may occur due to prolonged exposure to elevated temperatures.

The rate onset temperature is an indirect measure of the volatility of a compound. The higher the rate onset temperature the lower is the volatility of a compound.

### General procedure for fabrication of OLEDs

For inventive examples and comparative examples in Table 8, a glass substrate with an anode layer comprising a first anode sub-layer of 120 nm Ag, a second anode sub-layer of 8 nm ITO and a third anode sub-layer of 10 nm ITO was cut to a size of 25 mm x 25 mm x 0.7 mm, ultrasonically washed with water for 60 minutes and then with isopropanol for 20 minutes. The liquid film was removed in a nitrogen stream, followed by plasma treatment to prepare the anode layer. The plasma treatment was performed in an atmosphere comprising 98.4 vol.-% nitrogen and 1.6 vol.-% oxygen.

Then, a metal complex, which may be the compound of Formula (I) or a comparative compound, and N-(9,9-diphenyl-9H-fluoren-2-yl)-N,9-diphenyl-9H-carbazol-2-amine ([1607480-22-7]) as a matrix compound were co-deposited in vacuum on the anode layer, to form a hole injection layer (HIL) having a thickness of 10 nm. The composition of the HIL can be seen in Table 8. The formulae of the quinone of formula (I) and comparative compounds can be seen in Table 6.

Then, N-(9,9-diphenyl-9H-fluoren-2-yl)-N,9-diphenyl-9H-carbazol-2-amine ([1607480-22-7]) was vacuum deposited on the HIL, to form a HTL having a thickness of 128 nm. The compound that forms the HTL is selected the same as the matrix compound in the HIL.

Then N,N-di([1,1'-biphenyl]-4-yl)-3'-(9H-carbazol-9-yl)-[1,1'-biphenyl]-4-amine ([CAS 1464822-27-2]) was vacuum deposited on the HTL, to form an electron blocking layer (EBL) having a thickness of 5 nm.

Then the emission layer (EML) having a thickness of 20 nm was formed on the EBL by co-depositing 99 vol.-% 7-(phenyl-d5)-1-(10-(phenyl-d5)anthracen-9-yl)dibenzo[b,d]furan ([2457172-82-4]) as EML host and 1 vol.-% 5H ,9H -[1]Benzothieno[2',3':5,6][1,4]-azaborino[2,3,4-kl ]phenazaborine, 2,7,11-tris(1,1-dimethylethyl)-5,9-bis[4-(1,1-dimethylethyl)phenyl] ([2482607-57-6]) as blue dopant.

Then a hole blocking layer (HBL) having a thickness of 5 nm was formed on the EML by depositing 4-([1,1'-biphenyl]-4-yl)-6-(3'-(9,9-dimethyl-9H-fluoren-4-yl)-[1,1'-biphenyl]-4-yl)-2-phenylpyrimidine ([CAS 1955546-40-3]).

Then the electron transport layer (ETL) having a thickness of 31 nm was formed on the hole blocking layer by vacuum depositing 50 wt.-% 6,6'-(naphthalene-1,2-diylbis(4,1-phenylene))bis(2,4-diphenyl-1,3,5-triazine) ([CAS 2244287-14-5]) and 50 wt.-% of LiQ (8-Hydroxyquinolinato)lithium; ([25387-93-3] or [850918-68-2]).

Then an electron injection layer (EIL) was formed on the electron transport layer by vacuum depositing a layer of 2 nm Yb.

Then Ag:Mg (90:10 vol.-%) was evaporated at a rate of 0.01 to 1 Å/s at 10⁻⁷ mbar to form a cathode layer with a thickness of 13 nm on the EIL.

Then, N-([1,1'-biphenyl]-4-yl)-9,9-dimethyl-N-(4-(9-phenyl-9H-carbazol-3-yl)-phenyl)-9H-fluoren-2-amine ([1242056-42-3]) was deposited on the cathode layer to form a capping layer with a thickness of 75 nm.

To assess the performance of the inventive examples compared to the prior art, the current efficiency is measured at 20°C. The current-voltage characteristic is determined using a Keithley 2635 source measure unit, by sourcing a voltage in V and measuring the current in mA flowing through the device under test. The voltage applied to the device is varied in steps of 0.1V in the range between 0V and 10V. Likewise, the luminance-voltage characteristics and CIE coordinates are determined by measuring the luminance in cd/m² using an Instrument Systems CAS-140CT array spectrometer (calibrated by Deutsche Akkreditierungsstelle (DAkkS)) for each of the voltage values. The cd/A efficiency at 15 mA/cm² is determined by interpolating the luminance-voltage and current-voltage characteristics, respectively.

In bottom emission devices, the emission is predominately Lambertian and quantified in percent external quantum efficiency (EQE). The light is emitted through the anode layer. To determine the efficiency EQE in % the light output of the device is measured using a calibrated photodiode at 15 mA/cm2.

In top emission devices, the emission is forward directed through the cathode layer, non-Lambertian and also highly dependent on the mirco-cavity. Therefore, the efficiency EQE will be higher compared to bottom emission devices. To determine the efficiency EQE in % the light output of the device is measured using a calibrated photodiode at 15 mA/cm².

Lifetime LT of the device is measured at ambient conditions (20°C) and 20 mA/cm², using a Keithley 2400 source meter, and recorded in hours.

The brightness of the device is measured using a calibrated photo diode. The lifetime LT is defined as the time till the brightness of the device is reduced to 97 % of its initial value.

To determine the voltage stability over time U(100h)-(1h), a current density of at 20 mA/cm² was applied to the device. The operating voltage was measured after 1 hour and after 100 hours, followed by calculation of the voltage stability for the time period of 1 hour to 100 hours. A low value for U(100h)-(1h) denotes a low increase in operating voltage over time and thereby improved voltage stability.

### Technical Effect of the invention

In Table 5, the LUMO energy level, Egap (Difference between HOMO energy level and LUMO energy level), and dipole moment of compounds of formula (I) are shown.

**Table 5: LUMO energy level, Egap (Difference between HOMO energy level and LUMO energy level), and dipole moment of compounds of formula (I)**

| | | Compound | LUMO [eV] | Dipole moment [debye] | Egap [eV] |
|---|---|---|---|---|---|
| Comparative compound | K-1 | 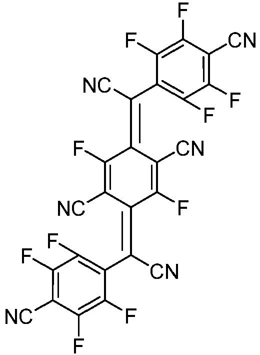 | -5.06 | 0 | 3.03 |
| Comparative compound | K-2 | 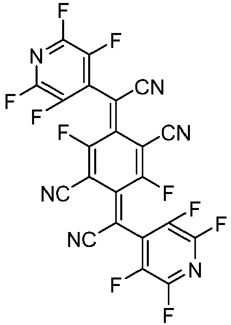 | -4.98 | 0 | 2.96 |
| Comparative compound | K-3 | 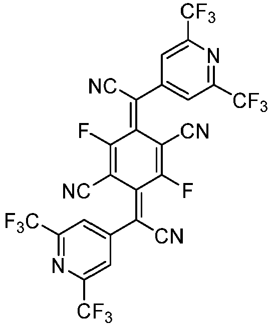 | -5.11 | 0 | 3.02 |
| Inventive compound | A1 | 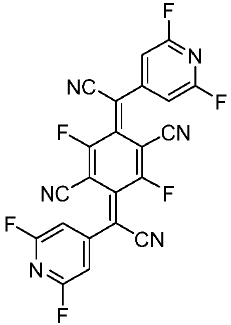 | -4.75 | 0.26 | 3.23 |
| Inventive compound | A2 | 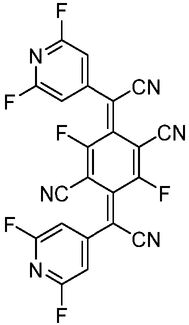 | -4.76 | 0.99 | 3.23 |
| Inventive compound | A3 | 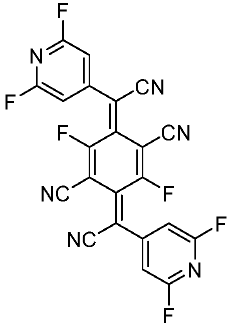 | -4.83 | 0.00 | 3.08 |
| Inventive compound | A4 | 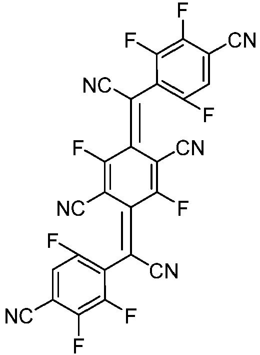 | -4.86 | 1.04 | 3.12 |
| Inventive compound | A5 | 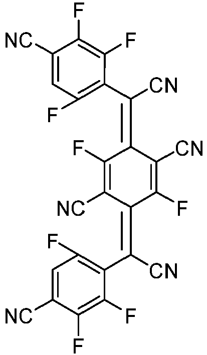 | -4.86 | 1.51 | 3.10 |
| Inventive compound | A6 | 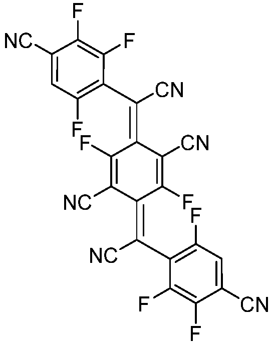 | -4.82 | 1.61 | 3.17 |
| Inventive compound | A7 | 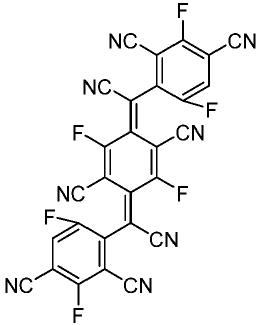 | -4.82 | 3.86 | 3.21 |
| Inventive compound | A8 | 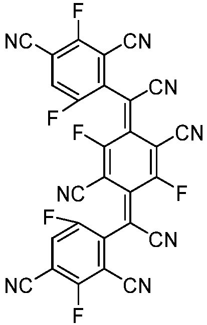 | -5.06 | 6.79 | 3.08 |
| Inventive compound | A9 | 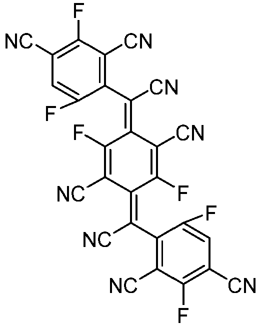 | -5.06 | 6.79 | 3.08 |
| Inventive compound | A10 | 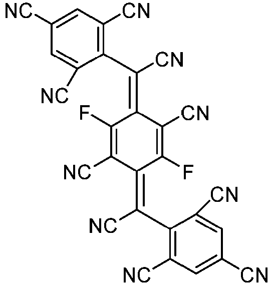 | -4.89 | 1.18 | 3.34 |
| Inventive compound | A11 | 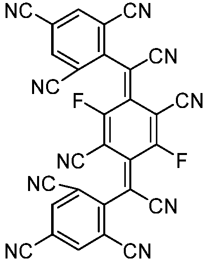 | -5.04 | 5.10 | 3.18 |
| Inventive compound | A12 | 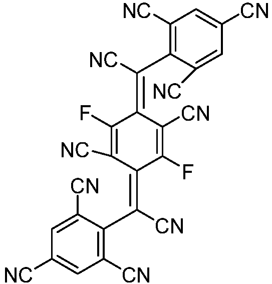 | -5.08 | 0.00 | 3.12 |
| Inventive compound | A13 | 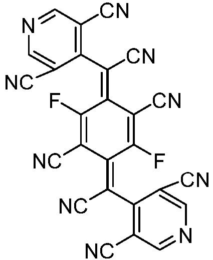 | -4.81 | 2.28 | 3.37 |
| Inventive compound | A14 | 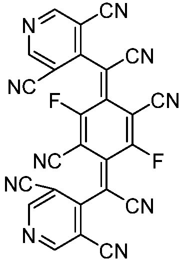 | -4.96 | 7.87 | 3.20 |
| Inventive compound | A15 | 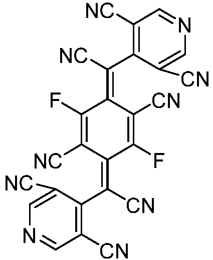 | -4.85 | 2.39 | 3.34 |
| Inventive compound | B1 | 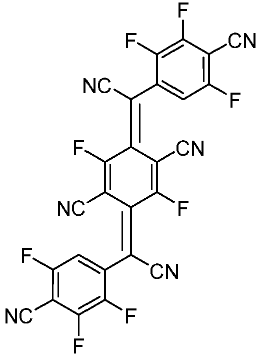 | -4.92 | 1.49 | 3.09 |
| Inventive compound | B2 | 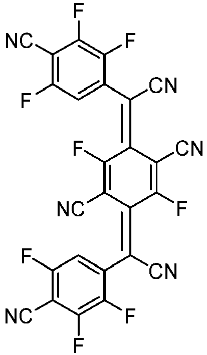 | -4.94 | 0.68 | 3.11 |
| Inventive compound | B3 | 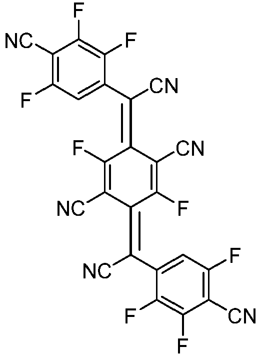 | -4.89 | 1.78 | 3.16 |
| Inventive compound | B4 | 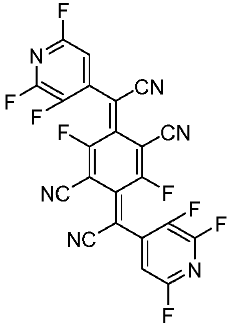 | -4.80 | 1.39 | 3.16 |
| Inventive compound | B5 | 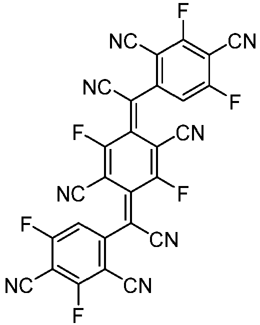 | -5.08 | 0.00 | 3.02 |
| Inventive compound | B6 | 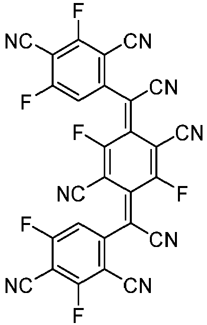 | -5.05 | 5.32 | 3.09 |
| Inventive compound | B7 | 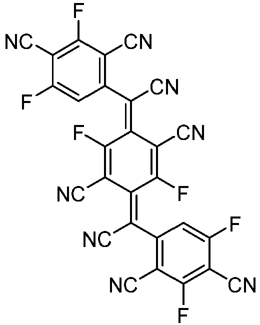 | -4.83 | 4.14 | 3.32 |
| Inventive compound | B8 | 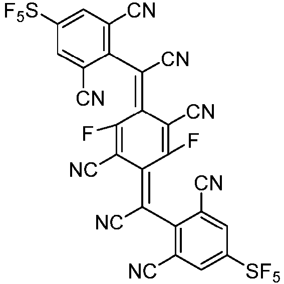 | -4.66 | 2.66 | 3.38 |
| Inventive compound | B9 | 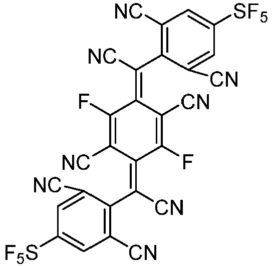 | -4.72 | 2.58 | 3.32 |
| Inventive compound | B10 | 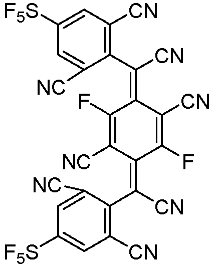 | -4.83 | 8.74 | 3.20 |
| Inventive compound | B11 | 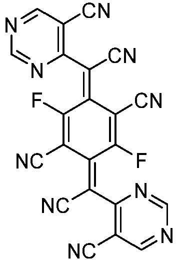 | -4.84 | 0.00 | 3.07 |
| Inventive compound | B12 | 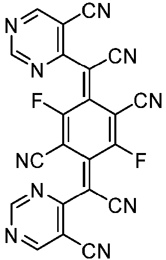 | -4.81 | 837 | 3.20 |
| Inventive compound | B13 | 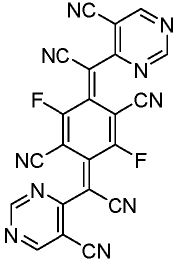 | -4.81 | 4.02 | 3.21 |
| Inventive compound | B14 | 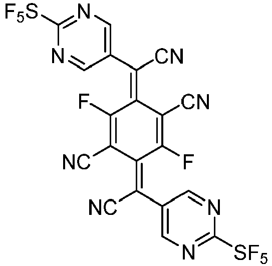 | -4.86 | 0.89 | 3.06 |
| Inventive compound | B15 | 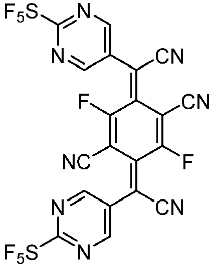 | -4.89 | 0.75 | 2.99 |
| Inventive compound | B16 | 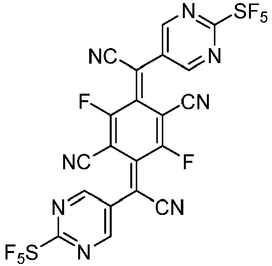 | -4.87 | 0.69 | 3.00 |
| Inventive compound | B17 | 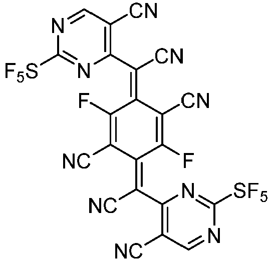 | -4.91 | 1.37 | 3.31 |
| Inventive compound | B18 | 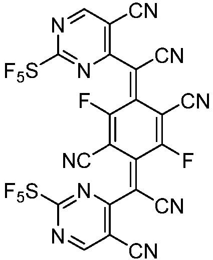 | -5.03 | 5.33 | 3.18 |
| Inventive compound | B19 | 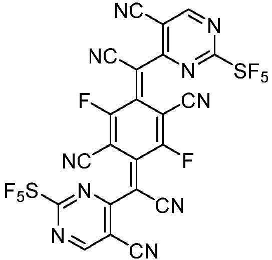 | -4.94 | 1.44 | 3.30 |
| Inventive compound | B20 | 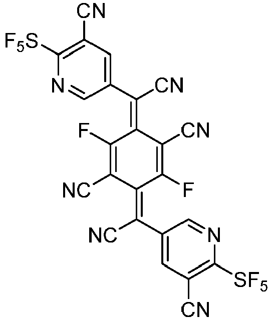 | -5.00 | 2.93 | 3.13 |
| Inventive compound | B21 | 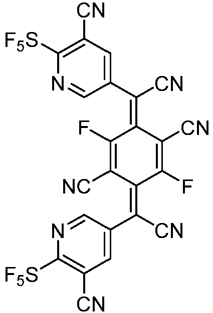 | -5.03 | 2.22 | 3.08 |
| Inventive compound | B22 | 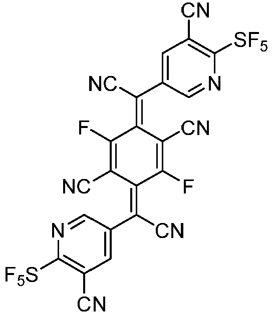 | -5.01 | 2.98 | 3.08 |
| Inventive compound | C1 | 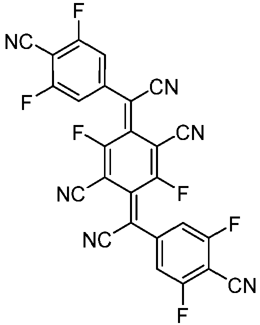 | -4.94 | 0.00 | 2.94 |
| Inventive compound | C2 | 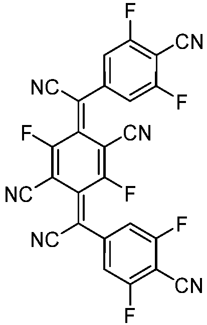 | -4.86 | 2.29 | 3.08 |
| Inventive compound | C3 | 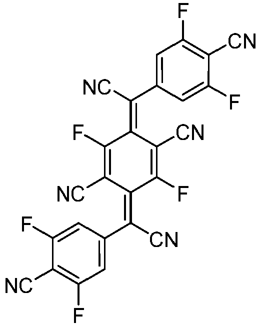 | -4.92 | 0.00 | 2.96 |
| Inventive compound | C4 | 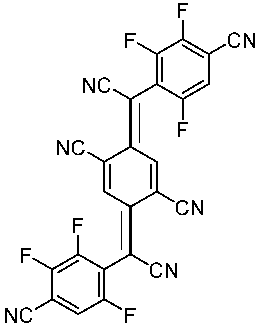 | -4.75 | 1.96 | 3.00 |
| Inventive compound | C5 | 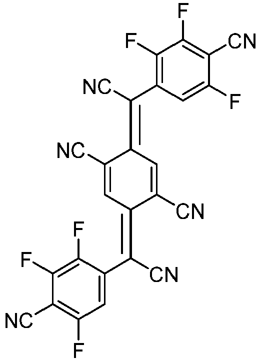 | -4.81 | 0.35 | 3.02 |
| Inventive compound | C6 | 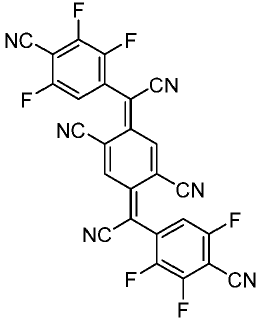 | -4.92 | 2.53 | 3.06 |
| Inventive compound | C7 | 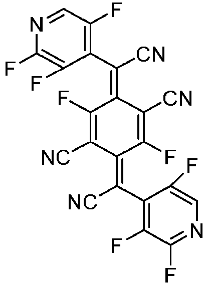 | -4.72 | 1.62 | 3.23 |
| Inventive compound | C8 | 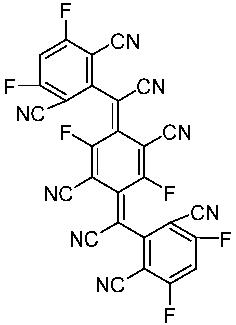 | -4.66 | 3.23 | 3.40 |
| Inventive compound | C9 | 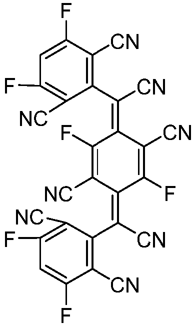 | -4.84 | 10.04 | 3.22 |
| Inventive compound | C10 | 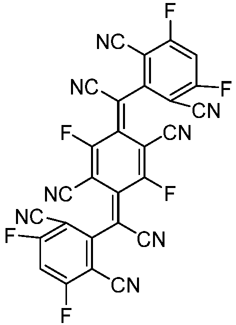 | -4.73 | 3.23 | 3.33 |
| Inventive compound | C11 | 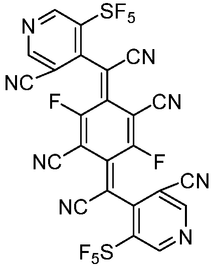 | -4.64 | 1.72 | 3.38 |
| Inventive compound | C12 | 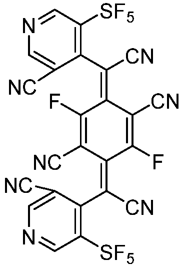 | -4.72 | 7.82 | 3.24 |
| Inventive compound | C13 | 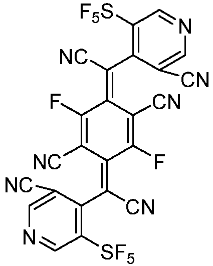 | -4.71 | 3.61 | 3.27 |
| Inventive compound | C14 | 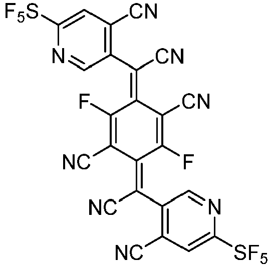 | -4.68 | 0.74 | 3.25 |
| Inventive compound | C15 | 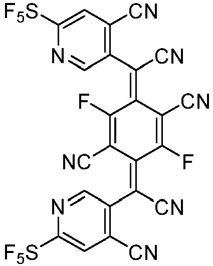 | -4.84 | 3.82 | 3.10 |
| Inventive compound | C16 | 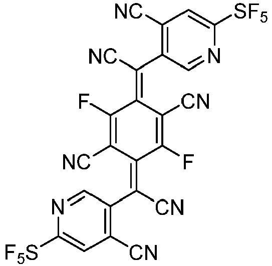 | -4.71 | 0.82 | 3.2 |
| Inventive compound | C17 | 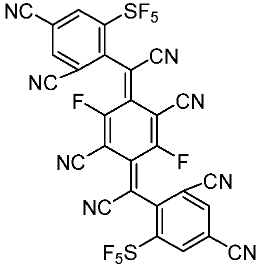 | -4.78 | 4.01 | 3.33 |
| Inventive compound | C18 | 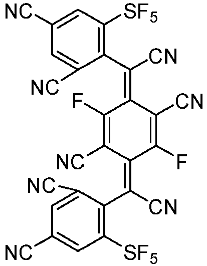 | -4.82 | 3.94 | 3.24 |
| Inventive compound | C19 | 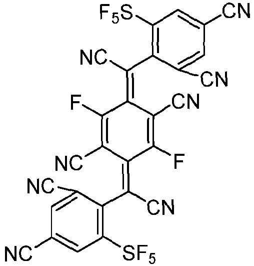 | -4.81 | 4.01 | 3.27 |
| Inventive compound | C20 | 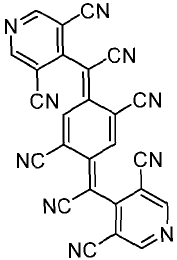 | -4.77 | 0.00 | 3.23 |
| Inventive compound | C21 | 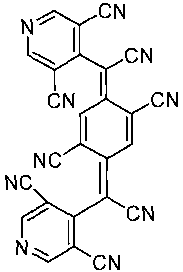 | -4.86 | 7.78 | 3.13 |
| Inventive compound | C22 | 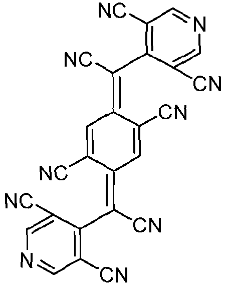 | -4.83 | 2.62 | 3.21 |
| Inventive compound | C23 | 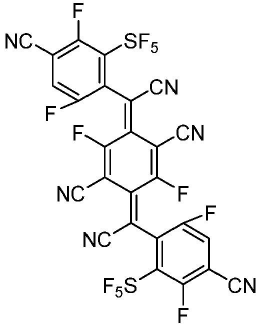 | -4.74 | 5.91 | 3.15 |
| Inventive compound | C24 | 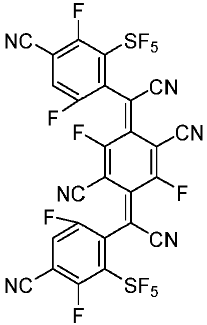 | -4.78 | 0.94 | 3.26 |
| Inventive compound | C25 | 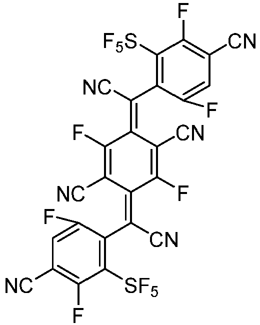 | -4.78 | 1.62 | 3.27 |
| Inventive compound | C26 | 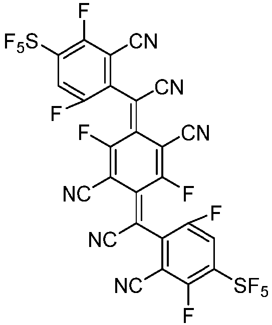 | -4.53 | 1.98 | 3.33 |
| Inventive compound | C27 | 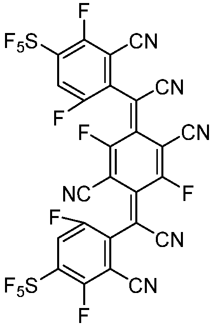 | -4.74 | 5.91 | 3.15 |
| Inventive compound | C28 | 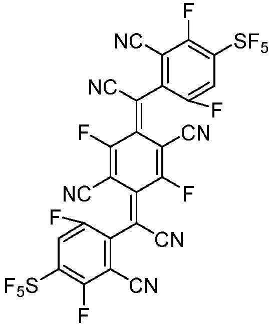 | -4.57 | 2.34 | 3.26 |
| Inventive compound | D1 | 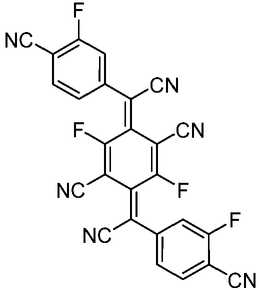 | -4.81 | 0.00 | 2.87 |
| Inventive compound | D2 | 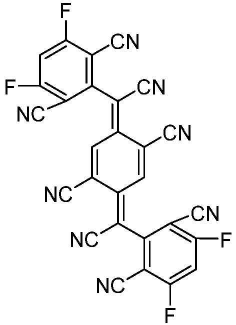 | -4.68 | 0.00 | 3.24 |
| Inventive compound | D3 | 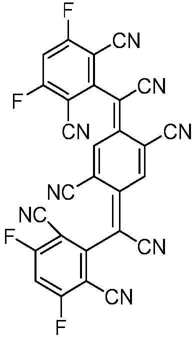 | -4.74 | 10.06 | 3.14 |
| Inventive compound | D4 | 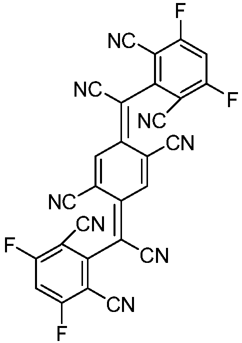 | -4.70 | 2.62 | 3.21 |

With respect to Table 5, the comparative compound K-1 comprises a phenyl substituent having four F groups and one CN group. Comparative compound K-1 exhibits a LUMO energy level of -5.06 eV, a dipole moment of 0 debye and an Egap of 3.03 eV.

The inventive compound A4 comprises a phenyl substituent having one hydrogen group, three F groups and one CN group. Inventive compound A4 exhibits a LUMO energy level of -4.86 eV, a dipole moment of 1.04 debye, and an Egap of 3.12 eV.

Thus, the inventive compound A4 having one hydrogen group at the phenyl substituent exhibits a larger LUMO energy level than the comparative compound K-1 having no hydrogen at the phenyl substituent.

The comparative compound K-2 comprises a pyridyl substituent having four F groups. Comparative compound K-2 exhibits a LUMO energy level of -4.98 eV, a dipole moment of 0 debye, and an Egap of 2.96 eV.

The inventive compound A3 comprises a pyridyl substituent having two F groups and 2 hydrogen groups. Inventive compound A3 exhibits a LUMO energy level of -4.83 eV, a dipole moment of 0 debye, and an Egap of 3.08 eV.

Thus, the inventive compound A3 having two hydrogen group at the pyridyl substituent exhibits a larger LUMO energy level than comparative compound K-2 having no hydrogen group at the pyridyl substituent.

The comparative compound K-3 comprises a pyridyl substituent having two CF₃ groups, and two hydrogen groups. Comparative compound K-3 exhibits a LUMO energy level of -5.11 eV, a dipole moment of 0 debye and an Egap of 3.02 eV.

The inventive compound A1 comprises a pyridyl substituent having two F groups and 2 hydrogen groups. Inventive compound A exhibits a LUMO energy level of -4.75 eV, a dipole moment of 0.26 debye, and an Egap of 3.23 eV.

Thus, the inventive compound A1 having two F groups and two hydrogen groups exhibits a larger LUMO energy level than the comparative compound K-3 having two CF₃ groups, and two hydrogen groups.

A larger value of the LUMO energy level may be beneficial for increasing the sheet resistance of an organic semiconductor. Thereby, the cross-talk between two pixels in a display may be decreased, and the color purity in a display device or resolution in a display may be improved.

In Table 5, LUMO energy level, dipole moment and Egap for further inventive compounds are shown.

With respect to Table 7, the comparative compound K-1 comprising a phenyl substituent having four F groups and one CN group exhibits a melting point Tm of 323°C, a rate onset temperature T_{RO} of 170°C, and a TGA5% of 366°C.

The inventive compound A4 comprising a phenyl substituent having one hydrogen group, three F groups and one CN group exhibits a glass transition temperature of 120°C, a melting point Tm of 350°C, a rate onset temperature of 240°C, and a TGA5% of 377°C.

The inventive compound C1 comprising a phenyl substituent having one hydrogen group, three F groups and one CN group exhibits a melting point Tm of 356°C, and a TGA5% of 413°C.

Thus, the inventive compound A4 having one hydrogen group at the phenyl substituent exhibits a higher glass transition temperature, a higher melting point, a higher rate onset temperature and a higher TGA5% than the comparative compound K-1 having no hydrogen at the phenyl substituent; and the inventive compound B3 having one hydrogen group at the phenyl substituent exhibits a higher melting point, and a higher TGA5% than the comparative compound K-1 having no hydrogen at the phenyl substituent.

The comparative compound K-2 comprising a pyridyl substituent having four F groups exhibits a glass transition temperature of 104°C, a melting point Tm of 293°C, a rate onset temperature of 161°C, and a TGA5% of 312°C.

The comparative compound K-3 comprising a pyridyl substituent having two CF₃ groups, and two hydrogen groups exhibits a melting point Tm of 305°C, a rate onset temperature of 181°C, and a TGA5% of 336°C .

The inventive compound A1 comprising a pyridyl substituent having two F groups and 2 hydrogen groups exhibits a melting point Tm of 350°C, a rate onset temperature of 240°C, and a TGA5% of 377°C .

Thus, the inventive compound A1 having two F groups and two hydrogen groups exhibits a higher rate onset temperature and a higher TGA5% than and the comparative compound K-2 having no hydrogen group at the pyridyl substituent, and the comparative compound K-3 having two CF₃ groups, and two hydrogen groups.

A high rate onset temperature may be desirable for good control of the evaporation rate in mass production of organic electronic devices.

A high TGA5% may be desirable for improved thermal stability.

A high melting may be beneficial for processing stability, i.e. avoiding decomposition and reduction of risk of clogging of nozzles if linear source systems are utilized.

A high glass transition temperature may be beneficial for increased temperature stability during bake-out processes which are necessary in mass production.

In Table 8, data are shown for an organic electroluminescent device comprising an organic semiconductor layer comprising a compound of formula (I) or a comparative compound.

In comparative example 1, the organic semiconductor layer comprises 2 vol% of comparative compound K-1 and 98 vol% of N-(9,9-diphenyl-9H-fluoren-2-yl)-N,9-diphenyl-9H-carbazol-2-amine. As can be seen in Table 8, the operating voltage is 3.55 V, the current efficiency (Ceff) is 10.5 cd/A, the external quantum efficiency (EQE or Qeff) is 20.8%, the voltage rise over time U(100h) -U(lh) is 0.122 V, and the lifetime LT97 is 110h. In addition, the sheet resistance (Rs) is 3021 GS2/sq.

In comparative example 2, the organic semiconductor layer comprises 4 vol% of comparative compound K-2 and 96 vol% of N-(9,9-diphenyl-9H-fluoren-2-yl)-N,9-diphenyl-9H-carbazol-2-amine. As can be seen in Table 8, the operating voltage is 3.52 V, the current efficiency (Ceff) is 10.6 cd/A, the external quantum efficiency (EQE or Qeff) is 21.0%, the voltage rise over time U(100h) -U(1h) is 0.074 V, and the lifetime LT97 is 116 h. In addition, the sheet resistance (Rs) is 2661 GΩ/sq.

In comparative example 3, the organic semiconductor layer comprises 1.4 vol% of comparative compound K-3 and 98.6 vol% of N-(9,9-diphenyl-9H-fluoren-2-yl)-N,9-diphenyl-9H-carbazol-2-amine. As can be seen in Table 8, the operating voltage is 3.52 V, the current efficiency (Ceff) is 10.7 cd/A, the external quantum efficiency (EQE or Qeff) is 21.2%, the voltage rise over time U(100h) -U(1h) is 0.084 V, and the lifetime LT97 is 123 h. In addition, the sheet resistance (Rs) is 3338 GS2/sq.

In inventive example 1, the organic semiconductor layer comprises 7.1 vol% of compound of formula (I) A1 and 92.9 vol% of N-(9,9-diphenyl-9H-fluoren-2-yl)-N,9-diphenyl-9H-carbazol-2-amine. As can be seen in Table 8, the operating voltage is improved 3.47 V, the current efficiency (Ceff) is good with a value of 10.5 cd/A, the external quantum efficiency (EQE or Qeff) is good with a value of 20.8%, the voltage rise over time U(100h) -U(lh) is improved to 0.066 V, the lifetime LT97 is improved to 124 h, and the sheet resistance (Rs) is improved to 8989 GΩ/sq compared to comparative examples 1 to 3.

In inventive example 2, the organic semiconductor layer comprises 9.7 vol% of compound of formula (I) A1 and 90.3 vol% of N-(9,9-diphenyl-9H-fluoren-2-yl)-N,9-diphenyl-9H-carbazol-2-amine. As can be seen in Table 8, the operating voltage is improved to 3.47 V, the current efficiency (Ceff) is good with a value of 10.5 cd/A, the external quantum efficiency (EQE or Qeff) is good with as values of 20.5%, the voltage rise over time U(100h) -U(lh) is improved to 0.058 V, the lifetime LT97 is improved to 127 h, and the sheet resistance (Rs) is improved to 5943 GΩ/sq compared to the comparative examples 1 to 3..

In inventive example 3, the organic semiconductor layer comprises 8 vol% of compound of formula (I) A4 and 92 vol% of N-(9,9-diphenyl-9H-fluoren-2-yl)-N,9-diphenyl-9H-carbazol-2-amine. As can be seen in Table 8, the operating voltage is improved to 3.44 V, the current efficiency (Ceff) is good with a value of 10.4 cd/A, the external quantum efficiency (EQE or Qeff) is good with a value of 20.5%, the voltage rise over time U(100h) -U(lh) is improved to 0.067 V, the lifetime LT97 is improved to 127 h, the sheet resistance (Rs) is improved 11310 GΩ/sq compared to the comparative examples 1 to 3.

In inventive example 4, the organic semiconductor layer comprises 11 vol% of compound of formula (I) and 89 vol% of N-(9,9-diphenyl-9H-fluoren-2-yl)-N,9-diphenyl-9H-carbazol-2-amine. As can be seen in Table 8, the operating voltage is improved to 3.45 V, the current efficiency (Ceff) is good with a value of 10.4 cd/A, the external quantum efficiency (EQE or Qeff) is good with a value of 20.4%, the voltage rise over time U(100h) -U(lh) is improved to 0.063 V, the lifetime LT97 is improved to 125 h, and the sheet resistance (Rs) is improved to 7079 GΩ/sq compared to the comparative examples 1 to 3.

In summary, a substantial improvement of the sheet resistance, operating voltage, voltage rise over time and/or lifetime have been obtained without detrimental effect on the current efficiency and/or EQE.

A lower operating voltage may be important for a lower power consumption of organic electronic devices, in particular mobile devices.

A long lifetime may result in improved long-term stability of electronic devices.

A low voltage rise over time may result in improved long-term stability of electronic devices.

A high efficiency may be beneficial for low power consumption of organic electronic devices, in particular in mobile devices.

An improved sheet resistance may result in improved pixel crosstalk. Thereby, the color purity in a display device or resolution in a display may be improved.

**Table 6:Compounds of formula (I) and comparative compounds**

| | | |
|---|---|---|
| Comparative compound | K-1 | 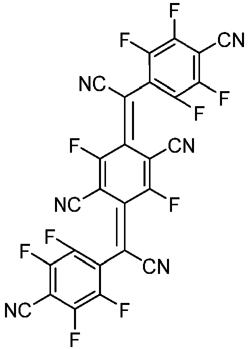 |
| Comparative compound | K-2 | 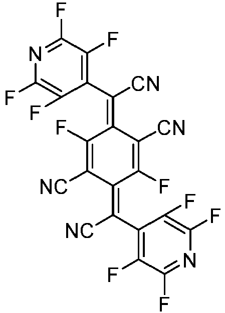 |
| Comparative compound | K-3 | 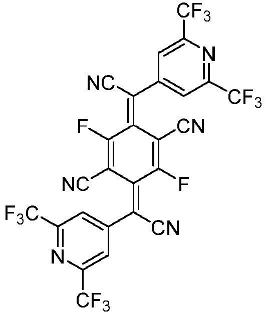 |
| Inventive compound | A1 | 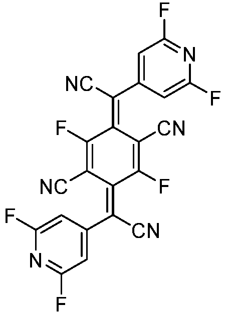 |
| Inventive compound | A4 | 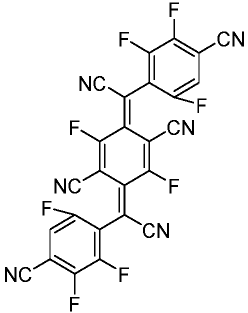 |
| Inventive compound | C1 | 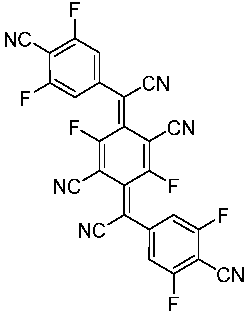 |

**Table 7: Properties of compounds of formula (I) and comparative compounds**

| Example | Compound | Tg [°C] | Tm [°C] | T_{RO} [°C] | [TGA5%] |
|---|---|---|---|---|---|
| Comparative compound example | K-1 | Not observed | 323 | 170 | 366 |
| Comparative example | K-2 | 104 | 293 | 161 | 312 |
| Comparative example | K-3 | Not observed | 305 | 181 | 336 |
| Inventive example | A1 | Not observed | 294 | 226 | 360 |
| Inventive example | A4 | 120 | 350 | 240 | 377 |
| Inventive example | C1 | --- | 356 | --- | 413 |

**Table 8: Performance and stability of organic electroluminescent devices and comparative examples**

| | Quinone in HIL | Concentration of quinone in HIL [vol%] | Operating Voltage at 15 mA/cm² [V] | QEff at 15 mA/cm² [%] | CEff at 15 mA/cm² [cd/A] | CEff/CIEy at 15 mA/cm² [cd/A] | LT97 at 20mA/cm² [h] | Voltage rise (U(100 h) - U(1h)), at 20mA/cm²) [V] | OLED-Rs [GΩ/sq] |
|---|---|---|---|---|---|---|---|---|---|
| Comparative Example 1 | K-1 | 2% | 3.55 | 20.8 | 10.5 | 211.4 | 110 | 0.122 | 3021 |
| Comparative Example 2 | K-2 | 4% | 3.52 | 21.0 | 10.6 | 213.8 | 116 | 0.074 | 2661 |
| Comparative Example 3 | K-3 | 1.4% | 3.52 | 21.2 | 10.7 | 216.7 | 123 | 0.084 | 3338 |
| Example 1 | A1 | 7.1% | 3.47 | 20.8 | 10.5 | 210.9 | 124 | 0.066 | 8989 |
| Example 2 | A1 | 9.7% | 3.47 | 20.5 | 10.5 | 207.7 | 127 | 0.058 | 5943 |
| Example 3 | A4 | 8% | 3.44 | 20.5 | 10.4 | 207.5 | 127 | 0.067 | 11310 |
| Example 4 | A4 | 11% | 3.45 | 20.4 | 10.4 | 206.4 | 125 | 0.063 | 7079 |

The particular combinations of elements and features in the above detailed embodiments are exemplary only; the interchanging and substitution of these teachings with other teachings in this and the patents/applications incorporated by reference are also expressly contemplated. As those skilled in the art will recognize, variations, modifications, and other implementations of what is described herein can occur to those of ordinary skill in the art without departing from the spirit and the scope of the invention as claimed. Accordingly, the foregoing description is by way of example only and is not intended as limiting. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. The invention's scope is defined in the following claims and the equivalents thereto. Furthermore, reference signs used in the description and claims do not limit the scope of the invention as claimed.

## Claims

1. A compound of formula (I): 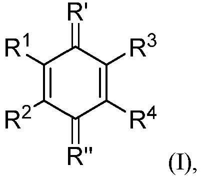 wherein
R¹, R², R³ and R⁴ are independently selected from the group comprising H, D, F or CN; wherein
two R¹, R², R³ and R⁴ are selected from CN and two R¹, R², R³ and R⁴ are selected from H, D or F;
R' is selected from formula (II): 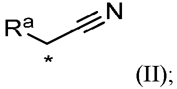
R^{a} is selected from formula (III): 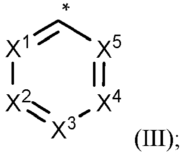
wherein the asterisks "*" denotes the binding position;
wherein
X¹ is selected from CR⁶ or N;
X² is selected from CR⁷ or N;
X³ is selected from CR⁸ or N;
X⁴ is selected from CR⁹ or N;
X⁵ is selected from CR¹⁰ or N;
wherein
R⁶, R⁷, R⁸, R⁹ and R¹⁰ are independently selected from CN, unsubstituted C₁ to C₈ alkyl, substituted or unsubstituted C₆ to C₁₈ aryl, substituted or unsubstituted C₃ to C₁₈ heteroaryl F, SF₅, D or H,
wherein the one or more substituent on R⁶, R⁷, R⁸, R⁹ and R¹⁰ are independently selected from CN, F, SF₅, D;
wherein
at least one X¹ to X⁵ are independently selected from CH or CD and at least one R⁶ to R¹⁰ is CN or F;
R" is selected from formula (IV): 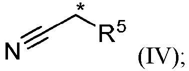
wherein the asterisks "*" denotes the binding position;
wherein
R⁵ is selected from substituted or unsubstituted C₆ to C₁₉ aryl, substituted or unsubstituted C₆ to C₁₉ aryl with one, two or three aromatic six-member rings, substituted or unsubstituted C₉ to C₁₉ aryl with two or three fused aromatic six-member rings, substituted or unsubstituted C₆ to C₁₂ aryl with one or two aromatic six-member rings, substituted or unsubstituted phenyl, substituted or unsubstituted biphenyl, substituted or unsubstituted C₃ to C₂₀ heteroaryl, substituted or unsubstituted C₃ to C₂₀ heteroaryl with one, two or three aromatic six-member rings, substituted or unsubstituted C₇ to C₂₀ heteroaryl with two or three fused aromatic six-member rings or CN,
wherein the one or more substituents are selected from electron-withdrawing group, CN, F, SF₅, unsubstituted C₁ to C₈ alkyl or D;
wherein the compounds E1 to E7 are excluded:

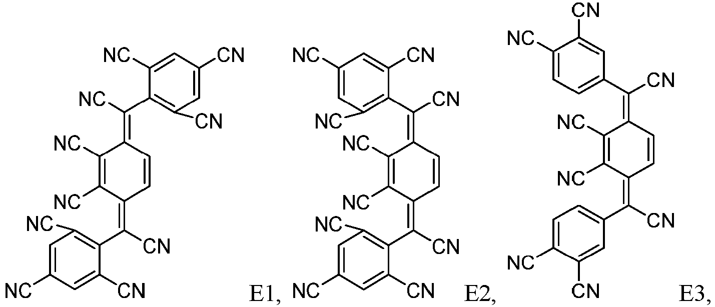

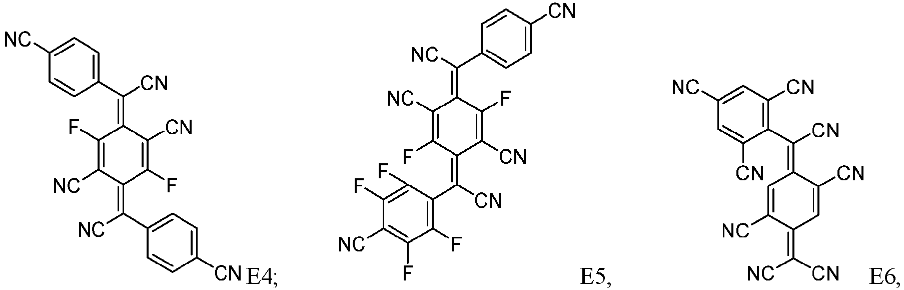

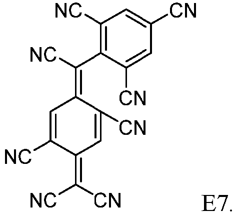

2. The compound of formula (I) according of claim 1, wherein the compounds of formulae (I) are excluded comprising the groups Ex-1 to Ex-4, wherein R¹, R², R³, R⁴, R' and R" are selected as follows:
| group | R1 | R2 | R³ | R⁴ | R' | R" |
|---|---|---|---|---|---|---|
| Ex-1 | CN | CN | H | H | 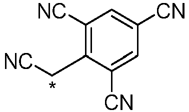 | 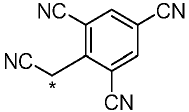 |
| Ex-2 | CN | CN | H | H | 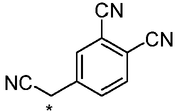 | 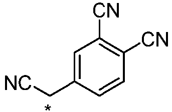 |
| Ex-3 | F | CN | CN | F | 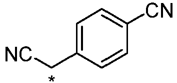 | 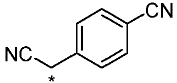 |
| Ex-4 | CN | F | F | CN | 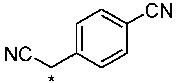 | 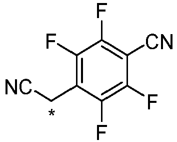 |

3. The compound of formula (I) according of claim 1 or 2, wherein R¹, R², R³ and R⁴ are independently selected from the group comprising F or CN; wherein two R¹, R², R³ and R⁴ are selected from CN and two R¹, R², R³ and R⁴ are selected from F.

4. The compound of formula (I) according to any one of claims 1 to 3, wherein the compound of formula (I) is selected from formulae (V) to (XVIII):

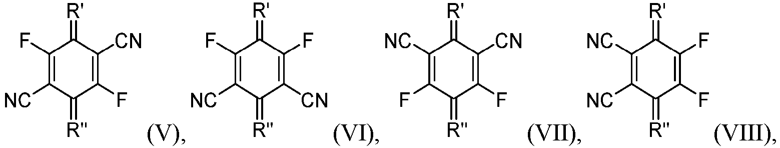

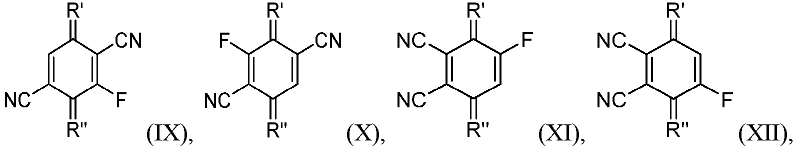

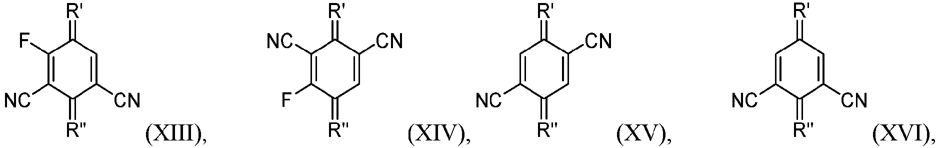

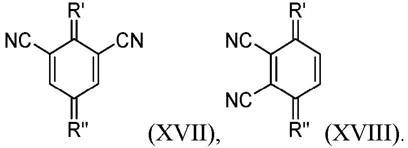

5. The compound of formula (I) according to any one of claims 1 to 4, wherein R¹, R², R³, R⁴, R' and R" of the compound of formula (I) are selected from the groups J1 to J26 as follows:
| group | R¹ | R² | R³ | R⁴ | R' | R" |
|---|---|---|---|---|---|---|
| J1 | F | CN | CN | F | 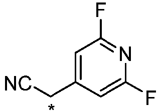 | 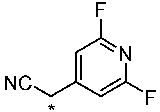 |
| J2 | F | CN | CN | F | 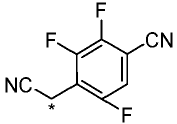 | 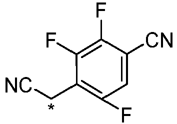 |
| J3 | F | CN | CN | F | 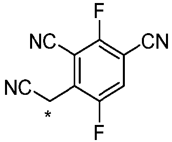 | 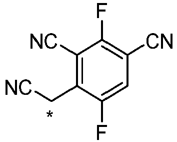 |
| J4 | F | CN | CN | F | 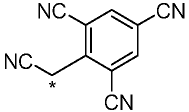 | 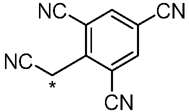 |
| J5 | F | CN | CN | F | 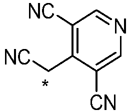 | 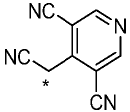 |
| J6 | F | CN | CN | F | 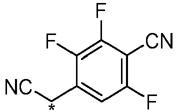 | 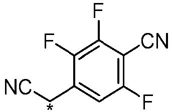 |
| J7 | F | CN | CN | F | 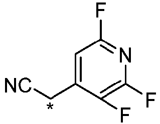 | 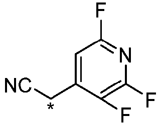 |
| J8 | F | CN | CN | F | 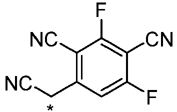 | 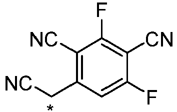 |
| J9 | F | CN | CN | F | 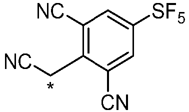 | 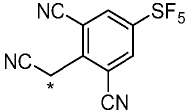 |
| J10 | F | CN | CN | F | 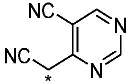 | 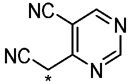 |
| J11 | F | CN | CN | F | 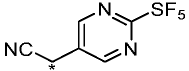 | 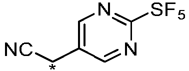 |
| J12 | F | CN | CN | F | 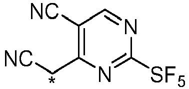 | 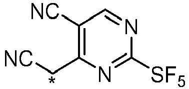 |
| J13 | F | CN | CN | F | 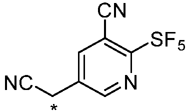 | 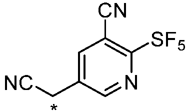 |
| J14 | F | CN | CN | F | 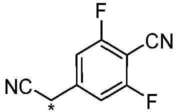 | 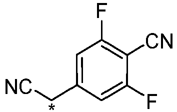 |
| J15 | CN | H | H | CN | 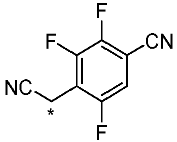 | 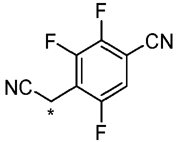 |
| J16 | CN | H | H | CN | 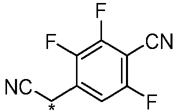 | 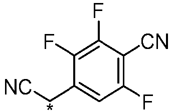 |
| J17 | F | CN | CN | F | 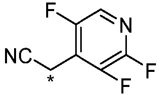 | 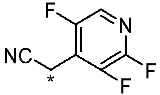 |
| J18 | F | CN | CN | F | 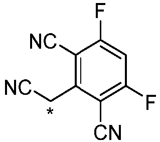 | 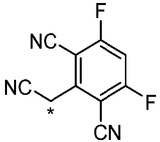 |
| J19 | F | CN | CN | F | 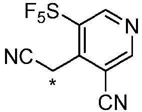 | 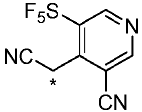 |
| J20 | F | CN | CN | F | 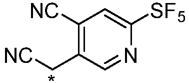 | 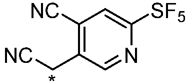 |
| J21 | F | CN | CN | F | 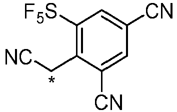 | 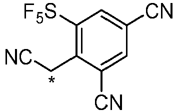 |
| J22 | H | CN | CN | H | 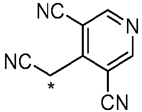 | 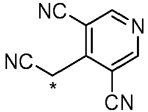 |
| J23 | F | CN | CN | F | 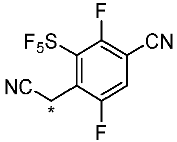 | 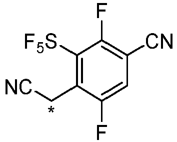 |
| J24 | F | CN | CN | F | 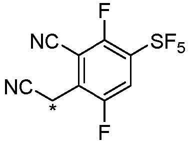 | 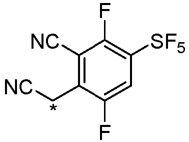 |
| J25 | F | CN | CN | F | 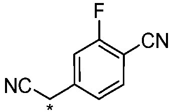 | 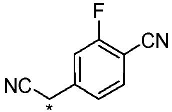 |
| J26 | H | CN | CN | H | 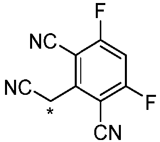 | 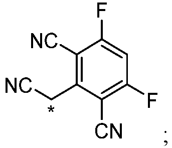 |
preferably the compound of formula (I) is selected from J1 to J24; further preferred wherein the compound of formula (I) is selected from J1 to J13; and additional preferred wherein the compound of formula (I) is selected from J1 to J5.

6. The compound of formula (I) according to any one of claims 1 to 5, wherein the compound of formula (I) is selected from formulae (XIX) to (XXX): 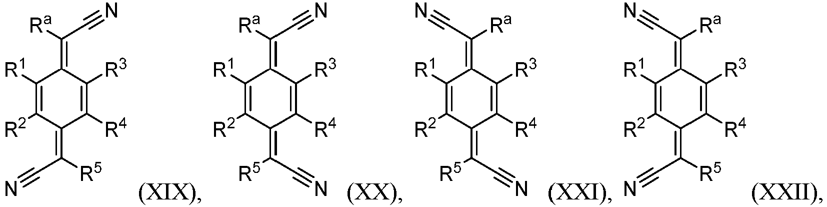 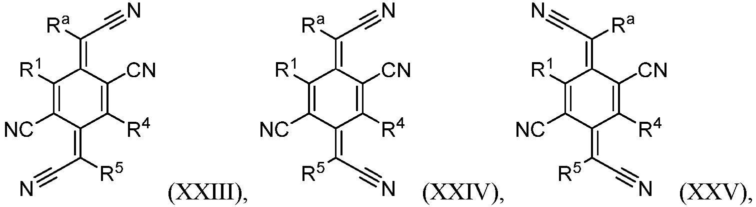 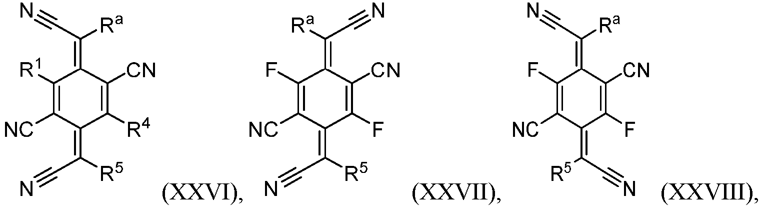 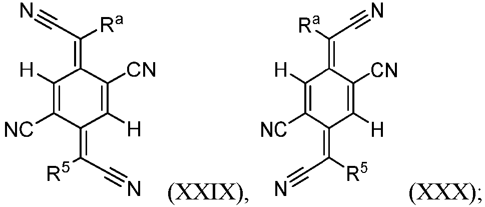 preferably the compound of formula (I) is selected from formulae (XIX) to (XXII), further preferred the compound of formula (I) is selected from formulae (XXIII) to (XXVI), and also preferred the compound of formula (I) is selected from formulae (XXVII) to (XXX).

7. The compound of formula (I) according to any of claims 1 to 6, wherein at least one to at most three X¹ to X⁵ are independently selected from CH or CD; or wherein at least one to at most two X¹ to X⁵ are independently selected from CH or CD.

8. The compound of formula (I) according to any of claims 1 to 7, wherein further at least one X¹ to X⁵ is CCN or N; or wherein ≥ 0 and ≤ 3 of X¹, X², X³, X⁴ and X⁵ are selected from N.

9. The compound of formula (I) according to any of claims 1 to 8, wherein at least one X¹ to X⁵ are independently selected from CH or CD, and at least two of R⁶ to R¹⁰ are independently selected from CN or F; preferably wherein at least one X¹ to X⁵ are independently selected from CH or CD, and at least three of R⁶ to R¹⁰ are independently selected from CN or F; also preferred at least two to three X¹ to X⁵ are independently selected from CH or CD, and at least one R⁶ to R¹⁰ are independently selected from CN or F; furthermore preferred wherein at least one to at most three X¹ to X⁵ are independently selected from CH or CD, and at least two R⁶ to R¹⁰ are independently selected from CN or F; additionally preferred wherein at least one to two X¹ to X⁵ are independently selected from CH or CD, and at least one R⁶ to R¹⁰are independently selected from CN or F; and moreover preferred at least one to at most two X¹ to X⁵ are independently selected from CH or CD, and at least two R⁶ to R¹⁰ are independently selected from CN or F.

10. The compound of formula (I) according to any of claims 1 to 9, wherein at least one X¹ to X⁵ are independently selected from CH or CD, and at least one of X¹ to X⁵ are independently selected from CCN or N; further preferred wherein at least one to at most three X¹ to X⁵ are independently selected from CH or CD, and at least one of X¹ to X⁵ are independently selected from CCN or N; further more preferred wherein one to three X¹ to X⁵ are independently selected from CH or CD, and at least one of X¹ to X⁵ are independently selected from CCN or N; and additionally preferred wherein at least one to at most two X¹ to X⁵ are independently selected from CH or CD, and at least one of X¹ to X⁵ are independently selected from CCN or N.

11. The compound of formula (I) according to any of claims 1 to 10, wherein at least one of X¹ to X⁵ are independently selected from CCN or N, and at least one R⁶ to R¹⁰ are independently selected from CN or F; preferably wherein at least one of X¹ to X⁵ are independently selected from CCN or N, and at least two R⁶ to R¹⁰ are independently selected from CN or F; further preferred wherein at least one of X¹ to X⁵ are independently selected from CCN or N, and at least one of R⁶ to R¹⁰ is F; and additionally preferred wherein at least one of X¹ to X⁵ are independently selected from CCN or N, and at least two of R⁶ to R¹⁰ are F.

12. The compound of formula (I) according to any of claims 1 to 11, wherein at least one X¹ to X⁵ are independently selected from CH or CD, and at least one of X¹ to X⁵ are independently selected from CCN or N, and at least one R⁶ to R¹⁰are independently selected from CN or F; preferably wherein at least one X¹ to X⁵ are independently selected from CH or CD, and at least one of X¹ to X⁵ are independently selected from CCN or N, and at least two R⁶ to R¹⁰ are independently selected from CN or F; further preferred wherein at least one to at most three X¹ to X⁵ are independently selected from CH or CD, and at least one of X¹ to X⁵ are independently selected from CCN or N, and at least one R⁶ to R¹⁰ are independently selected from CN or F; also preferred wherein at least one to at most three X¹ to X⁵ are independently selected from CH or CD, and at least one of X¹ to X⁵ are independently selected from CCN or N, and at least two R⁶ to R¹⁰ are independently selected from CN or F; furthermore preferred wherein at least one to at most two X¹ to X⁵ are independently selected from CH or CD, and at least one of X¹ to X⁵ are independently selected from CCN or N, and at least one R⁶ to R¹⁰ are independently selected from CN or F; and still preferred wherein at least one to at most two X¹ to X⁵ are independently selected from CH or CD, and at least one of X¹ to X⁵ are independently selected from CCN or N, and at least two R⁶ to R¹⁰ are independently selected from CN or F.

13. The compound of formula (I) according to any of claims 1 to 12, wherein R⁶, R⁷, R⁸, R⁹ and R¹⁰ are independently selected from CN, F, SF₅, D or H.

14. The compound of formula (I) according to any of claims 1 to 13, wherein R⁵ has the formulae (IVa) or (IVb);
wherein formula (IVa) is represented by: 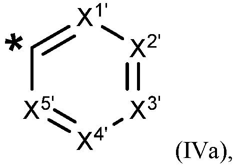
wherein
X¹ is selected from CR^{6'} or N;
X^{2'} is selected from CR^{7'} or N;
X^{3'} is selected from CR^{8'} or N;
X^{4'} is selected from CR^{9'} or N;
X^{5'} is selected from CR^{10'} or N;
wherein
R^{6'}, R^{7'}, R^{8'}, R^{9'} and R^{10'} are independently selected from CN, F, SF₅, D, H, substituted or unsubstituted C₆ to C₁₈ aryl or substituted or unsubstituted C₃ to C₁₈ heteroaryl, preferably H, D, CN, F, substituted or unsubstituted C₆ aryl or substituted or unsubstituted C₃ to C₅ heteroaryl;
wherein the one or more substituents on substituted C₆ to C₁₃ aryl, substituted C₃ to C₁₄ heteroaryl, substituted C₆ aryl, substituted C₃ to C₅ heteroaryl is selected from CN, F, unsubstituted C₁ to C₈ alkyl or D, preferably CN, F;
or
formula (IVb) is represented by: 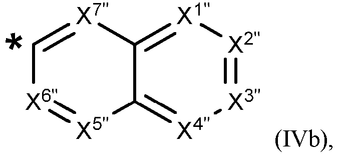
wherein
X^{1"} is selected from CR^{6"} or N;
X^{2"} is selected from CR^{7"} or N;
X^{3"} is selected from CR^{8"} or N;
X^{4"} is selected from CR^{9"} or N;
X^{5"} is selected from CR^{10"} or N;
X^{6"} is selected from CR^{11"} or N;
X^{7"} is selected from CR^{12"} or N;
wherein
R^{6"}, R^{7"}, R^{8"}, R^{9"}, R^{10"}, R^{11"} and R^{12"} are independently selected from CN, F, SF₅, D or H,
preferably H, D, CN, F;
and wherein the "*" denotes the binding position.

15. The compound of formula (I) according to any of claims 1 to 14, wherein R^{a} and/or R⁵ is independently selected from the group of F1 to F5, G1 to G8, H1 to H9, and I1 to I46: 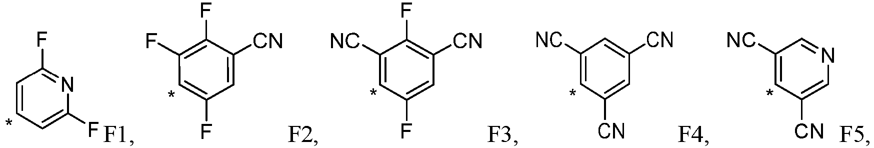 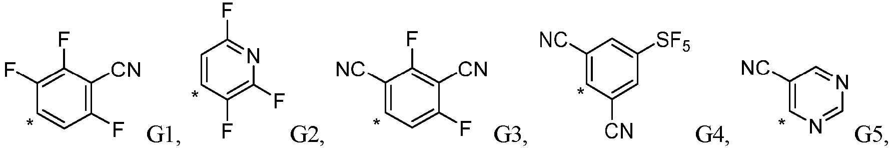 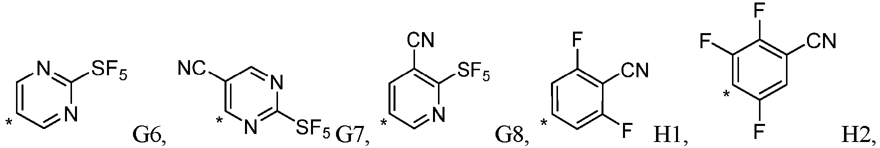 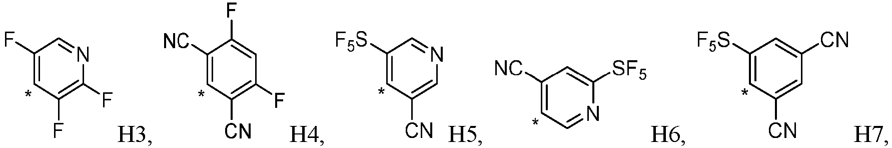 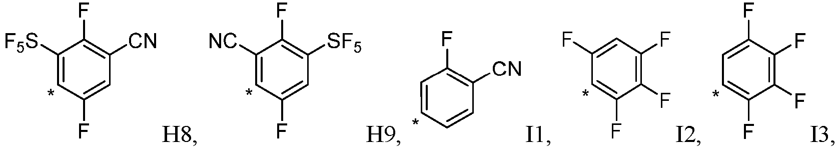 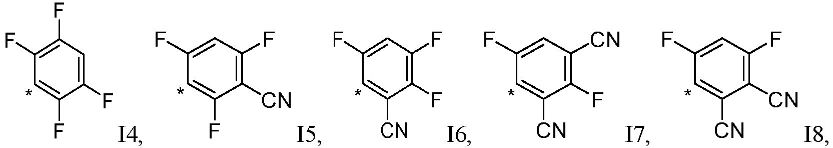 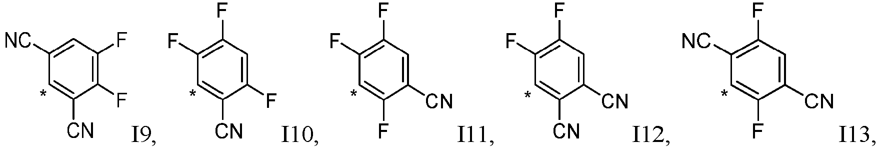 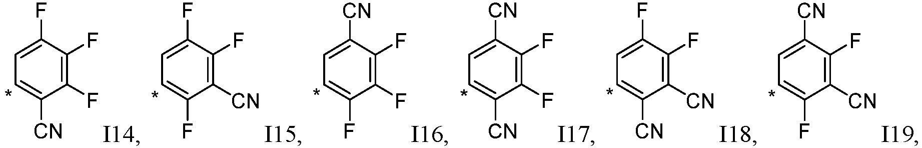 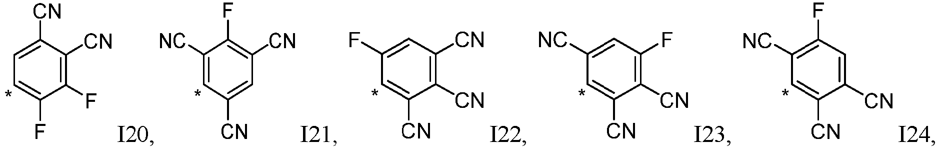 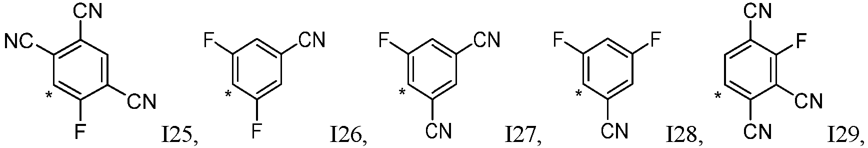 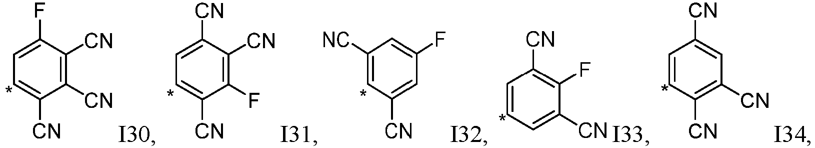 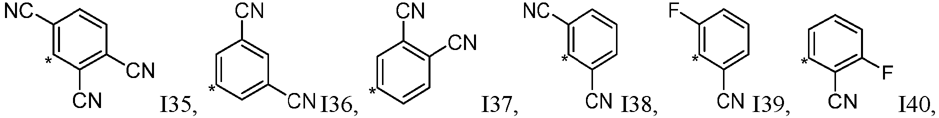 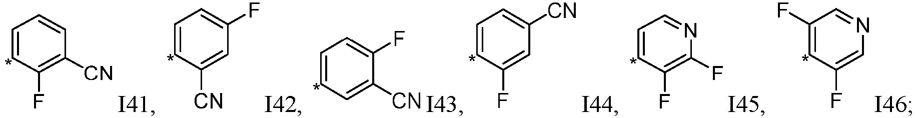 preferably R^{a} and/or R⁵ is independently selected from F1 to F5, G1 to G8, and H1 to H9; further preferred wherein R^{a} and/or R⁵ is independently selected from F1 to F5, and G1 to G8; and additionally preferred wherein R^{a} and/or R⁵ is independently selected from F1 to F5.

16. The compound of formula (I) according to any of claims 1 to 15, wherein R^{a} and R⁵ are independently selected from formula (III); and further preferred wherein R^{a} and R⁵ are selected from formula (III) and R^{a} and R⁵ are selected the same.

17. The compound of formula (I) according to any of claims 1 to 16, wherein R' and/or R" is independently selected from the group comprising F-CN1 to F-CN5, G-CN1 to G-CN8, H-CN1 to H-CN9, and I-CN1 to I-CN46: 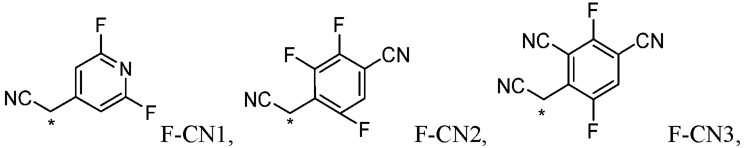 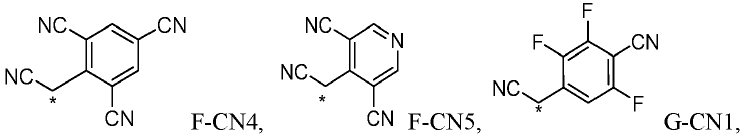 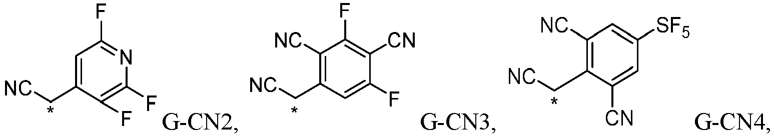 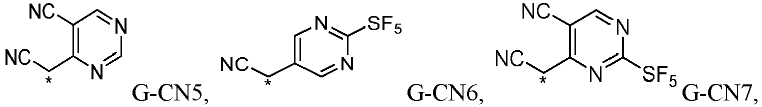 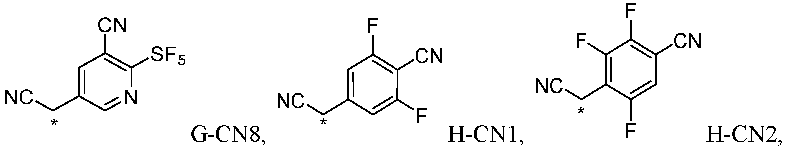 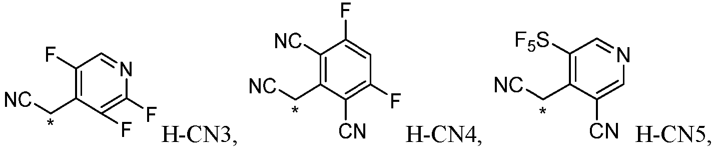 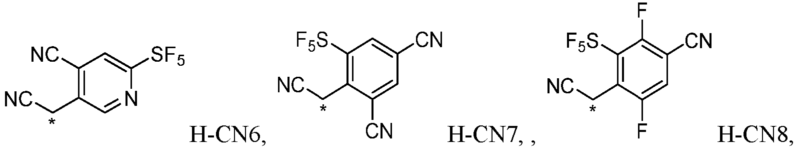 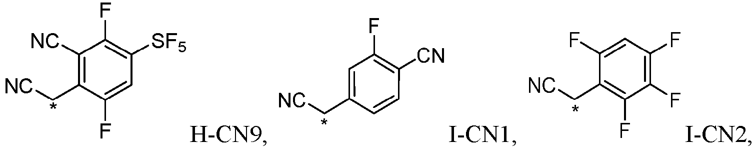 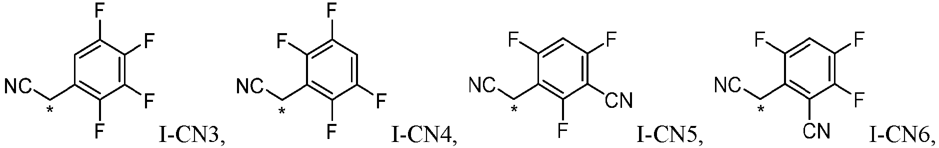 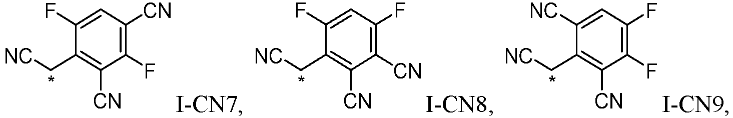 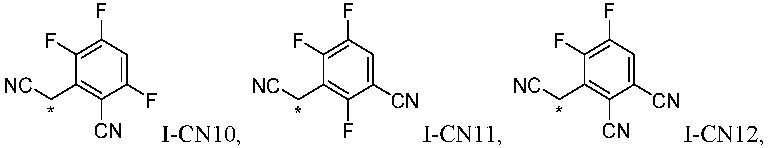 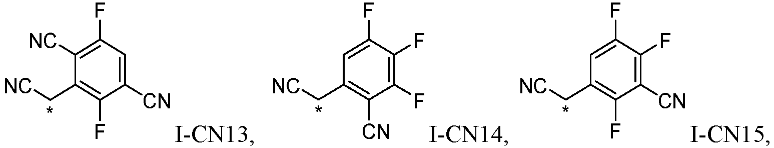 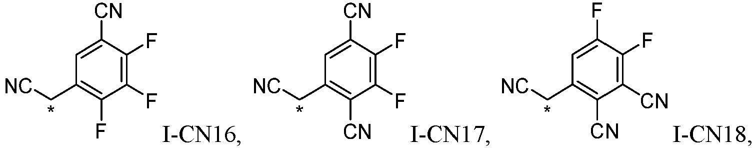 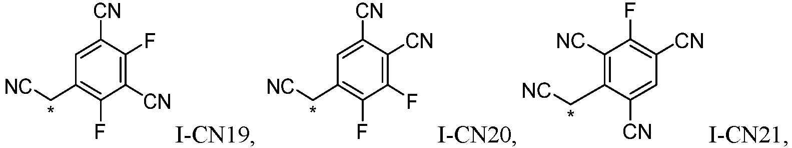 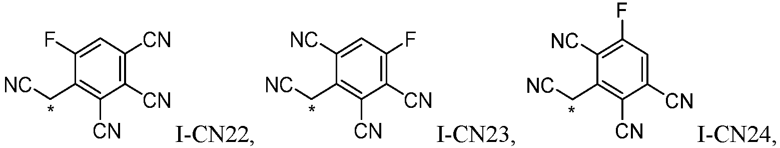 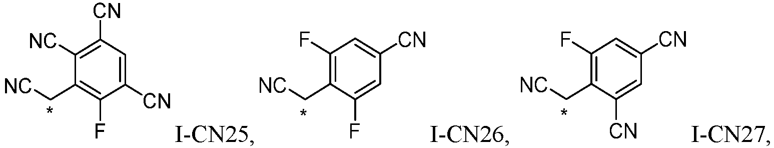 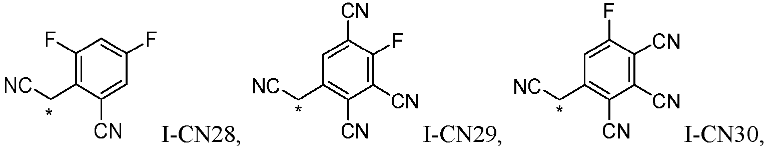 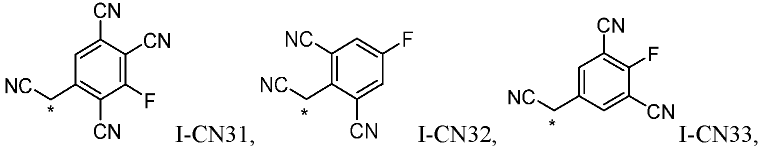 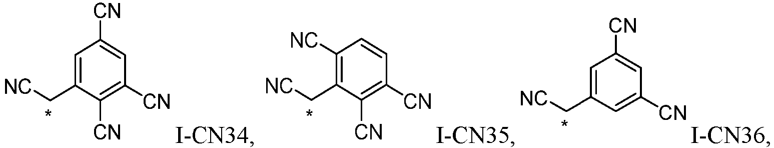 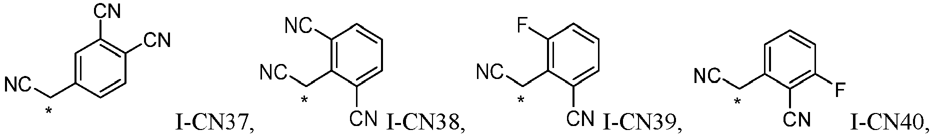 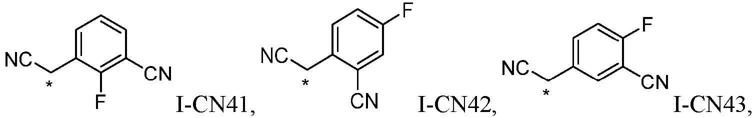 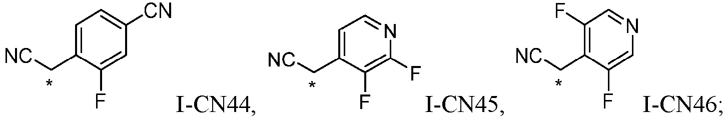 preferably wherein R' and/or R" is independently selected from F-CN1 to F-CN5, G-CN1 to G-CN8, and H-CN1 to H-CN9; further preferred wherein R' and/or R" is independently selected from F-CN1 to F-CN5, and G-CN1 to G-CN8; and additionally preferred wherein R' and/or R" is independently selected from F-CN1 to F-CN5.

18. The compound of formula (I) according to any of claims 1 to 17, wherein R' and R" are independently selected from formula (II); and preferably wherein R' and R" are selected from formula (II) and R' and R" are selected the same.

19. The compound of formula (I) according to any of claims 1 to 18, wherein the compound of formula (I) is selected from the compounds A1 to A15, B1 to B22, C1 to C28, and D1 to D4; 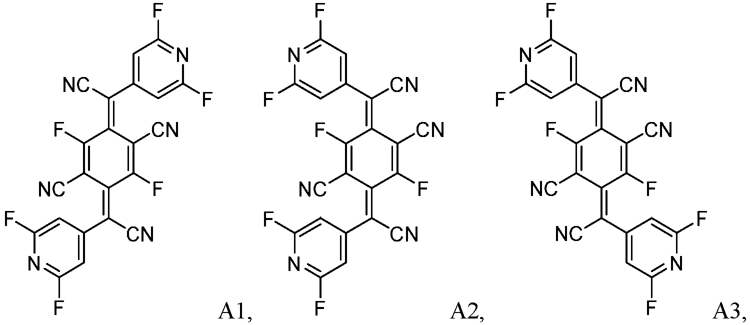 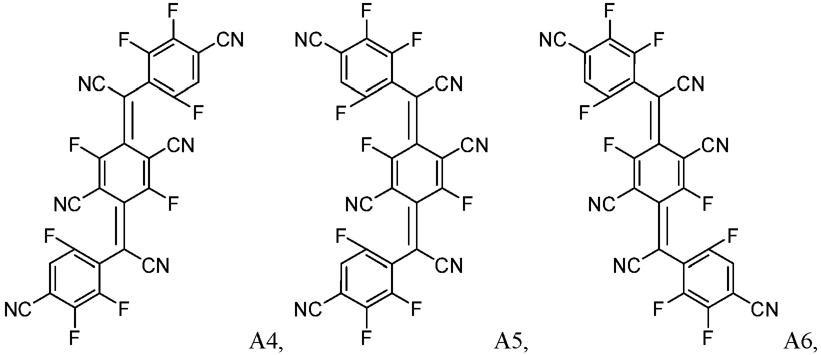 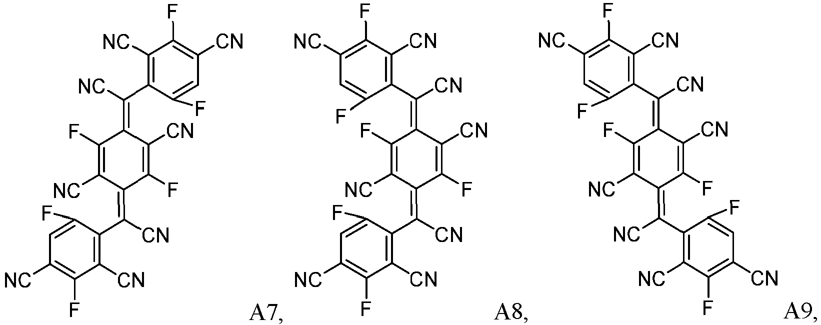 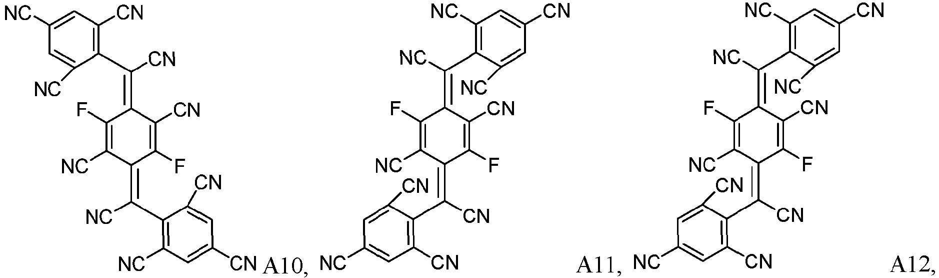 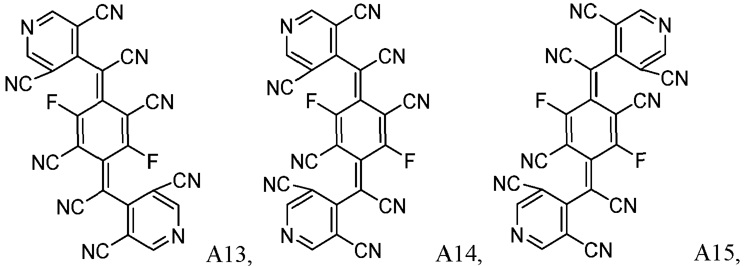 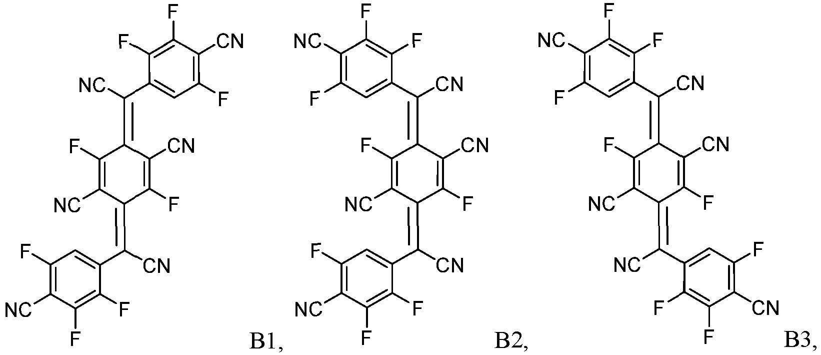 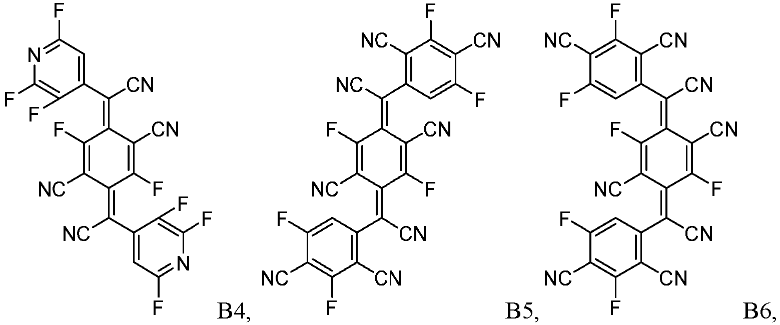 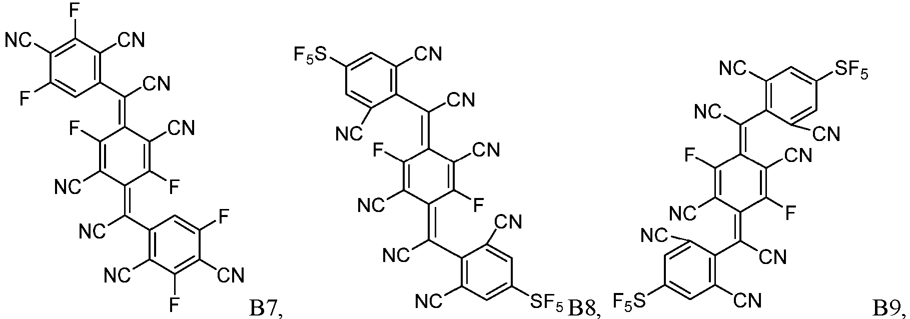 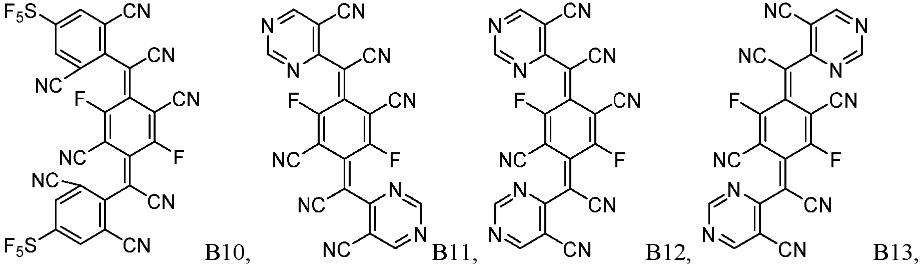 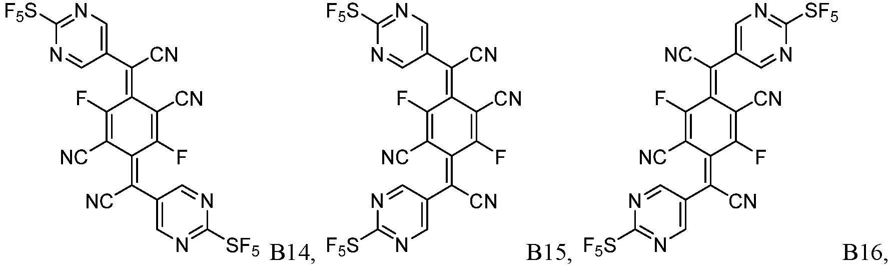 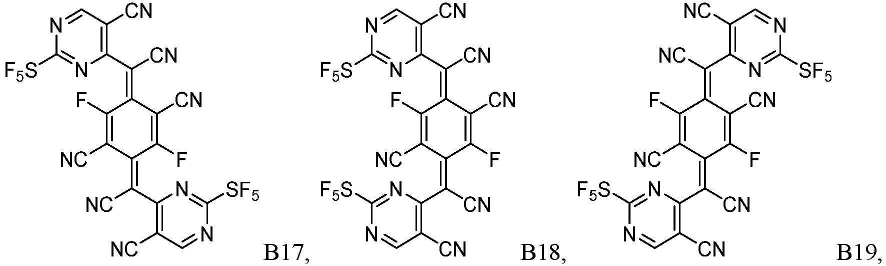 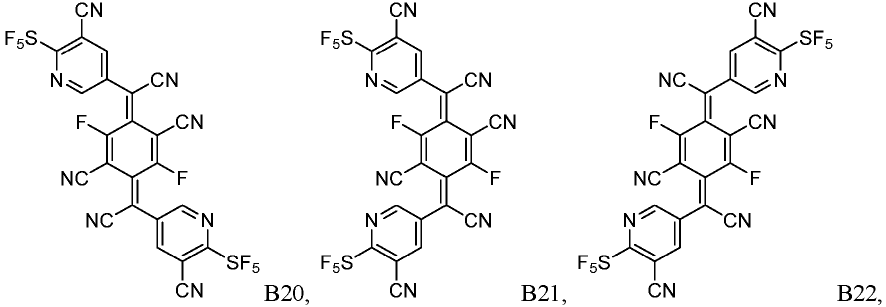 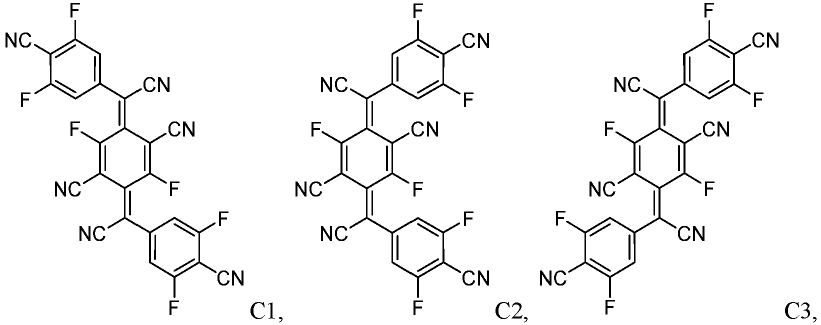 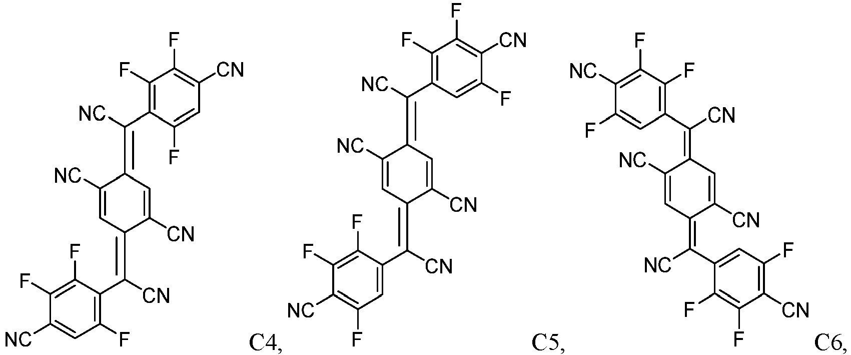 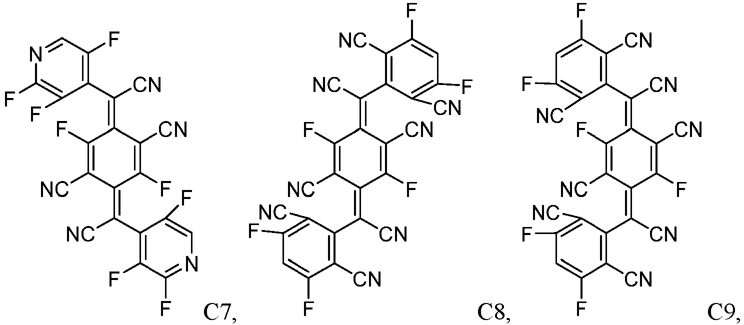 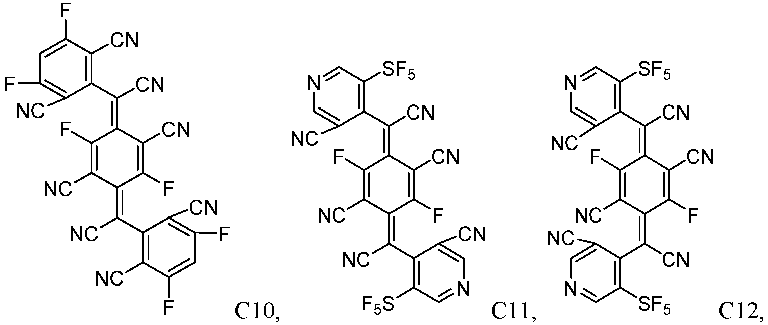 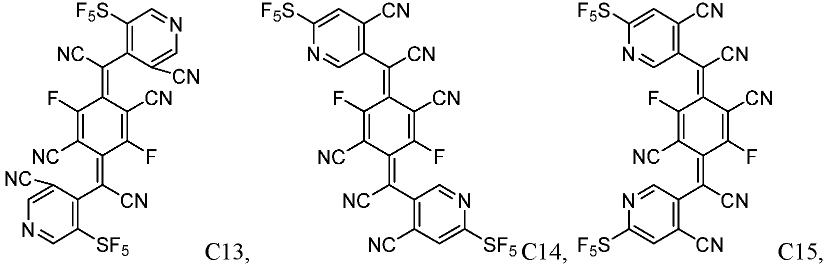 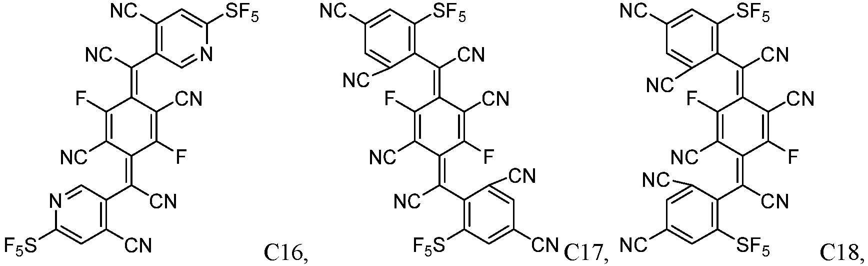 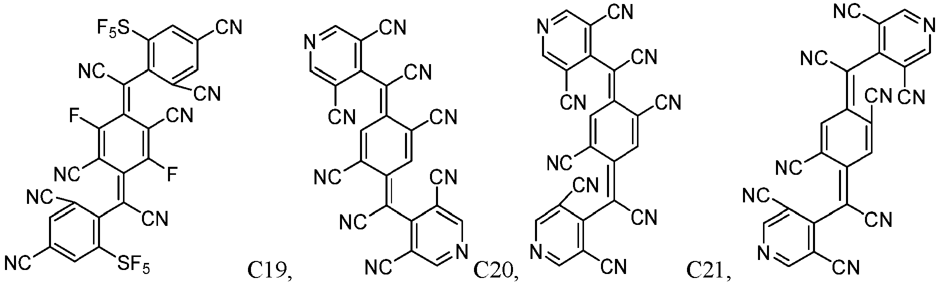 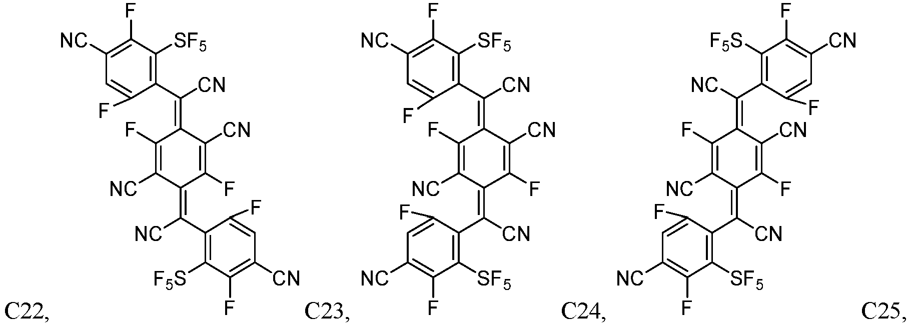 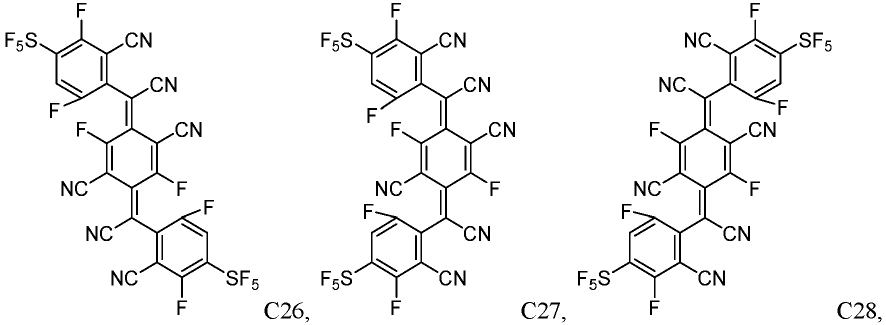 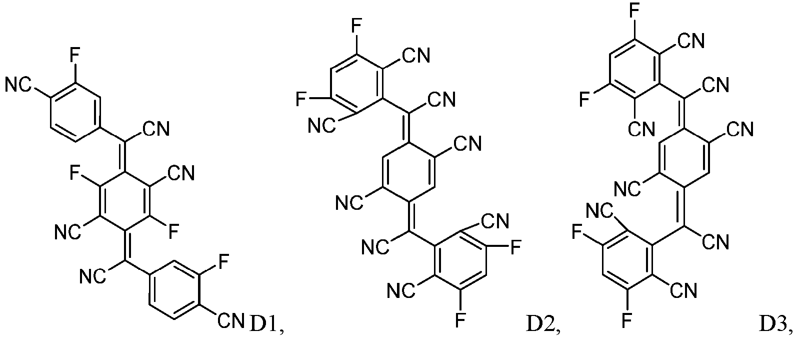 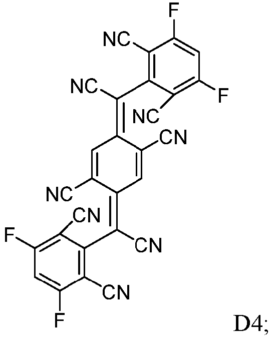 wherein the compound of formula (I) is preferably selected from the compounds A1 to A15, B1 to B22, and C1 to C28; further preferred wherein the compound of formula (I) is more preferably selected from the compounds A1 to A15, and B1 to B22; and additionally preferred wherein the compound of formula (I) is most preferably selected from the compounds A1 to A15.

20. An organic semiconductor layer, whereby the organic semiconductor layer comprises a compound of any of the preceding claims 1 to 19.

21. An organic electronic device comprising an anode layer, a cathode layer, and at least one organic semiconductor layer, wherein the at least one organic semiconductor layer is arranged between the anode layer and the cathode layer, and wherein the at least one organic semiconductor layer comprises a compound of formula (I) according to claims 1 to 20.

22. The organic electronic device of claim 21, whereby the organic electronic device further comprises at least one photoactive layer, wherein the at least one photoactive layer is arranged between the anode layer and the cathode layer; preferably at least one of the at least one organic semiconductor layers is arranged between the anode layer and the at least one photoactive layer.

23. The organic electronic device according to claim 21 or 22, whereby the photoactive layer is a light emitting layer.

24. The organic electronic device of any of the preceding claims 21 to 23, whereby the organic electronic device is an electroluminescent device, an organic light emitting diode (OLED), a light emitting device, thin film transistor, a battery, a display device or an organic photovoltaic cell (OPV).

25. A display device comprising an organic electronic device according to any of the preceding claims 21 to 24.
